# EUROPEAN PATENT APPLICATION

(11) **EP 4 582 541 A2**
(43) Date of publication of application: **09.07.2025**
(21) Application number: 25165144.4
(22) Date of filing: 26.01.2022
(51) Int. Cl.: C12N 15/10

(54) **FUNCTIONAL NUCLEIC ACID MOLECULE AND METHOD**

(30) Priority: 27.01.2021 EP 21153875
(62) Divisional of application: 22703431.1
(71) Applicant: The University Court Of The University of Edinburgh, Edinburgh EH8 9YL (GB)
(72) Inventor: POLLARD, Steven, Edinburgh, EH16 4UU (GB); KOEBER, Ute, Edinburgh, EH16 4UU (GB); MATJUSAITIS, Mantas, Edinburgh, EH16 4UU (GB)
(74) Representative: HGF

(57) **Abstract**

The disclosure relates to synthetic super-enhancers activated by one or more transcription factors. In particular, the present disclosure describes synthetic super-enhancers that are activated by one or more transcription factors expressed in a target cell and used to express transgenes, such as adverse payloads, upon activation of the synthetic super-enhancer by the transcription factor(s).

## Description

### FIELD

The disclosure relates to synthetic super-enhancers activated by one or more transcription factors. In particular, the present disclosure relates to synthetic super-enhancers that are activated by transcription factors expressed in target cells and are coupled to an adverse payload in order to kill target cells upon activation. The disclosure also relates to synthetic super-enhancers activated by one or more transcription factors of the SOX family.

### BACKGROUND

There is great interest in identification of cell type-specific enhancers and promoters that can be deployed in gene therapies, particularly where a therapeutic protein cargo needs to be expressed in highly cell selective manner to ensure maximum dosing and reduced off target effects. However, current strategies have limited success, and must often make compromises in size, selectivity or strength.

Two distinct, yet complementary, strategies can be used to identify cell type specific enhancers. First, strong and selective natural enhancers for a cell type of interest may emerge from systematic functional genetic dissection of regulatory networks for the cell type or lineage of interest. Second, fully synthetic enhancers can be constructed based on artificially synthesized DNA binding motifs of the known key transcription factors (TFs). Databases of such motifs - typically around 5-10 base pairs long - have been defined based on biochemical assays for strongest binding, such as JASPAR (Jolma et al. 2013). These transcription factor binding motifs can be synthesized and screened in high throughout using pooled libraries (Jolma et al. 2013). However, there are drawbacks to each approach. Individual natural enhancers are typically not strong enough to be useful in phenotypic assays or limited by size constraints or inappropriate selectivity. Artificial promoters built upon concatemers of the transcription factor binding motifs can induce expression, but do not have the appropriate 'grammar' - the spacing, order and appropriate affinity - that exists in natural enhancer sequences and is required for recruitment of cooperating TFs and co-factors (Farley et al. 2015). These will therefore lack selectivity and robustness.

Promoter sequences, enhancer sequences and methods of identifying such sequences are described in the art, but these suffer from the drawbacks discussed above. WO2014066848 and US2014/0296218 describe natural super-enhancers that are claimed to be useful for modulating expression of cell type-specific genes and methods for identifying said super-enhancers. WO2017155973 describes synthetic enhancers for use in recombinant parvoviruses, particularly enhancers with reduced size to enable delivery of large genetic payloads. WO2012101191 describes designing promoters for selective expression of genes by using transcription factor binding site motif prediction to create minimized promoters based on these motifs.

Therefore, there remains a need in the art to provide enhancers that have suitable selectivity and activity to be used in therapeutic methods, such as gene therapies.

### SUMMARY

According to a first aspect, there is provided a super-enhancer activated by one or more transcription factors, wherein the super-enhancer comprises two or more enhancer sequences derived from different genomic loci, wherein each enhancer sequence comprises a binding site for the transcription factor. In an alternative teaching, there is provided a synthetic super-enhancer activated by one or more transcription factors, wherein the synthetic super-enhancer comprises two or more enhancer sequences derived from different genomic loci, wherein each enhancer sequence comprises a binding site for the transcription factor and between 20 and 400 nucleotides upstream and/or downstream of the binding site for the transcription factor present in its genomic locus.

The term "synthetic" may embrace super-enhancer constructs where two or more enhancer sequences, normally located in different genomic loci, are isolated and combined within a new construct. A synthetic super-enhancer may therefore not exist in nature and may be described as artificial or 'manufactured'. Accordingly, disclosed herein is a construct activated by one or more transcription factors, wherein the construct comprises two or more enhancer sequences derived from different genomic loci, wherein each enhancer sequence comprises a binding site for the transcription factor. For convenience, the term 'synthetic-super enhancer' will be used to embrace any of the super-enhancers/constructs described herein.

According to a further aspect, there is provided a functional nucleic acid molecule comprising the synthetic super-enhancer described herein, operably linked to a transgene. In one teaching the functional nucleic acid molecule comprises a super-enhancer construct as described herein, operably linked to a transgene

According to a further aspect, there is provided a functional nucleic acid molecule comprising a synthetic super-enhancer activated by one or more transcription factors expressed in a target aberrant cell and an adverse payload, wherein said payload is expressed upon activation of the synthetic super-enhancer by the transcription factor(s). In an additional teaching there is provided a functional nucleic acid molecule comprising a super-enhancer construct activated by one or more transcription factors expressed in a target aberrant cell and an adverse payload, wherein said payload is expressed upon activation of the super-enhancer construct by the transcription factor(s)

According to a further aspect, there is provided a synthetic super-enhancer activated by one or more transcription factors of the SOX family, wherein the synthetic super-enhancer comprises two or more enhancer sequences and at least one SOX motif and/or SOX dimer motif. In one teaching, there is provided a super-enhancer construct activated by one or more transcription factors of the SOX family, wherein the super-enhancer construct comprises two or more enhancer sequences and at least one SOX motif and/or SOX dimer motif.

According to a further aspect, there is provided a synthetic super-enhancer activated by SOX2, wherein the synthetic super-enhancer comprises four enhancer sequences derived from different genomic loci and wherein each of the enhancer sequences comprises at least one SOX dimer motif. In one teaching, there is provided a super-enhancer construct activated by SOX2, wherein the super-enhancer construct comprises four enhancer sequences derived from different genomic loci and wherein each of the enhancer sequences comprises at least one SOX dimer motif.

According to a further aspect, there is provided a vector comprising the synthetic super-enhancer, the super enhancer construct or the functional nucleic acid molecule as described herein.

According to a further aspect, there is provided a composition comprising the synthetic super-enhancer, the super enhancer construct, the functional nucleic acid molecule or the vector as described herein, and an acceptable carrier.

According to a further aspect, there is provided a composition comprising the functional nucleic acid molecule described herein, for use in therapy.

According to a further aspect, there is provided a composition comprising the functional nucleic acid molecule described herein, for use in the treatment of cancer.

According to a further aspect, there is provided a kit comprising the functional nucleic acid molecule comprising an adverse payload as described herein, wherein the adverse payload is a suicide gene which encodes a protein capable of converting an inactive prodrug into a cytotoxic drug, and wherein the kit additionally comprises said inactive prodrug for administration with the functional nucleic acid molecule, vector or composition.

According to a further aspect, there is provided a method for treating cancer comprising administering the functional nucleic acid molecule or the composition as described herein, to a patient.

According to a further aspect, there is provided a method for treating glioblastoma comprising administering a functional nucleic acid molecule and an inactive prodrug to a patient,
wherein the functional nucleic acid comprises a synthetic super-enhancer activated by a SOX transcription factor and a suicide gene which encodes a protein capable of converting the inactive prodrug into a cytotoxic drug, and
wherein the suicide gene is expressed upon activation of the synthetic super-enhancer by the SOX transcription factor in glioblastoma cells.

In one teaching, there is provided a method for treating glioblastoma comprising administering a functional nucleic acid molecule and an inactive prodrug to a patient,
wherein the functional nucleic acid comprises a super-enhancer construct activated by a SOX transcription factor and a suicide gene which encodes a protein capable of converting the inactive prodrug into a cytotoxic drug, and
wherein the suicide gene is expressed upon activation of the super-enhancer construct by the SOX transcription factor in glioblastoma cells.

### BRIEF DESCRIPTION OF FIGURES

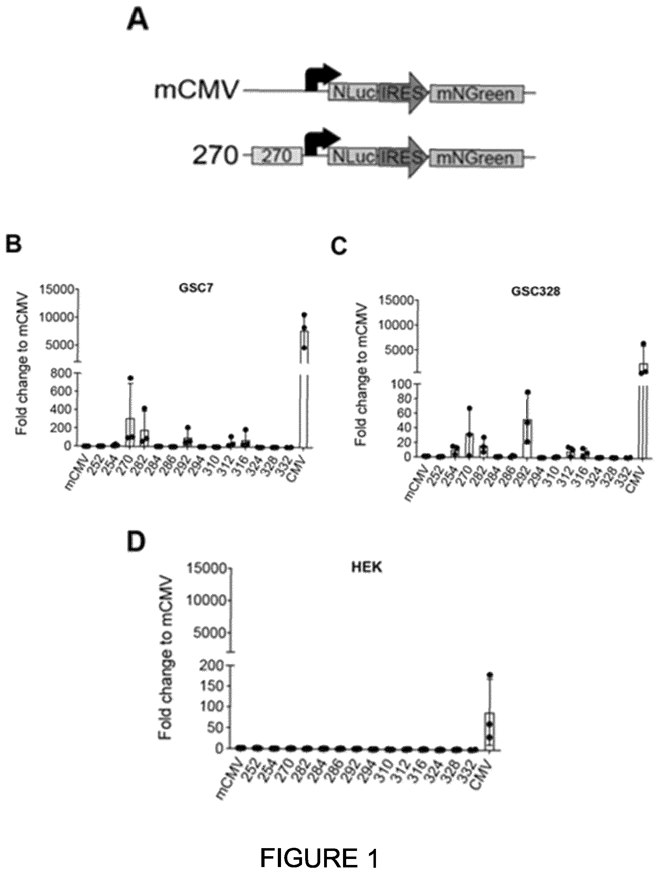

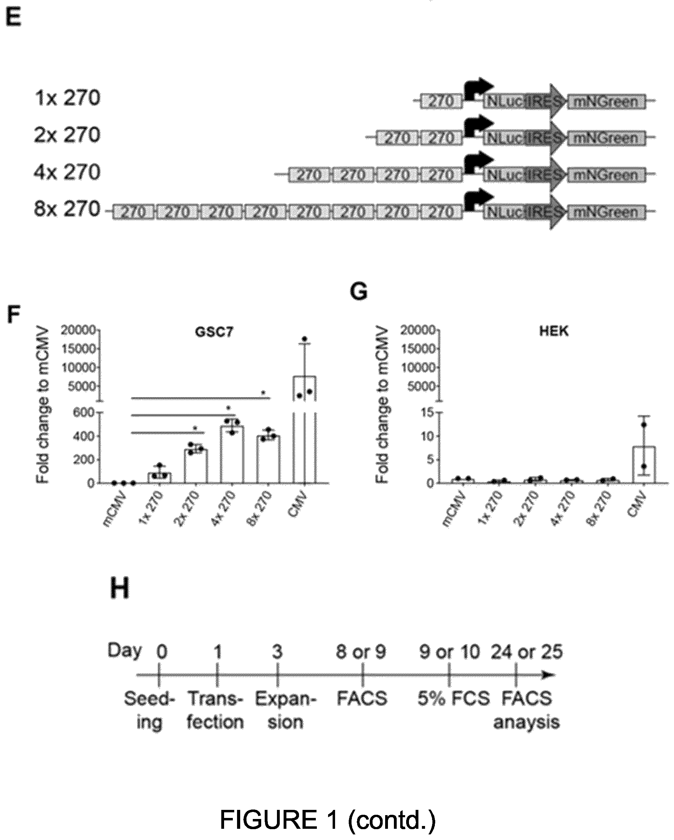

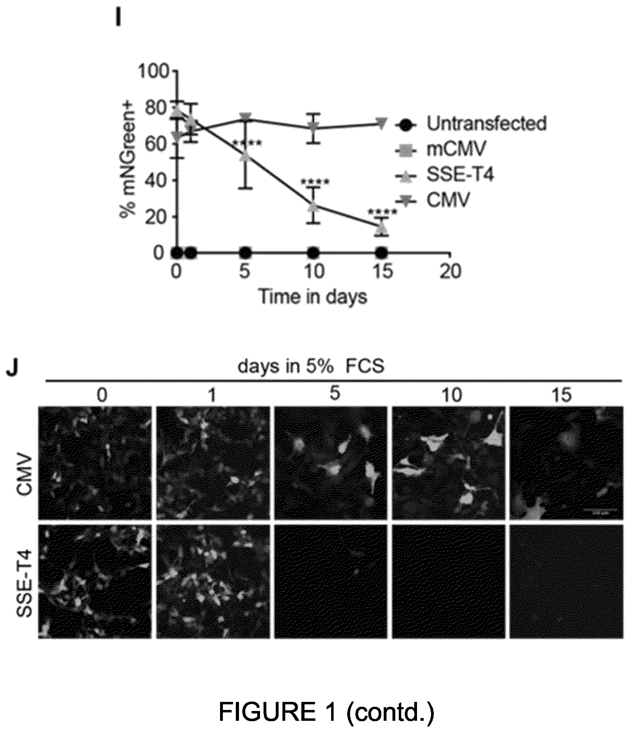

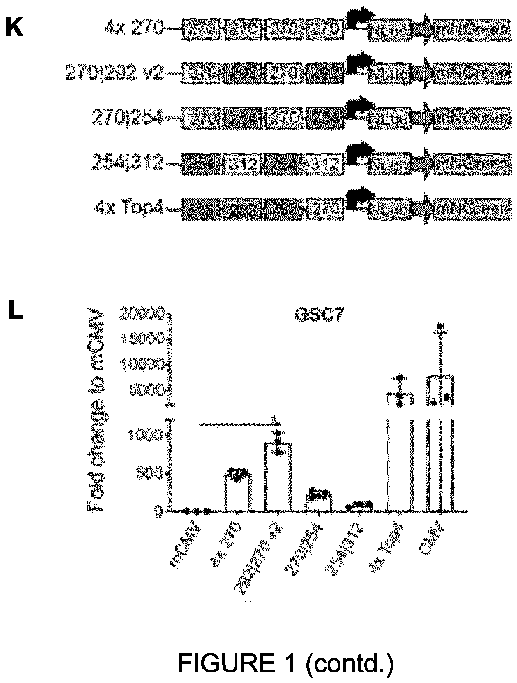

**FIGURE 1****: Candidate full-length enhancers identified from SOX2 ChIP-Seq peaks can act synergistically and are selectively active.** (A) Schematic of the constructs driven by a mCMV and a candidate enhancer, illustrated by candidate enhancer 270. Activity of 14 candidate enhancers in GSC7 (B), or G328 (C), when upstream of mCMV; a full-length CMV promoter is provided as positive reference control. (D) Activity of candidate enhancers in HEK293 cells. (E) Schematics of enhancer 270 clustering. (F) Enhancer activity is highest when clustering 4 x 270 enhancers together. (G) Activity of clustered enhancer constructs in HEK293 cells. (H) Outline of selectivity in differentiating GSC. (I) Flow cytometry of the activity of SSE-T4 (referred to as '4x Top4' in panel K). (J) Live cell images of the activity in SSE-T4 (also referred to herein as SSE-4) in cells scored in panel (I). (K) Schematic of construct designs for testing of multiple enhancers combined. (L) Relative activity of constructs from panel K in luciferase assays in GSC7 (not to scale). Paired Two-way Anova, * = p-Value ≤0.05, ** = p-Value ≤0.01. Every dot represents a biological replicate.

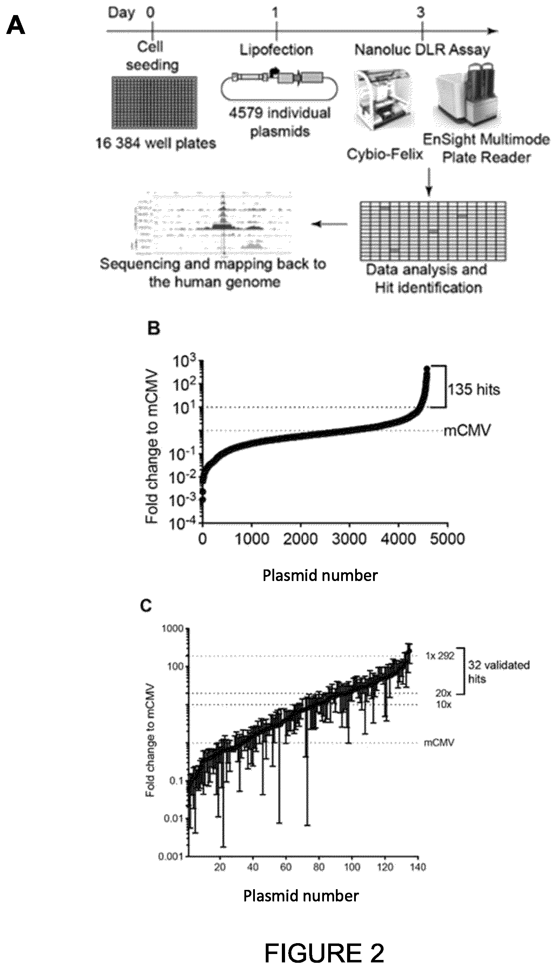

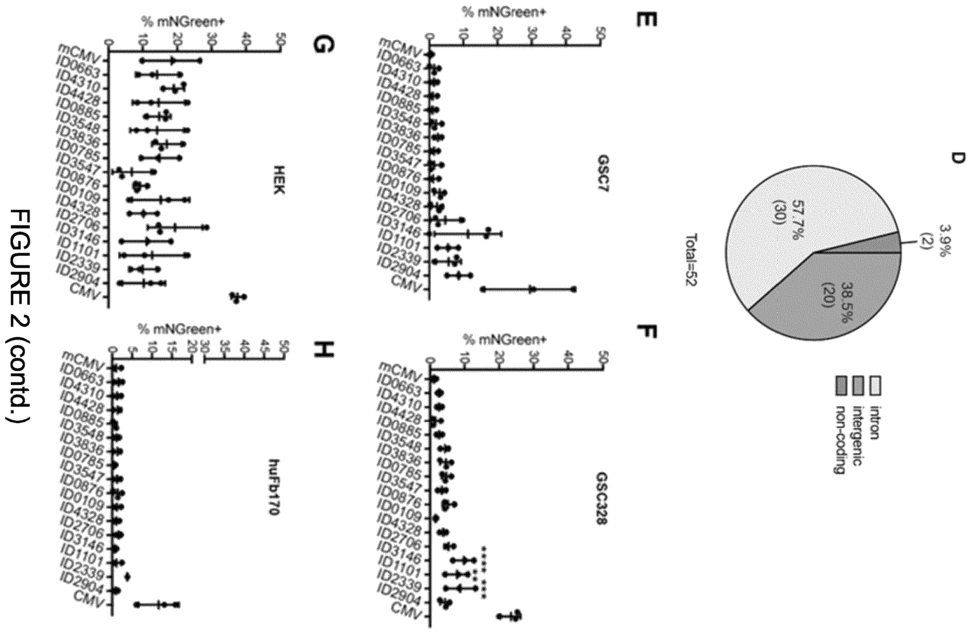

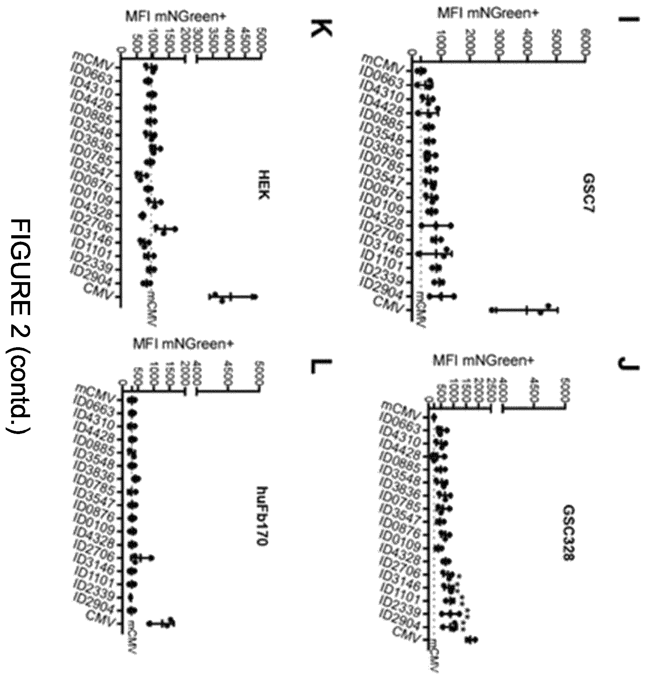

**FIGURE 2****: Functional enhancer screening of an arrayed plasmid library (~4000 plasmids) using plate-based luciferase reporter assays.** (A) Experimental outline of the enhancer screen. (B) Results for the enhancer screen (C) Validation of the 135 hits of the initial screen in Nanoluc DLR assay (D) Most of the hits (>10 greater than mCMV) lie in intronic regions. % of mNGreen+ cells for the Top 17 hits in (E) GSC7, (F) GSC328, (G) HEK293, (H) huFb710. MFI of mNGreen+ cells in (I) GSC7, (J) GSC328, (K) HEK293 and (L) huFb170.

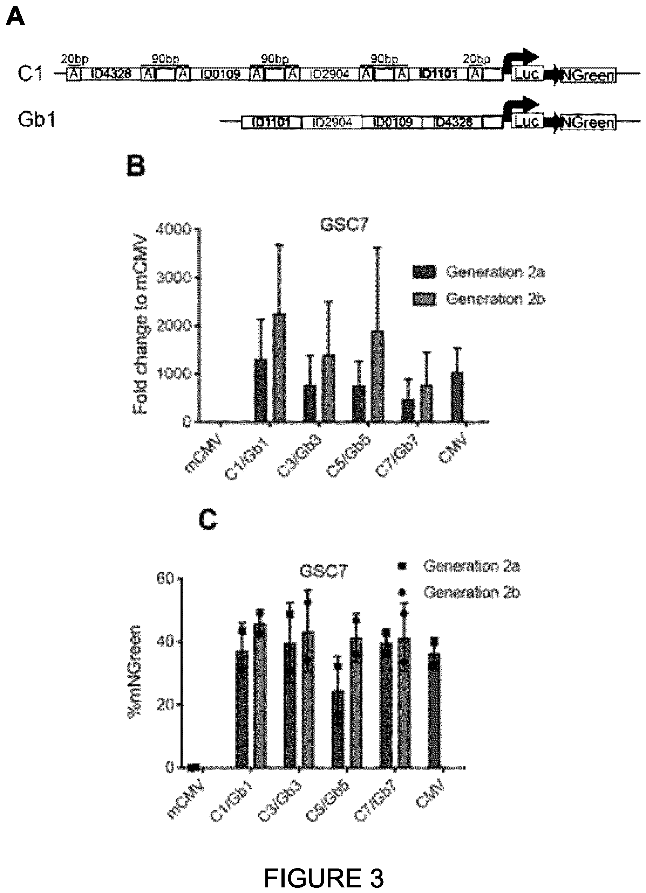

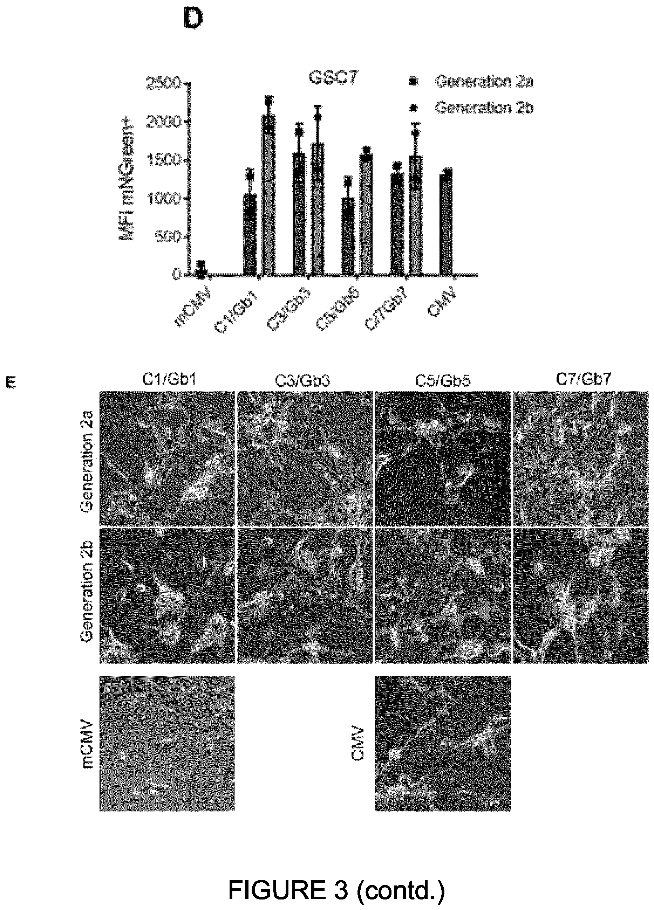

**FIGURE 3****: Comparison of synthetic super-enhancers with restriction enzyme adaptor cloning sites removed.** (A) Summary of design for second generation synthetic super-enhancers - C1 represents generation 2a (974 bp) and Gb1 represents generation 2b (640 bp); A = 20bp adapter, adjacent white box = 90bp spacer; n = 3 biological replicates. Note: The apparent discrepancy in size is due to 6 x 4 bp cloning sites which is not accounted for in the scheme for simplicity. (B) Nanoglo DLR assay to compare generation 2a and generation 2b synthetic super-enhancers (with and without cloning sites/adaptors removed, respectively). (C) Percentage of mNGreen+ cells of Generation 2a and Generation 2b synthetic super-enhancers. (D) MFI of mNGreen+ cells in Generation 2a and Generation 2b synthetic super-enhancers. (E) Representative live cells images for Generation 2a and Generation 2b synthetic super-enhancers; 20x magnification, scalebar = 50 µm.

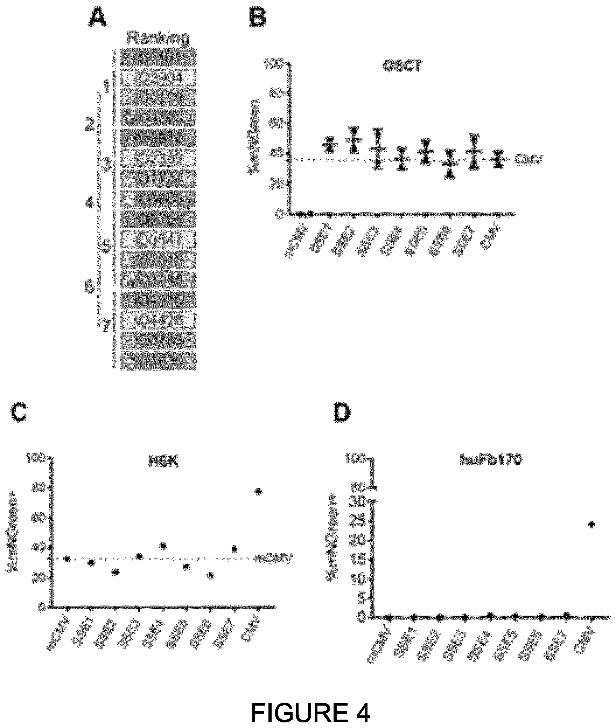

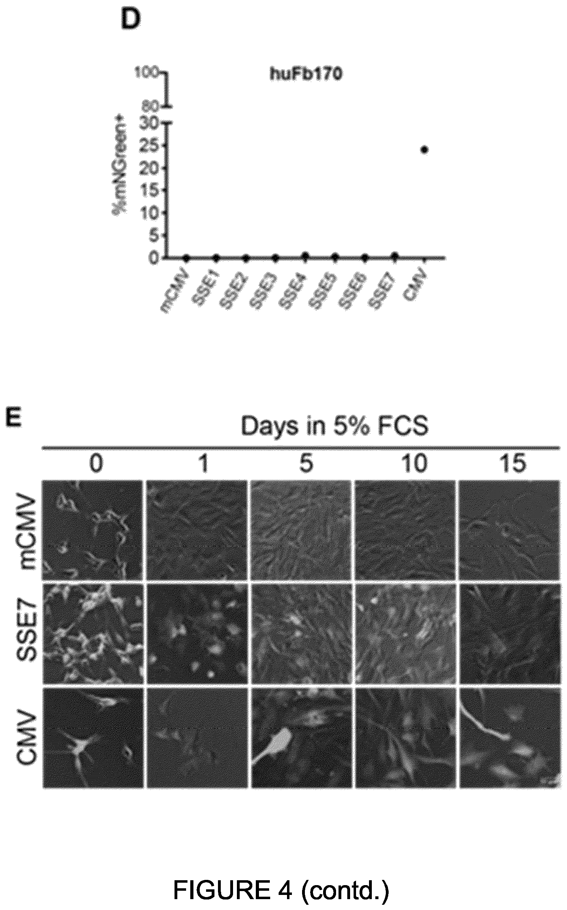

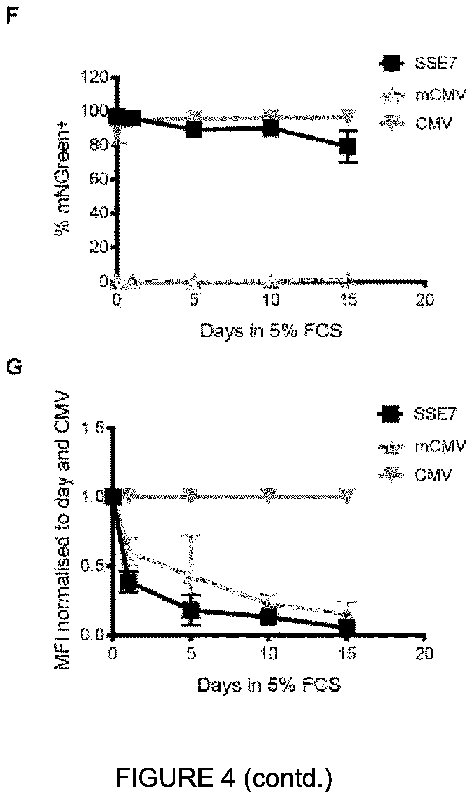

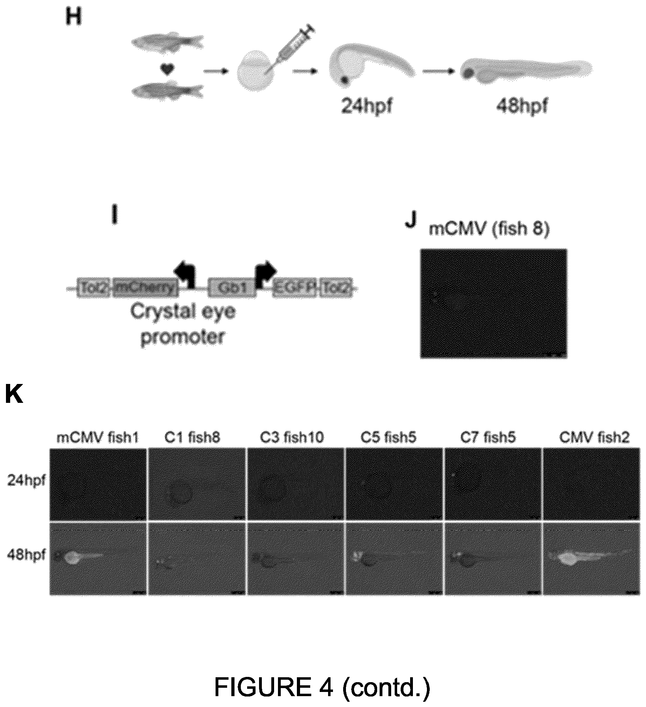

**FIGURE 4****: SSE-7 has selectivity for GSCs and forebrain neural stem cells (NSCs).** (A) Schematic of designs for four-part combinations comprising SSEs (SSE-1 through SSE-7; left numbers). (B) Flow cytometry confirms these all have activity at similar levels to CMV. (C) In HEK293 cells all SSEs are inactive. (D) In human fibroblasts (huFb170) all SSEs are inactive. (E) Live cell images for SSE-7 and controls during astrocytic differentiation using 5% FCS. (F) % of mNGreen+ cells of SSE-7 and controls in 5% FCS. (G) MFI of mNGreen+ cells for SSE-7 normalised to CMV expression at day 0. (H) Experimental outline for the assessment of SSE-7 in zebrafish embryos and larvae. (J) Injection control (I) Schematics of the construct used for this experiment. (K) Activity of generation 2a plasmids C1, C3, C5 and C7, (note: these correspond to the latter derivatives: SSE-1, 3, 5 and 7, respectively, and contain adapter sequences) at 24 and 48 hours post fertilisation (hpf).

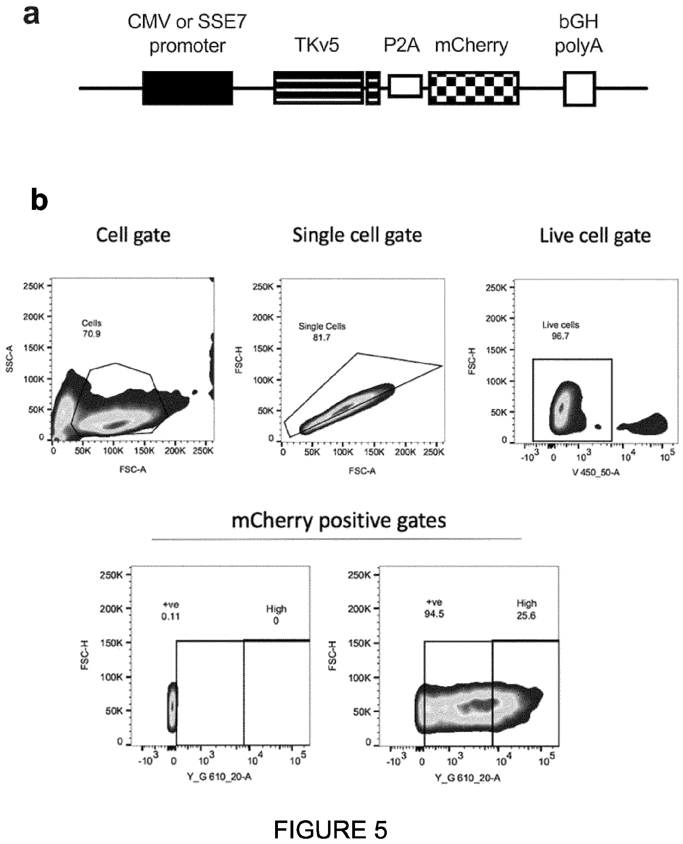

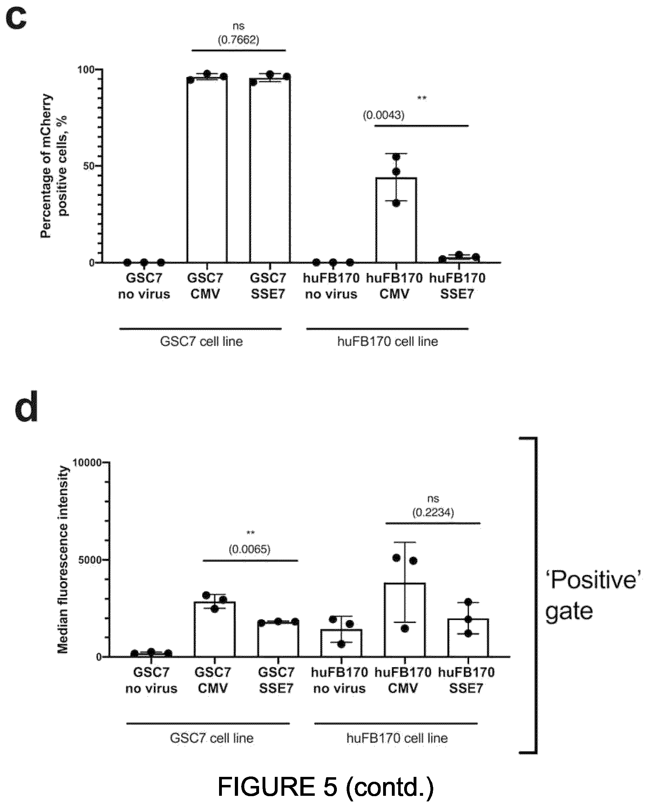

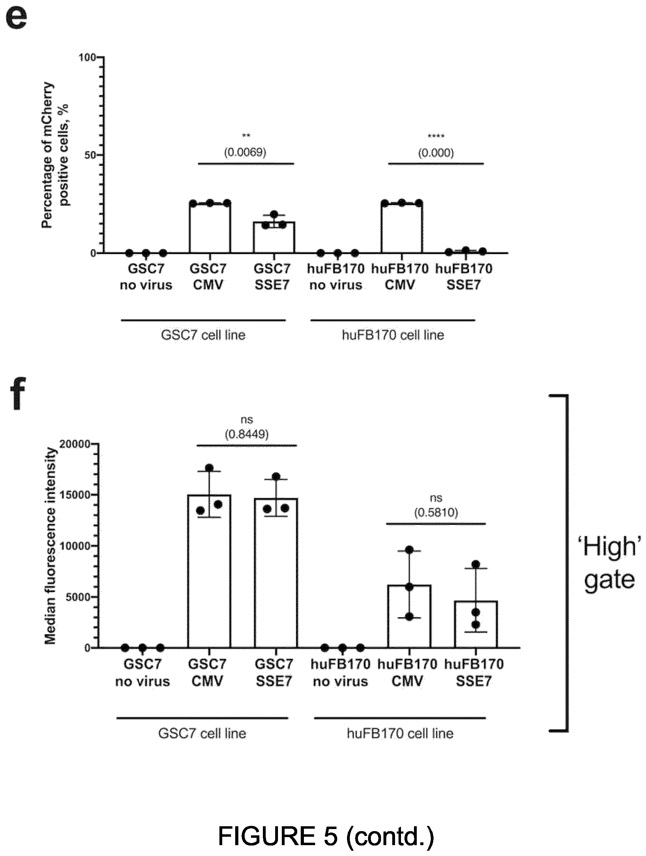

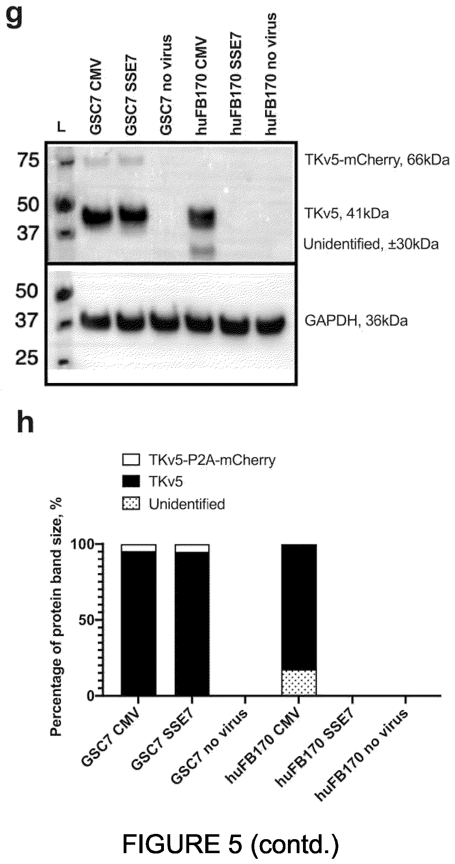

**FIGURE 5****: Flow cytometry and Western immunoblot results for SSE-7 driving expression of multiple proteins** (a) Schematic of the expression cassette, illustrating both mCherry and HSV-TKv5 expression from SSE-7 in a polycistronic transcript (including a P2A self-cleaving peptide sequence). (b) Flow cytometry data showing mCherry expression levels following delivery of this nucleic acid with a viral delivery (AAV). (c) Quantitation of flow cytometry results to score % of positive mCherry expressing cells and (d, e and f) shows the gating strategy. (g) Western blot confirming HSV-TK protein expression (using a fused v5 C-terminal epitope tag for detection). (h) Quantification of Western blot results in (g).

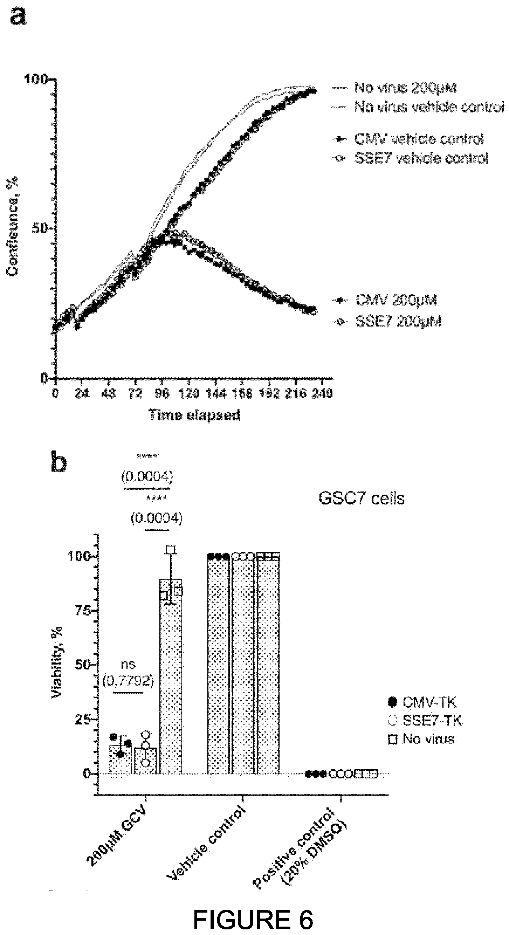

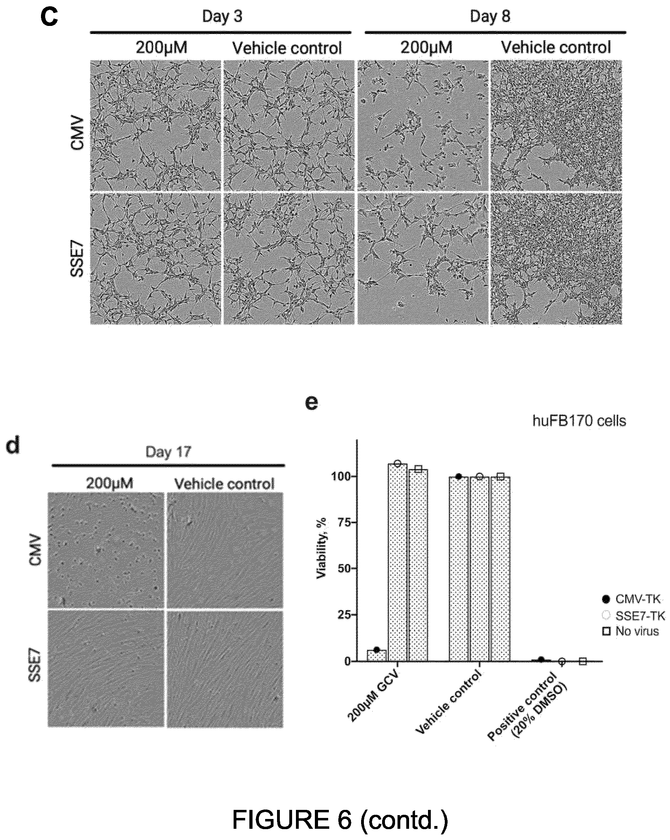

**FIGURE 6****: Cytotoxicity results from MTT assay and live Incucyte imaging.** (a) Confluence change over time of human GSC7 cells transduced or non-transduced with CMV or SSE-7-driven TKv5-P2A-mCherry in presence of 200µM GCV, 0.2% DMSO (vehicle control) or 20% DMSO (positive control). (b) Summary of MTT assay data in GSC7 cells in described conditions. Each dot represents biological replicate. MTT values were normalized to vehicle control. Error bars represent SD (N=3). (c) Live images of GSC7 cells at day 3 and day 8 demonstrating cell number and morphological changes in cells transduced with constructs in presence of 200µM GCV. (d) Live images of huFB170 cells at day 17 demonstrating cell number and morphological changes in cells transduced with constructs in presence of 200µM GCV. (e) Summary of MTT assay data in huFB170 cells in described conditions. Each dot represents biological replicate. MTT values were normalized to vehicle control (N=1).

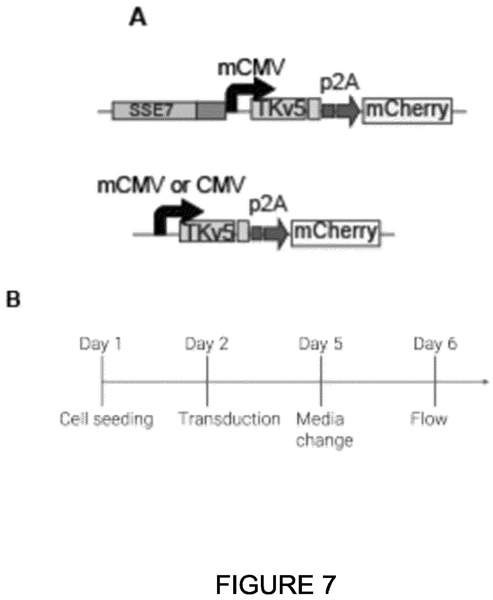

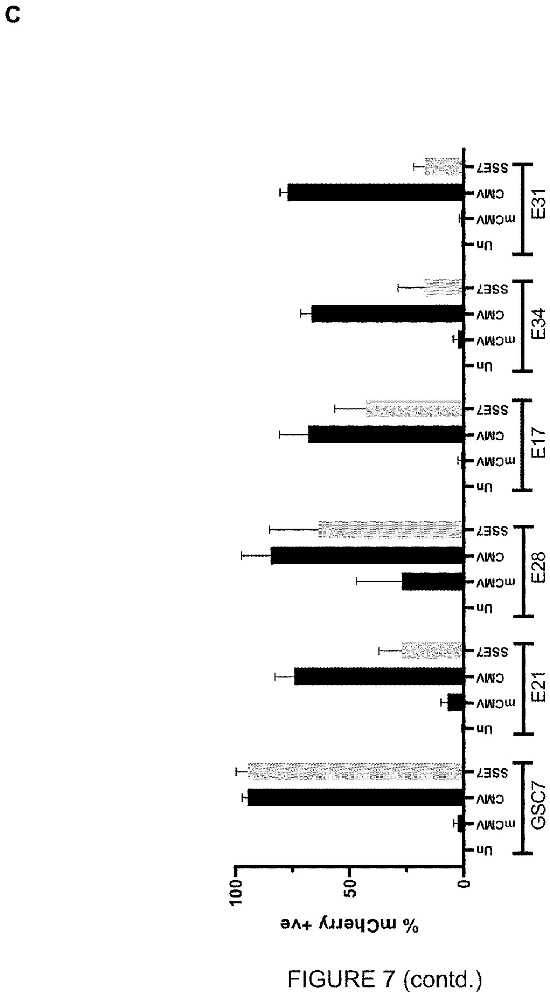

**FIGURE 7****: Activity of SSE-7 in a range of GSCs.** (A) Schematic of the expression cassette. (B) Experimental outline, (C) Results of % mCherry expression for individual cells across the various cell lines tested. GSC7 was the positive control cell line tested (from which the fragments were initially screened). E17, E28 (previously termed G328), E21, E31, E34 refers to the five additional patient-derived glioblastoma cell lines.

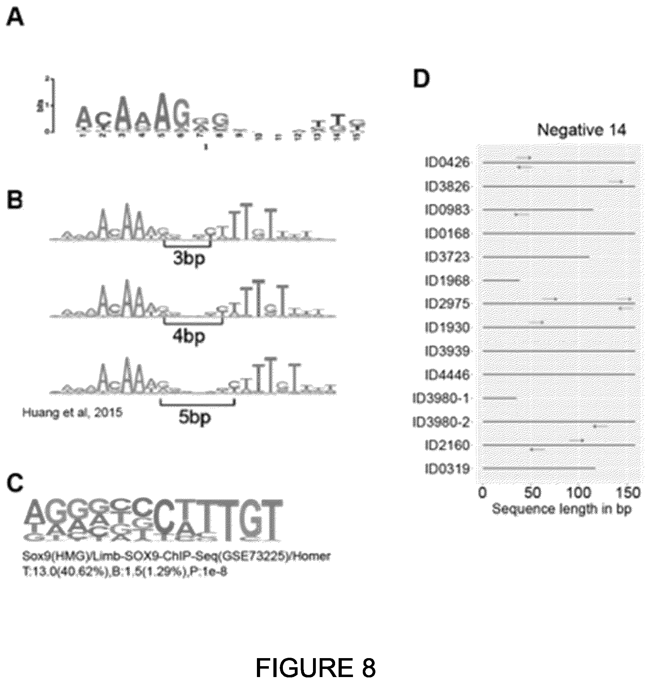

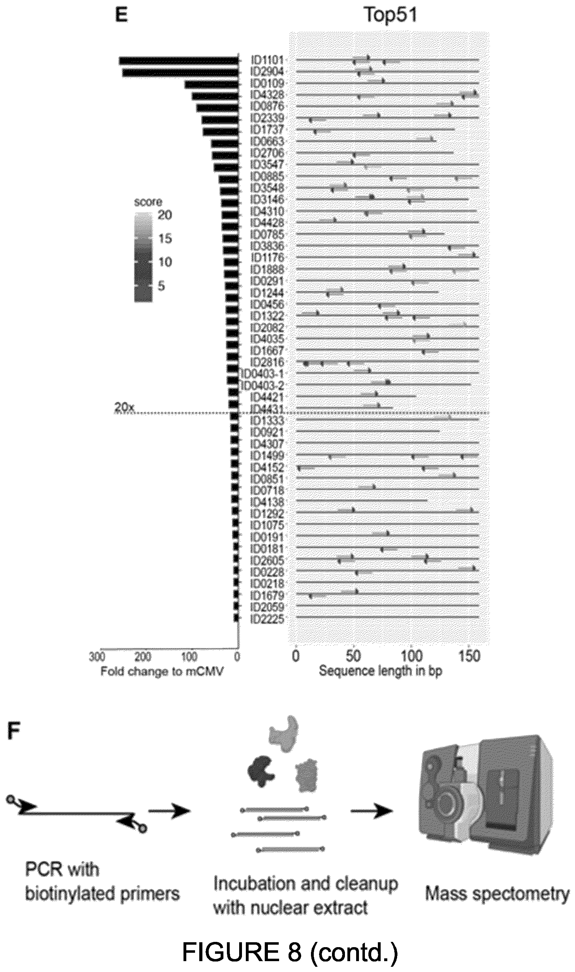

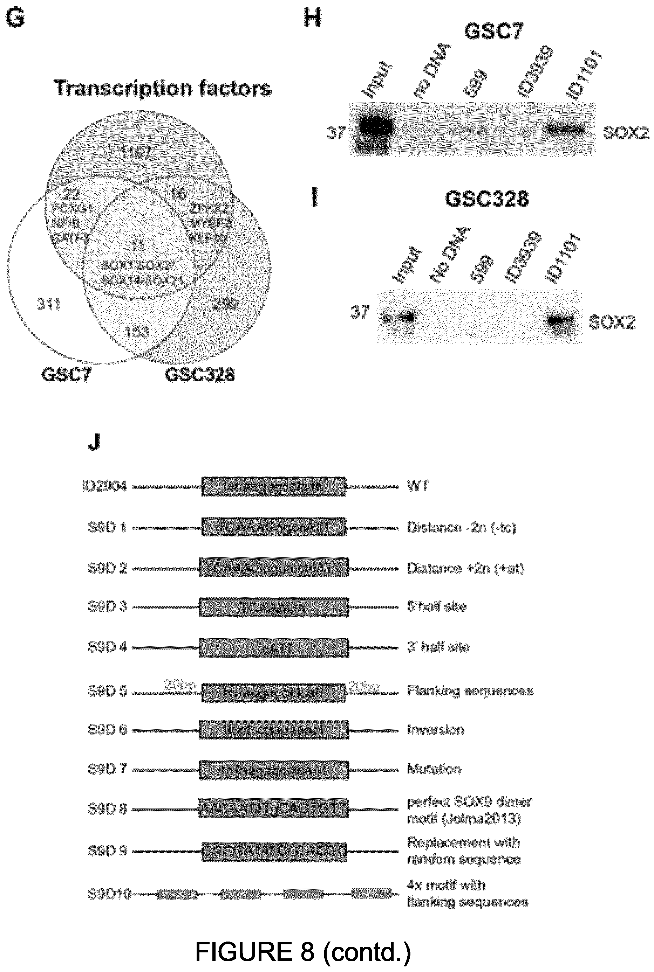

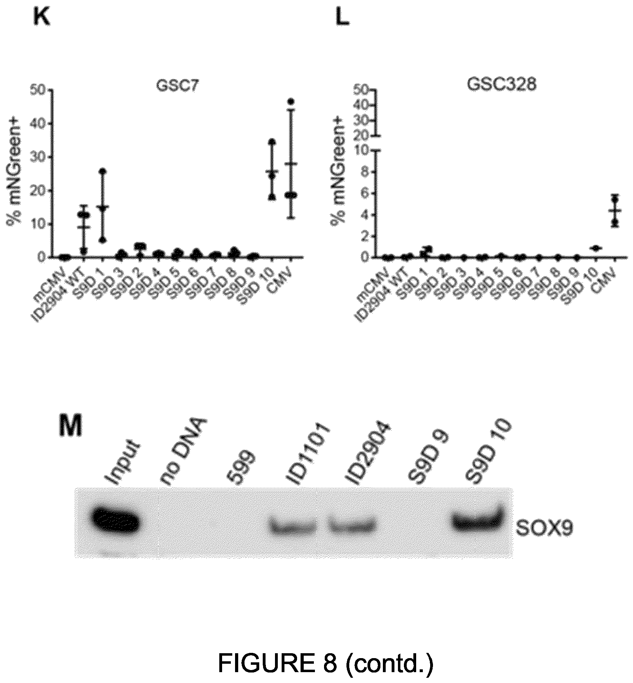

**FIGURE 8****: A SOX dimer motif is a major contributor to activity and is bound by both SOX2 and SOX9.** (A) SOX dimer motif as identified by MEME in our data set being enriched in the top 32 active fragments. (B) Dimeric SOXE motif from the TRANSFAC database (Huang et al. 2015). (C) Strongest motif found in known motifs by HOMER. (D) Mapping of the SOX dimer motif found in our dataset in putative enhancers that lacked transcriptional activity. (E) Mapping of the SOX dimer motif from (A) to the Top51 positive hits of the 1^{st} generation screen confirms this motif is found at higher frequency. The activity of the fragments is shown on the left side. (F) DNA was PCR-amplified with biotinylated primers and incubated with nuclear extract. The pulled down elution was analysed by mass spectrometry to identify bound proteins. (G) Summary of proteins pulled down by ID1101 (top circle representing all human transcription factors). SOX2 is a shared target between two cell lines to interact with ID1101. Within the 11 shared hits across GSC7 and GSC328 are three proteins of interest (NOC2L, and DMAP1, which are both components of histone acetyltransferase complexes; TFIID, a key component of the basal transcription machinery); several SOX family member proteins were also isolated. SOX2 interacts selectively with ID1101 in (H) GSC7 and (I) GSC328. (J) SOX dimer motif variants (S9D1-10) of the wild-type (WT) sequence used to investigate activity. WT sequence included as SEQ ID NO: 83, S9D1 - S9D2 are included as SEQ ID NOs: 84-85, and S9D6-S9D9 are included as SEQ ID NOs: 86-89. Activity of SOX dimer motif mutants in (K) GSC7 and (L) GSC328. (M) SOX9 selectively interacts with ID1101 and ID2904. Sequence 599 is a random DNA negative control.

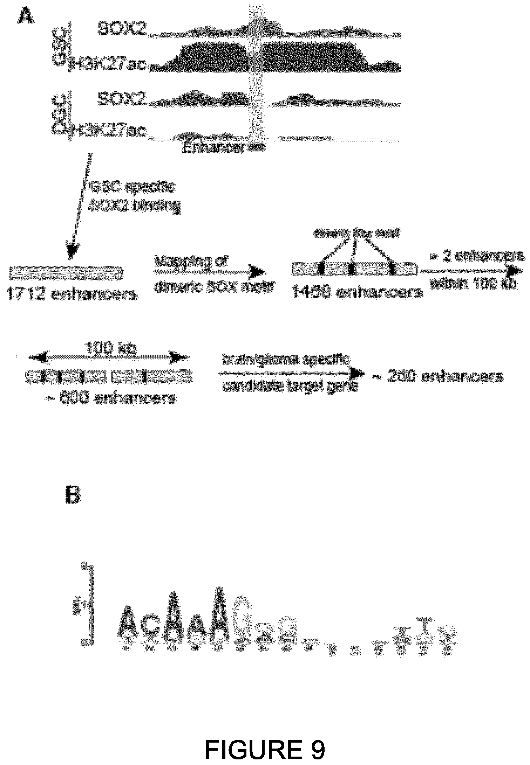

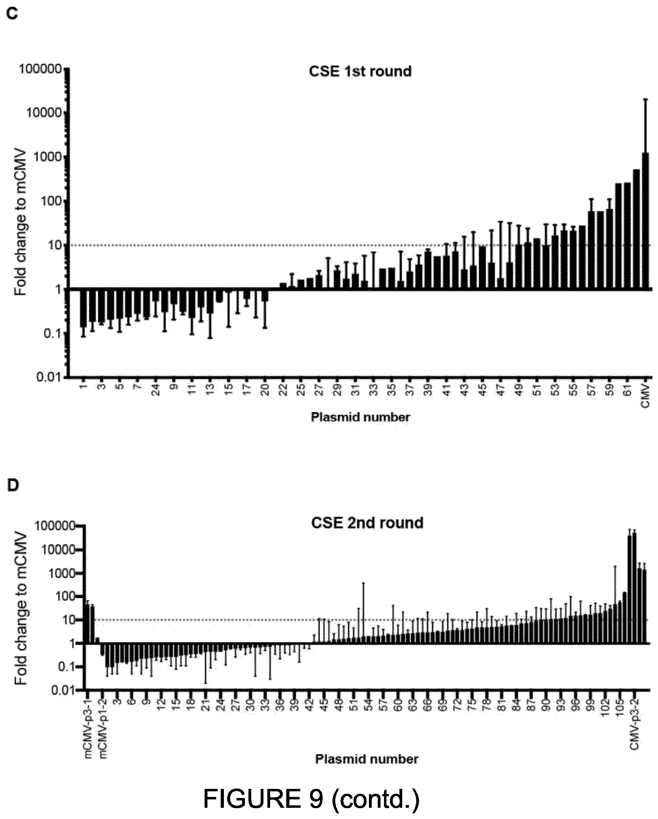

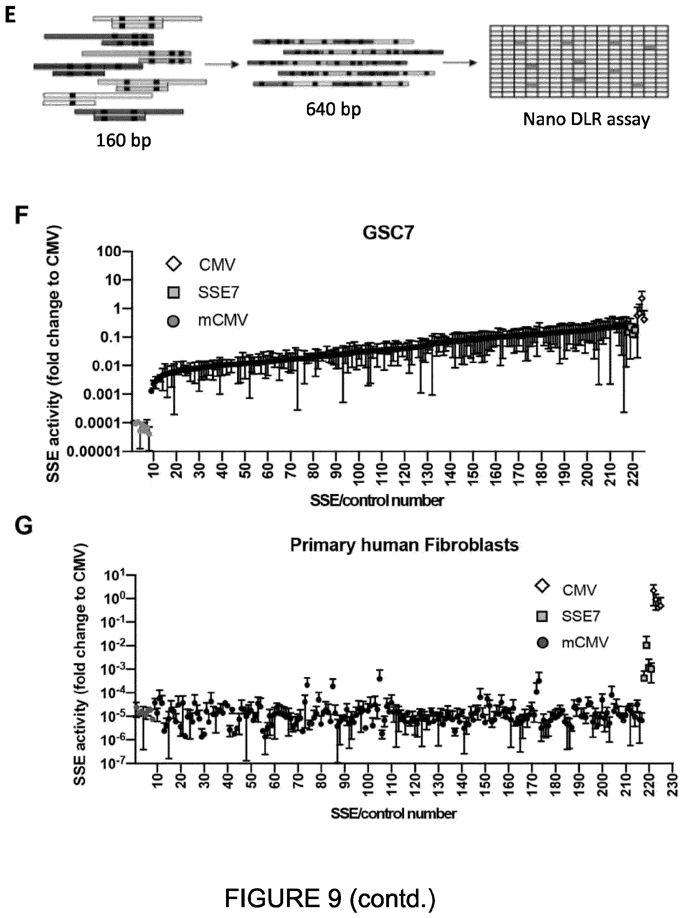

**FIGURE 9****: A second generation screen of enhancers enriched in the SOX dimer motif that have putative target genes with brain or glioma affiliated expression was performed.** (A) Selection of candidate enhancer for the screening of the second generation. (B) SOX dimer motif. (C) First and (D) second screen to identify active enhancers based on SOX dimer motif and candidate target genes. (E) Schematics of assembly of 2nd generation SSE and subsequent measurement of using a reporter gene assay. (F) Screen across 209 second generation SSE in (F) GSC7 and (G) huFb170.

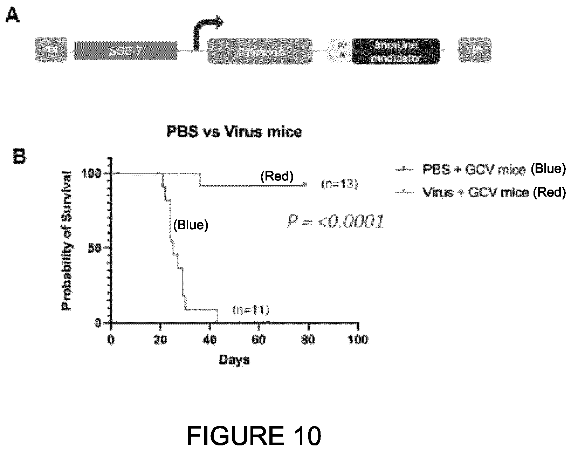

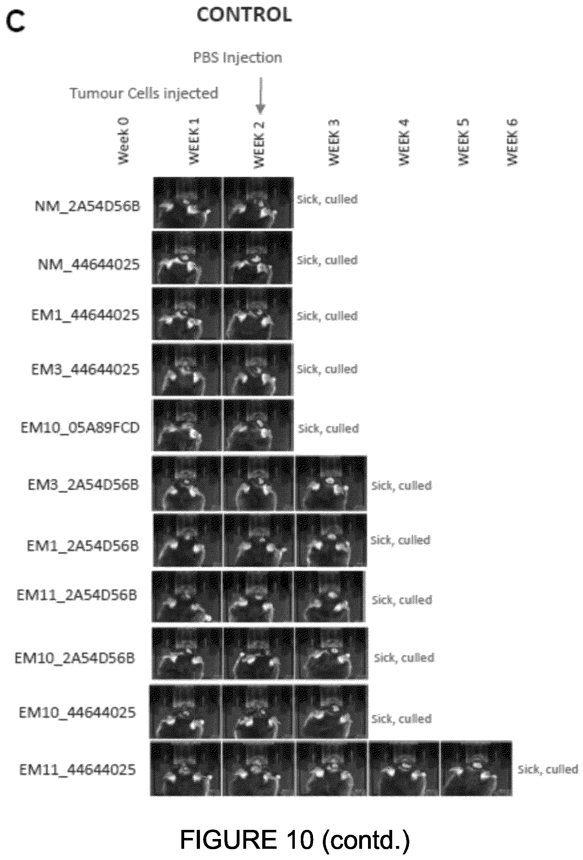

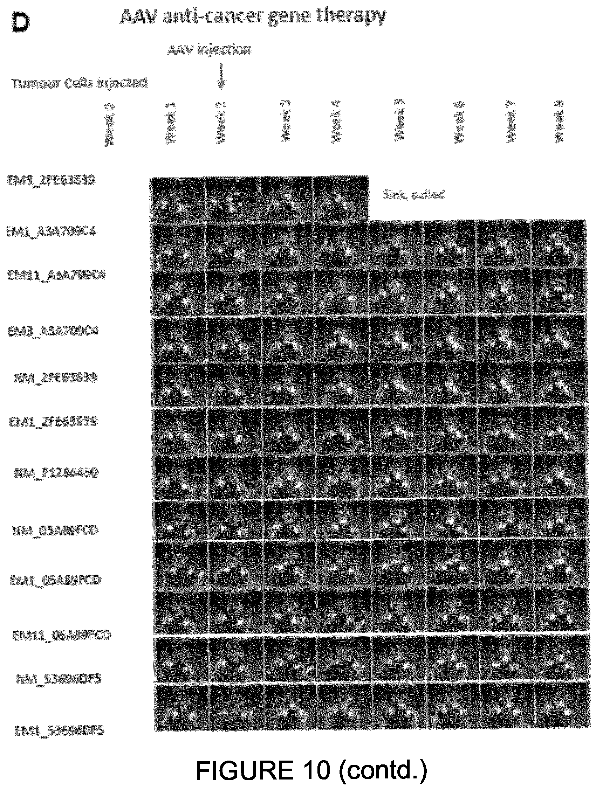

**FIGURE 10****: Significant survival and tumour clearance observed with exemplary SSE-7 with dual payload.** GBM stem cell selective expression of a cytotoxic and immune modulatory payload demonstrates proof-of-concept for the selective clearance of tumour cells in vivo. (A) Schematic of the AAV design, with SSE-7 driving dual expression of a cytotoxic enzyme/prodrug payload (HSV-TK) together with the immunostimulatory cytokine IL-12. These are generated on a single cistron (via P2A) controlled by SSE-7, ensuring combined expression. (B) Kaplan-Meier survival curves demonstrates the significant survival advantage of the gene therapy using SSE-7 and long term viability and lack of toxicities of the treated mice. (C and D) Bioluminescence imaging of live mice enables tracking of tumour growth. (C) In a immunocompetent mouse model of GBM, the tumours grow rapidly and kill mice within 3-4 weeks. (D) If tumours are treated by direct injection of the AAV with SSE-driven payload, the tumours are completely with several weeks, with no detectable tumour cells >5 weeks after all control untreated mice shown in (C) had died with large tumour masses.

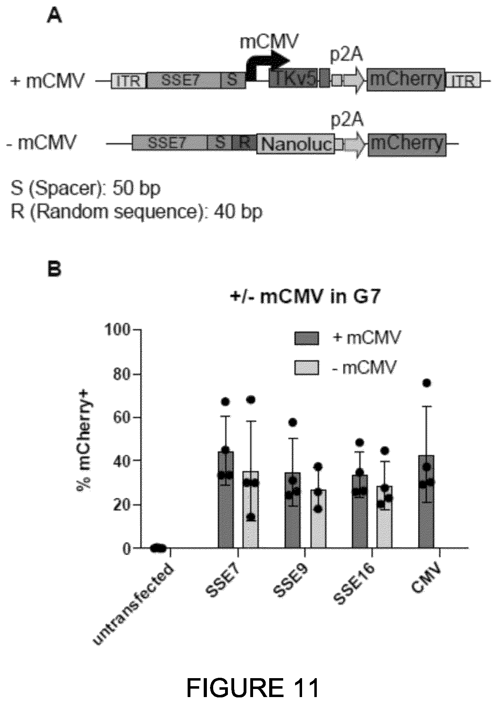

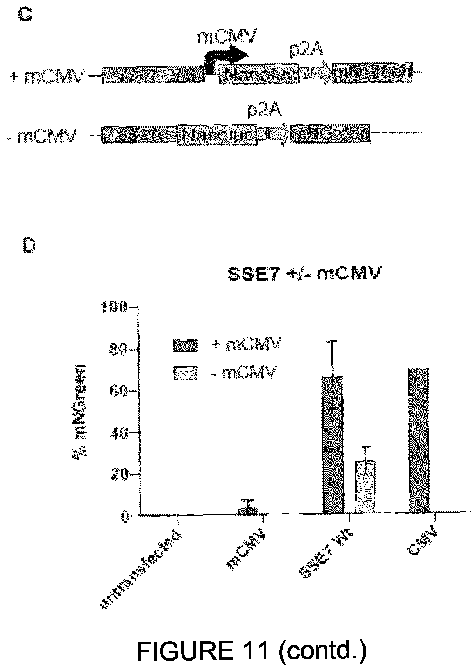

**Figure 11****: The mCMV promoter is not needed for the SSE activity.** (A) Schematic of the vector designs used to explore the requirements for mCMV promoter tethered to SSEs, with the mCMV replaced by a random DNA sequence. (B) Flow cytometry analysis of the SSE with and without mCMV confirms that the SSE remains functional and is highly active. (C) Schematic of the vector design used to explore the requirements for the random DNA sequence, serving as a spacer to position the SSE upstream from the transcriptional start site. (D) Flow cytometry analysis of activity confirms that the SSE can still function without the additional random sequence for positioning, but with reduced levels of activity.

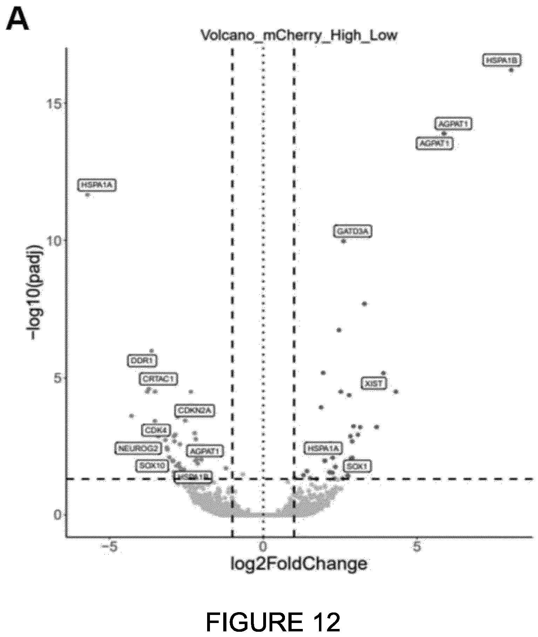

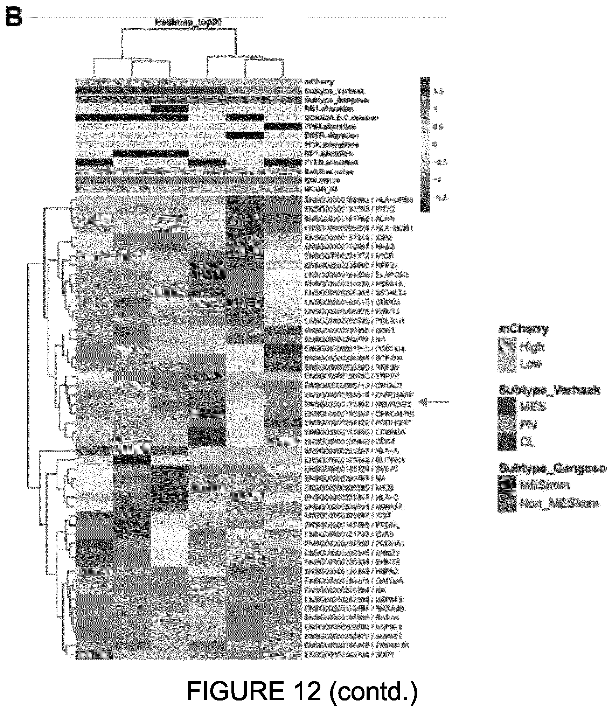

**Figure 12****: Variability of SSE-7 activity between patient GSCs correlates with levels of neuronal differentiation markers, such as *NEUROG2.*** SSE constructs disclosed herein are highly specific to immature GBM stem cell-like cells. Activity of the SSEs decreases as the cells begin to enter alternative differentiation pathways, such as NEUROG2 driven neuronal differentiation or SOX10 driven oligodendrocyte differentiation. (A) Volcano plot showing the differentially expressed genes identified by RNA-seq profiling of the SSE-7-mCherry reporter high and low cells. The lower expressing cells have enriched expression of key transcription factors that mark the exit from the stem cell state into differentiation. By contrast the highest expressing have increased levels of the SOX1, which is the most closely related to SOX2. (B) Heatmap of RNA-seq gene expression patterns for the top 50 differentially expressed genes from (A). Arrowhead notes *NEUROG2.*

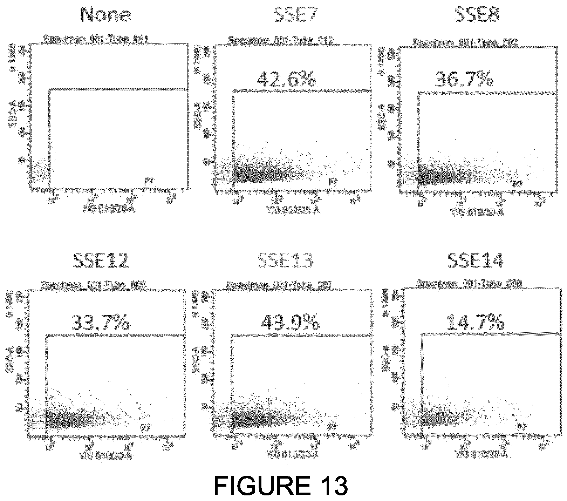

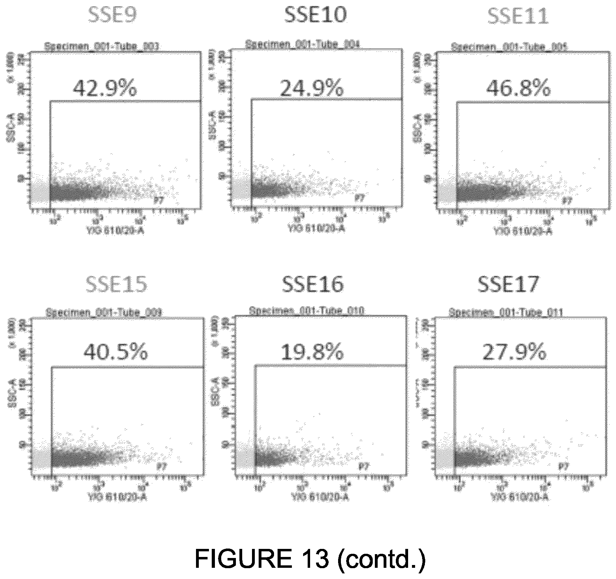

**Figure 13****: A range of 2^{nd} generation SSEs all demonstrate activity in mouse GSCs.** Flow cytometry to assess the activity of SSEs comprising enhancer fragments enriched for the SOX motifs and SOX dimer motif, confirms that these can all drive high levels of expression - comparable to full-length CMV - in GSCs.

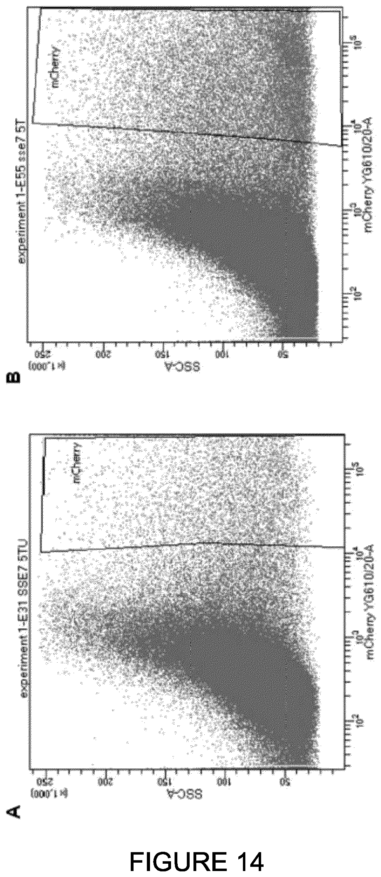

**Figure 14****: Exemplary SSE-7 also has good activity when deployed with lentivirus. Flow cytometry quantitation of levels of mCherry reporter in GSCs transduced with lentivirus harbouring SSE-7-mCherry reporters.** High levels of expression are achieved for lentivirus with SSE-7, which demonstrates that transgenes may be delivered using a broad range of viral vectors, using two different GSC patient lines, E31 and E55, in (A) and (B), respectively.

### DETAILED DESCRIPTION

The present disclosure relates to novel (synthetic) super-enhancers (S)SEs) and provides an innovative way of delivering transgenes, such as therapeutic and adverse payloads. Building upon advances in knowledge of cell type-specific gene regulation, the inventors reasoned it might be possible to create SSEs by artificially (or synthetically) assembling enhancers that retain the core functional elements and transcription factor motifs within full length enhancers. This may include utilizing the natural and functional flanking sequence surrounding the direct transcription factor motifs in these enhancers. This strategy aims to enable retention of the natural local transcription factor (TF) binding context for the key cell type-specific TFs (low affinity with appropriate spacing), but facilitates high concentrations of TF binding to trigger strong transcription, i.e., capturing the appropriate grammar (spacing, affinity and orientation of co-binding proteins) of the TF motifs within cell type specific enhancers, but boosting transcriptional activity by combining in multi-part arrays. As described herein, it was found that construction of SSEs using combinatorial assembly of functionally annotated enhancers, results in strong, yet highly cell type selective regulatory elements that will have many applications, most notably in gene therapies.

### Synthetic super-enhancers

According to a first aspect, there is provided a super-enhancer activated by one or more transcription factors, wherein the super-enhancer comprises two or more enhancer sequences derived from different genomic loci, wherein each enhancer sequence comprises a binding site for the transcription factor. In another teaching, there is provided a synthetic super-enhancer activated by one or more transcription factors, wherein the synthetic super-enhancer comprises two or more enhancer sequences derived from different genomic loci, wherein each enhancer sequence comprises a binding site for the transcription factor and between 20 and 400 nucleotides upstream and/or downstream of the binding site for the transcription factor present in its genomic locus. In a further teaching, there is provided a super-enhancer construct activated by one or more transcription factors, wherein the super-enhancer construct comprises two or more enhancer sequences derived from different genomic loci, wherein each enhancer sequence comprises a binding site for the transcription factor.

As used herein, "enhancer" refers to a DNA sequence (for example an "enhancer sequence") which is bound by proteins (for example, transcription factors) to enhance transcription of a gene. The term "enhancer" may embrace the specific short region (usually 50-1500 bp) of DNA which is bound by, for example, a transcription factor. Nevertheless, an enhancer sequence may further comprise part of the sequence which flanks or surrounds the direct transcription factor motifs. Enhancers typically act as cis-regulatory elements but may also act in trans, and may activate more than one target gene. Due to DNA folding and conformation or through promotion of multi-molecular compartments (referred to as transcriptional hubs or condensates, which may form by phase separation) they can be located distally from the transcription start site (TSS) of the gene being regulated. In contrast, promoters are upstream of the TSS on the same DNA sequence proximal to the site of transcriptional initiation. Promoters will be bound by RNA polymerase II in order to initiate transcription.

H3K27Ac is a histone modification commonly found at enhancers and can be used to predict regions of enhancer activity. Determination of whether a sequence enhances gene transcription (and therefore may be referred to as an "enhancer") can be determined using methods known in the art, most typically using a plasmid based reporter assay.

Super-enhancers are also involved in the activation or regulation of transcription. In nature, the term "super-enhancer" refers to a sequence that contains clusters of multiple enhancers, usually spanning a region of 5-15kb. These "super-enhancers" are highly occupied by transcriptional coactivators, such as the Mediator complex, compared to the average enhancers. They exhibit greater activity than average enhancers, and are frequently involved in driving expression of cell type-specific genes involved in maintaining cell identity or disease state (see, for example, US2014/0296218 and US2014/0287932, which are herein incorporated by reference in their entirety). It will be understood that reference herein to a "super-enhancer", "synthetic super-enhancer" (or "SSE") or super-enhancer construct refers to a super-enhancer which is not found in nature, i.e. is an artificially constructed super-enhancer designed for the purposes described herein, which may exhibit features associated with natural super-enhancers. In particular, an SSE is an artificial construct comprising two or more enhancer sequences (which may be derived from different genomic loci). Again, and for the avoidance of doubt, the term "SSE" will be used to embrace any of the super-enhancer, synthetic super-enhancer and/or super-enhancer constructs described herein. The SSE may be highly enriched for transcription factor motifs to stimulate transcriptional activation. This enables SSE sequences to be shorter than natural super-enhancers because the high concentration of transcription factor binding to the SSE promotes transcriptional activity.

In one embodiment, the present disclosure provides an innovative way of delivering a transgene by coupling it to an SSE that is activated by a transcription factor - or unique combination of transcription factors - expressed in a target cell. Therefore, endogenous expression of the transcription factor(s) in the target cell is used to initiate expression of the exogenous transgene in a highly selective manner. Preferably, the transcription factor has cell type or lineage-specific expression patterns and is therefore minimally expressed in cells other than the target cell, i.e., it is not ubiquitously expressed. This enables cell-type or cell state selective activation of the SSE, and expression of the transgene thereby minimising off-target toxicity. In one embodiment, the transcription factor is differentially expressed in the target cell, i.e. compared to other cell types. The use of an SSE also provides high expression of the transgene, preferably approaching or equivalent to the expression of the transgene when using a strong promoter, such as the full-length cytomegalovirus (CMV) promoter. SSEs are highly selective, strong and may be small in size (preferably less than 500 base pairs). These provide a 'switch' in gene expression for associated open reading frames/transgenes from minimal expression to very strong expression depending on the cell type identity or state.

Enhancer and super-enhancer function may involve the presence of a promoter, such as a minimal promoter (e.g. minimal CMV), which contains key regions to facilitate recruitment of transcriptional machinery and typically contains a TSS. In an alternative embodiment, a SSE of this disclosure may not comprise a promoter and /or a minimal CMV promoter.

As used herein, the term "transcription factor" refers to a protein that binds to a regulatory element of a target gene to modulate, e.g. increase or decrease, expression of the target gene. Transcription factors (TFs) are often grouped based upon the type of DNA binding domain present in the TF. In eukaryotes, the most common DNA binding domains are zinc fingers, homeodomains, basic leucine zippers, and basic helix-loop-helix domains. TF paralogs with the same DNA binding domain often recognize similar DNA sequences. The synthetic super-enhancers described herein may be bound by one or more transcription factors, i.e. one or more transcription factors bind to the SSE which results in activation. Activation occurs via recruitment of RNA polymerase II, and thereby activates expression of an associated gene (i.e. the transgene), enhancing transcription above the level of expression by a promoter alone. Binding of the transcription factor to the SSE may include the associated transcriptional and chromatin regulatory machinery.

In one embodiment, the transcription factor is a developmental or stem-cell affiliated transcription factor. These types of transcription factors are associated with high levels of activation in stem cells or during the development of a cell.

In one embodiment, the transcription factor is a transcription factor of the SOX family. The SOX (SRY-related HMG box) gene family of transcription factors comprises 20 members in human and mouse genomes and all share a high-mobility-group (HMG) box domain. The SOX family members with high homology are classified into groups: (SoxA, SoxB1, SoxB2, SoxC, SoxD, SoxE, SoxF, SoxG and SoxH). In one embodiment, the transcription factor is a SoxB1 (i.e. SOX1, SOX2, SOX3) or SoxE (i.e. SOX8, SOX9, SOX10) transcription factor.

*SOX2* has emerged as critical regulator of cancer stem cells (Bulstrode et al. 2017, Gangemi et al. 2009, Guerra-Rebollo et al. 2019, Lujan et al. 2012, D. K. Singh et al. 2017, S. K. Singh et al. 2004, and Boumahdi et al. 2014). ChlP-seq datasets for glioblastoma stem cells (GSCs) and their differentiated progeny have been reported by Suvà et al. 2014 who catalogued histone H3 enhancer marks (H3K27ac) marks and SOX2 binding unique to the GSCs. In particular, the present inventors have identified a set of candidate SOX2-regulated enhancers operational in GSCs across diverse patient-derived lines. Therefore, in one embodiment, the transcription factor is SOX2.

Transcription factors bind by virtue of the DNA binding domain of the transcription factor binding to a specific motif (i.e. transcription factor binding site) in the target enhancer. Typically, a transcription factor binding site sequence is 5-15 bp long.

In one embodiment, the synthetic super-enhancer (SSE) comprises at least one SOX dimer motif. Such motifs may be important for the function of an SSE in a target cell, for example many of the enhancer sequences described herein were shown to contain SOX dimer sites that were required to retain activity in glioblastoma stem cells. Enhancer elements can contain SOX binding sites (sometimes palindromic sequences) where SOX transcription factors may homo- or heterodimerise. Typically, the spacing between each individual monomer binding motif would be around 8-12bp.

In one embodiment, the SOX dimer motif comprises SEQ ID NO: 1:
ACAAAGRGSVBYTKK
where:
R represents A or G; S represents C or G; V represents A, C or G; B represents C, G or T; Y represents C or T; and K represents G or T.

In a further embodiment, the SOX dimer motif comprises SEQ ID NO: 2:
RRRRASARAGRRRBBHDDBWH
where:
R represents A or G; S represents C or G; B represents C, G or T; H represents A, C or T; D represents A, G or T; and W represents A or T.

In one embodiment, the synthetic super-enhancer comprises at least one SOX motif. In a further embodiment, the SOX motif is a SOX2 motif.

In one embodiment, the SOX2 motif comprises SEQ ID NO: 3:
WSARAGRSMYMHTBB
where:
W represents A or T; S represents C or G; R represents A or G; M represents A or C; Y represents C or T; H represents A, C or T; B represents C, G or T.

In one embodiment, wherein the synthetic super-enhancer comprises a sequence selected from: a SOX motif and/or a SOX dimer motif.

As explained herein, a super-enhancer refers to a cluster of enhancer sequences. Therefore, in one embodiment, the synthetic super-enhancer comprises two or more enhancer sequences. In a further embodiment, the synthetic super-enhancer comprises between two and eight enhancer sequences. In a yet further embodiment, the synthetic super-enhancer comprises four enhancer sequences.

Enhancer elements may be identified using techniques known in the art. For example, the methods described herein used ChIP-seq datasets for specific cell types to identify enhancers bound by transcription factors of interest or use of chromatin accessibility assays (e.g. ATAC-seq or MNase profiling). Publicly available resources are available to assist with genome-wide identification of active enhancers in various organisms and cell types, such as ENCODE or The Cancer Genome Atlas (TCGA) datasets.

Preferably, the enhancer sequences present in the SSE do not all have the same sequence (i.e. the SSE is not a concatemer of the same enhancer element). Therefore, in one embodiment, the SSE comprises at least two different enhancer sequences. In one embodiment, each of the enhancer sequences has a different sequence. In one embodiment, each of the enhancer sequence is activated by the same transcription factor(s).

The data presented herein shows that assembly of enhancers derived from different genomic loci into 4-part arrays (~640 bp) surprisingly led to orders of magnitude increases in their activity without compromising selectivity. Therefore, each of the enhancer sequences used in the SSE may be derived from different genes. In a further embodiment, each of the enhancer sequences is derived from different genomic loci. These enhancer sequences are all still preferably activated by a common transcription factor or set of cell type-affiliated transcription factor(s). In one embodiment, each of the enhancer sequences is activated by the transcription factor(s) and is derived from different genomic loci. In one embodiment, the synthetic super-enhancer comprises two or more, such as four enhancer sequences, derived from different genomic loci.

In one embodiment, each of the enhancer sequence comprises a binding site for the transcription factor and between 20 and 400 nucleotides upstream and/or downstream of the binding site for the transcription factor present in its genomic locus. This allows the context surrounding (i.e. directly upstream and/or downstream) the transcription factor binding site to be included in the enhancer sequence and therefore retain the enhancer function. The upstream and/or downstream sequences typically present in natural enhancer sequences that retain the spacing, order, orientation and/or affinity may be useful for recruitment of cooperating transcription factors and co-factors. In addition, the sequence context surrounding the transcription factor binding motif is likely to facilitate binding and act cooperatively to enhance transcription efficiency. A skilled person in the art would be capable of identifying/obtaining a sequence between 20 and 400 nucleotides upstream and/or downstream of the binding site of interest from a publicly available genome database. That same skilled person could determine transcription factor activity through functional analyses, such as reporter assays as described herein or other methods widely adopted in the art. In one embodiment, each enhancer sequence comprises a binding site for the transcription factor and between 20 and 400 nucleotides upstream and/or downstream of the binding site for the transcription factor present in its genomic locus, wherein the upstream and/or downstream sequence is sufficient to ensure the enhancer retains its function. In a further embodiment, the enhancer sequence comprises between 20 and 400 nucleotides upstream and/or downstream of the binding site for the transcription factor, such as between 20 and 300 nucleotides upstream and/or downstream of the binding site for the transcription factor or preferably between 20 and 200 nucleotides upstream and/or downstream of the binding site for the transcription factor. In a further embodiment, the enhancer sequence comprises between 50 and 400 nucleotides upstream and/or downstream of the binding site for the transcription factor, such as between 50 and 300 nucleotides upstream and/or downstream of the binding site for the transcription factor or preferably between 50 and 200 nucleotides upstream and/or downstream of the binding site for the transcription factor. In a further embodiment, the enhancer sequence comprises between 80 and 400 nucleotides upstream and/or downstream of the binding site for the transcription factor, such as between 80 and 300 nucleotides upstream and/or downstream of the binding site for the transcription factor or preferably between 80 and 200 nucleotides upstream and/or downstream of the binding site for the transcription factor.

In one embodiment, each of the enhancer sequences within the synthetic super-enhancer are less than 500 nucleotides long, such as less than 450, 400, 350, 300, 250 or 200 nucleotides long. In a further embodiment, each of the enhancer sequences within the SSE are less than 300 nucleotides long. In one embodiment, each of the enhancer sequences within the synthetic super-enhancer are between 20 and 500 nucleotides long, such as between 50 and 250 nucleotides long or between 100 and 200 nucleotides long.

It will be understood that these embodiments may be combined. Therefore, in a further embodiment, the enhancer sequence comprises between 20 and 400 nucleotides upstream and/or downstream of the binding site for the transcription factor, wherein the enhancer sequence is less than 500 nucleotides long. In a yet further embodiment, the enhancer sequence comprises between 20 and 200 nucleotides upstream and/or downstream of the binding site for the transcription factor, wherein the enhancer sequence is less than 300 nucleotides long.

In a further embodiment, each of the enhancer sequences within the SSE are about 160 nucleotides long. In particular, 160 base pairs (bp) was chosen as this provides sufficient size for nucleosome binding and is considered to be an important attribute in the evolution of the grammar within functional enhancers. Moreover, 160 bp is a convenient size for synthesis of a pooled oligonucleotide library that incorporates 20 bp ends for subsequent construction of an arrayed plasmid library.

In one embodiment, the synthetic super-enhancer is less than 2000 nucleotides long. In a further embodiment, the synthetic super-enhancer is less than 1500 nucleotides long, such as less than 1400, 1300, 1200, 1100, 1000, 950, 900, 850, 800, 750 or 700 nucleotides long. In one embodiment, the synthetic super-enhancer is less than 1200 nucleotides long. In a further embodiment, the synthetic super-enhancer is less than 700 nucleotides long. Preferably, the synthetic super-enhancer is less than 650 nucleotides long, such as 640 nucleotides long.

The motifs described herein may be present in one or more of the enhancers of the SSE (for example, see Figure 8E). Therefore, in one embodiment, one or more of the enhancers comprise a sequence selected from: a SOX motif and/or a SOX dimer motif.

In one embodiment, each of the enhancer sequences within the synthetic super-enhancer comprises a SOX motif, such as a SOX2 motif. In one embodiment, each of the enhancer sequences within the synthetic super-enhancer comprises a SOX dimer motif.

In one embodiment, the synthetic super-enhancer is activated by SOX2, wherein the synthetic super-enhancer comprises four enhancer sequences derived from different genomic loci and wherein each of the enhancer sequences comprises at least one SOX dimer motif and/or at least one SOX dimer motif. In one embodiment, the synthetic super-enhancer is activated by SOX2, wherein the synthetic super-enhancer comprises four enhancer sequences derived from different genomic loci and wherein each of the enhancer sequences comprises at least one SOX2 motif. In one embodiment, the synthetic super-enhancer is activated by SOX2, wherein the synthetic super-enhancer comprises four enhancer sequences derived from different genomic loci and wherein each of the enhancer sequences comprises at least one SOX dimer motif.

In one embodiment, the one or more enhancer sequences comprise a sequence with at least 80% sequence identity to a sequence selected from the group consisting of SEQ ID NO: 4-63 (Table 1). In a further embodiment, the one or more enhancer sequences comprise a sequence with at least 80% sequence identity to a sequence selected from the group consisting of SEQ ID NO: 4-13 and 56-63. In a yet further embodiment, the one or more enhancer sequences have at least 85%, 90%, 95%, 97%, 99% sequence identity or are 100% identical to a sequence selected from the group consisting of SEQ ID NO: 4-63 (such as SEQ ID NO: 4-13 and 56-63). The enhancer sequences described herein (i.e. Table 1) may be modified by at least one (such as less than 20, less than 10, less than 5) nucleotide substitution, deletion or addition, wherein the variant enhancer sequence substantially retains the functional characteristics of the sequence. It will be understood that variants of the enhancer sequences provided herein are still intended to retain the functional activity of the described enhancer sequences. For example, Example 4 shows that the described enhancer sequences had a more than 10-fold increase over mCMV when tested using the Nanoglo DLR assay. Therefore, the variant enhancer sequences included within this scope should not have an activity that is less than 75% of the activity of the starting sequence.

In one embodiment, the synthetic super-enhancer comprises one or more enhancer sequences presented in Table 1. Therefore, the SSE may comprise one or more enhancer sequences selected from the group consisting of SEQ ID NO: 4-63. Data is presented herein that shows arrangement of these enhancer sequences in multi-part arrays can increase transcriptional activity, without compromising cell specificity. In a further embodiment, the synthetic super-enhancer comprises between one and eight, between two and six, or between three and five enhancer sequences selected from the sequences presented in Table 1. In a yet further embodiment, the synthetic super-enhancer comprises four enhancer sequences selected from the sequences presented in Table 1.

In one embodiment, the SSE comprises one or more enhancer sequences selected from the group consisting of SEQ ID NO: 4-36. In an alternative embodiment, the SSE comprises one or more enhancer sequences selected from the group consisting of SEQ ID NO: 37-63, more preferably SEQ ID NO: 54-63.

In one embodiment, the SSE comprises one or more enhancer sequences selected from the group consisting of SEQ ID NO: 4-13 and 54-63.

In a further embodiment, the SSE comprises four enhancer sequences selected from the group consisting of SEQ ID NO: 4-36, such as 4-13, in particular 4-8. In an alternative embodiment, the SSE comprises four enhancer sequences selected from the group consisting of SEQ ID NO: 37-63, preferably four enhancer sequences selected from the group consisting of SEQ ID NO: 54-63, more preferably four enhancer sequences selected from the group consisting of SEQ ID NO: 56-63.

The SSE may comprise a sequence as described in Table 2. In one embodiment, the synthetic super-enhancer comprises a sequence with at least 80% sequence identity to a sequence selected from the group consisting of SEQ ID NO: 64-80. In a further embodiment, the sequence has at least 85%, 90%, 95%, 97%, 99% sequence identity or is 100% identical to a sequence selected from the group consisting of SEQ ID NO: 64-80. In a yet further embodiment, the SSE comprises SEQ ID NO: 70. Variants of the sequences provided herein are still intended to retain the functional activity of the described SSE, i.e. transgene expression upon activation of the SSE by the transcription factor.

For the purposes of comparing two closely-related polynucleotide sequences, the "% sequence identity" between a first nucleotide sequence and a second nucleotide sequence may be calculated using NCBI BLAST v2.0, using standard settings for nucleotide sequences (BLASTN). For the purposes of comparing two closely-related polypeptide sequences, the "% sequence identity" between a first polypeptide sequence and a second polypeptide sequence may be calculated using NCBI BLAST v2.0, using standard settings for polypeptide sequences (BLASTP).

Polypeptide or polynucleotide sequences are said to be the same as or "identical" to other polypeptide or polynucleotide sequences, if they share 100% sequence identity over their entire length. Residues in sequences are numbered from left to right, *i.e.* from N- to C-terminus for polypeptides; from 5' to 3' terminus for polynucleotides.

A "difference" between sequences refers to an insertion, deletion or substitution of a single amino acid residue or nucleotide in a position of the second sequence, compared to the first sequence. Two polynucleotide sequences can contain one, two or more such nucleotide differences. Two polypeptide sequences can contain one, two or more such amino acid differences. Insertions, deletions or substitutions in a second sequence which is otherwise identical (100% sequence identity) to a first sequence result in reduced % sequence identity.

Alternatively, for the purposes of comparing a first, reference sequence to a second, comparison sequence, the number of additions, substitutions and/or deletions made to the first sequence to produce the second sequence may be ascertained. An "addition" is the addition of one amino acid residue/nucleotide into the first sequence (including addition at either terminus of the first sequence). A "substitution" is the substitution of one amino acid residue/nucleotide in the first sequence with one different amino acid residue/nucleotide. With respect to polypeptide sequences, a substitution may be conservative or non-conservative. In the context of polynucleotide sequences, a substitution may be synonymous or nonsynonymous. A "deletion" is the deletion of one amino acid residue/nucleotide from the first sequence (including deletion at either terminus of the first sequence).

In one embodiment, the synthetic super-enhancer additionally comprises a transcriptional regulator, i.e. a sequence (or sequence encoding a protein) that controls gene expression.

According to another aspect, there is provided a synthetic super-enhancer activated by one or more transcription factors of the SOX family, wherein the synthetic super-enhancer comprises two or more enhancer sequences and at least one SOX motif and/or SOX dimer motif. It will be understood that the embodiments described herein that apply to the synthetic super-enhancer of the functional nucleic acid molecule, also apply to this aspect.

According to a further aspect, there is provided a synthetic super-enhancer activated by SOX2, wherein the synthetic super-enhancer comprises four enhancer sequences derived from different genomic loci and wherein each of the enhancer sequences comprises at least one SOX motif and/or SOX dimer motif.

### Functional nucleic acid molecules

The SSE may be present in a construct comprising additional elements forming a functional nucleic acid molecule. Therefore, according to a further aspect, there is provided a functional nucleic acid molecule comprising the synthetic super-enhancer as described herein, operably linked to a transgene.

According to a further aspect, there is provided a functional nucleic acid molecule comprising a synthetic super-enhancer activated by one or more transcription factors expressed in a target aberrant cell and an adverse payload, wherein said payload is expressed upon activation of the synthetic super-enhancer by the transcription factor(s).

The functional nucleic acid molecule may comprise additional components to the SSE and transgene. For example, the synthetic super-enhancer may be operably linked to a promoter. Suitable promoters are known in the art. In one embodiment, the promoter is a minimal CMV promoter. In one teaching the SSE may not be operably linked to a promoter. In an alternative embodiment, the promoter operably linked to the super-enhancer is not a minimal CMV promoter.

### Transgenes

As described herein, the SSEs may be used to enhance the expression of a transgene. In one embodiment, the transgene may be an exogenous sequence for expression in a cell. In one teaching, the transgene is a sequence encoding a product which is useful in biology and medicine, such as a prophylactic or a therapeutic transgene, e.g. protein or non-protein encoding oligonucleotide. Therefore, the transgene may encode a therapeutic payload. For example, the therapeutic payload may include: a therapeutic protein, such as an antigen, antibody, cytokine (e.g. to induce an immunogenic response), onco-suppressor protein (e.g. natural/unmutated p53), a differentiation factor (i.e. to modulate or reprogram cell fate or identity), or a protein with nucleic acid editing or gene activity modulation function (e.g. DNA or RNA editing, or transcriptional regulation) or to otherwise alter cellular phenotypes (e.g. motility, ECM production, cellular senescence or quiescence). In an alternative embodiment, the transgene encodes a non-protein encoding oligonucleotide, such as an RNA (other than mRNA), for example miRNA, siRNA, shRNA or IncRNA. It will be understood that transgenes encoding non-coding RNAs may be used in methods of gene silencing.

In one embodiment, the functional nucleic acid molecule comprises one or more transgenes. Therefore, it will be understood to a person skilled in the art that different combinations of transgenes may be included.

According to a further aspect, there is provided a method of expressing a transgene in a target cell, which comprises expressing a transgene operatively linked to a synthetic super-enhancer as described herein wherein one or more of said transcription factors are expressed in the target cell.

### Adverse payloads

As described herein, the transgene operably linked to the SSE may encode an adverse payload. The term "adverse payload" as used herein refers to a payload that has a negative impact upon the target cell. The negative impact may be, for example, a negative effect on the health or viability of the cell and/or the ability of the cell to divide. The adverse effect may be achieved directly (e.g. the transgene encodes a harmful substance to the cell, such as a proapoptotic gene or a suicide gene) or indirectly (e.g. the transgene encodes a substance that recruits an external factor that causes a harmful effect on the cell).

The adverse payload may be used to stimulate an immune response having a negative impact upon the target cell. Such a response may, for example, alter the local immune microenvironment. In one embodiment, the adverse payload encodes a protein that stimulates an immune response that leads to the activation of cytotoxic immune cells. In one embodiment, the adverse payload is selected from: a chemokine, cytokine, antibody or other immune modulatory protein. In a further embodiment, the adverse payload is a pro-inflammatory cytokine, such as a cytokine selected from: IL-12, IL-10, IL-2, IFN-α and GM-CSF. For example, the adverse payload may be a cytokine such as IL-12 which has been shown to have anticancer activity. IL-12 cause induction of IFN-γ production by resting and activated CD4+ T cells, CD8+ T cells, and natural killer (NK) cells, as well as enhancing the proliferation of activated T and NK cells, increasing the lytic activity of NK/lymphokine-activated killer cells, and facilitating specific cytotoxic T lymphocyte (CTL) responses. However, IL-12 cannot be administered as a systemic treatment due to excessive toxicity, therefore a targeted therapy where expression only occurs within a target cell would enable the delivery of this payload.

An "immune response" is a measurable change in at least one cell, or one cell-type, or one endocrine pathway, or one exocrine pathway, of the immune system (including but not limited to a cell-mediated response, a humoral response, a cytokine response, a chemokine response).

An "immune cell" is defined as a cell of the immune system including, but not limited to, CD34+ cells, B-Cells, CD45+ (lymphocyte common antigen) cells, alpha-beta T-cells, cytotoxic T-cells, helper T-cells, plasma cells, neutrophils, monocytes, macrophages, dendritic cells, phagocytes, granulocytes, innate lymphoid cells, Natural Killer (NK) cells and gamma delta T-cells. Typically, immune cells are classified with the aid of combinatorial cell surface molecule analysis (e.g., via flow cytometry) to identify or group or cluster to differentiate immune cells into sub-populations. In molecules and methods of the disclosure where the payload is an adverse payload, the stimulated immune response is intended to adversely affect the target cell. Reference to "cytotoxic immune cell" refers to immune cells that result in cell death, in particular cytotoxic T cells (also known as killer T cells).

In one embodiment, the adverse payload encodes a cytotoxic substance (i.e. a cytotoxic payload).

In one embodiment, the adverse payload is a suicide gene. Suicide genes are genes that express a protein that causes cell death. Alternatively, a suicide gene may require an externally supplied co-factor or co-drug (e.g. a prodrug) in order to work. The co-factor or co-drug may then be converted by the product of the suicide gene into a cytotoxic entity. In one embodiment, the suicide gene encodes a protein capable of converting an inactive prodrug into a cytotoxic drug. The inactive prodrug may be administered concomitantly or sequentially to the functional nucleic acid molecule.

In one embodiment, the suicide gene is herpes simplex virus thymidine kinase (HSV-TK). In particular, the HSV-TK gene is used in combination with the prodrug, ganciclovir (GCV) or an analogue thereof such as acyclovir and valacyclovir. In an alternative embodiment, the suicide gene is cytosine deaminase (CD). In particular, the CD gene is used in combination with the prodrug 5-fluorocytosine (5FC).

### Target cells

The functional nucleic acid molecules described herein can be used to target a variety of cell types. In particular, the cell is a mammalian cell, such as a human cell. The disclosure may include the presence of an adverse payload, therefore is suited to target aberrant cells (i.e. an abnormal cell, such as a non-healthy cell).

The aberrant cell may be a hyperproliferative cell, i.e. a cell with excessive, abnormal proliferation. In one embodiment, the aberrant cell is a cancer cell or a neoplastic cell. In this embodiment, the adverse payload may be an anti-cancer payload.

SOX2 may also be amplified in other aberrant cells, particularly cancer cells, because it has been identified as an oncogene. Examples of other aberrant cells that may be targeted using methods of the disclosure include squamous cell carcinomas of the lung and oesophagus, as well as many others.

In one embodiment, the aberrant cell is a cancer cell selected from: a glioblastoma stem cell, a glioma cell, a lung cancer cell (in particular squamous lung cancer cell), an oesophageal cancer cell, an ovarian cancer cell, a breast cancer cell, an oral cancer cell (e.g. mouth, tongue, pharynx), a stomach cancer cell, a small intestine cancer cell, a colorectal cancer cell, a rectal cancer cell, a liver cancer cell, a bile duct cancer cell, a gall bladder cancer cell, a pancreatic cancer cell, a bone cancer cell (e.g. osteosarcoma), a skin cancer cell, a uterine cancer cell, a prostate cancer cell, a testicular cancer cell, a bladder cancer cell, a kidney cancer cell, a retinal cancer cell, a thyroid cancer cell, a lymphoma cell, a myeloma cell or a leukaemia cell.

In a particular embodiment, the aberrant cell is a glioblastoma stem cell. Glioblastomas are an aggressive form of glioma that is driven by elevated levels/activity of master regulatory neural stem cell-associated transcription factors. Glioblastoma stem cells (GSCs) often have elevated expression or activity of key neurodevelopmental transcription factors, including SOX2, which is a key master regulator and reprogramming factor required for neural stem cell identity (Bulstrode et al. 2017, Gangemi et al. 2009, Guerra-Rebollo et al. 2019, Lopez-Bertoni et al. 2015, and MacLeod et al. 2019). Knockdown of *SOX2* in GSCs reduces their tumorigenicity (Gangemi et al. 2009), whereas its ectopic expression together with POU3F2, OLIG2 and SALL2 enforces the GSC state (Suvà et al. 2014). The Examples presented herein show that GSC-selective SSEs retained activity in adeno-associated viruses (AAVs) and were used to drive expression of cytotoxic payloads for targeted killing of GSCs. Furthermore, the activity of SSEs was extinguished during differentiation of GSCs and absent in HEK or fibroblast cells. Therefore, GSC selective expression was retained even though the strength of the promoter has been massively increased.

### Vectors

According to a further aspect, there is provided a vector comprising the synthetic super-enhancer or functional nucleic acid molecule as described herein.

The term "vector", as used herein, is intended to refer to a molecule capable of transporting the functional nucleic acid molecule. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g. bacterial vectors having a bacterial origin of replication and episomal mammalian and yeast vectors). Other vectors (e.g. non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids.

Exemplary expression vectors are known in the art and may include, for example, plasmid vectors, viral vectors (for example adenovirus, adeno-associated virus, retrovirus or lentivirus vectors), phage vectors, cosmid vectors and the like. The choice of expression vector may be dependent upon the type of host cell to be used and the purpose of use.

In one embodiment, the vector is a viral vector. Viral vectors, particularly those used in gene therapy applications, are well known in the art. Such viruses may be RNA and DNA viruses with either single-stranded (ss) or double-stranded (ds) genomes. For example, viral vectors include, but are not limited to, adenoviruses, adeno-associated viruses (AAV), alphaviruses, flaviviruses, herpes simplex viruses (HSV), measles viruses, rhabdoviruses, retroviruses, lentiviruses, Newcastle disease virus (NDV), poxviruses and picornaviruses. Insert capacity and tropism can vary, therefore a viral vector may be chosen based upon the intended application.

AAV vectors are increasingly favoured as an optimal gene therapy viral vector, as these have improved safety. However, a limitation is their smaller cargo size. The SSE 4-part assemblies described herein ideally suited to AAV. Therefore, in one embodiment, the vector is an AAV. Different AAV serotypes differ in their tropism, therefore the type of AAV vector used can be chosen depending upon the type of tissue targeted. The AAV vector may be selected from a number of serotypes known in the art, for example any one of AAV1, AAV2, AAV3, AAV4, AAV5, AAV6, AAV7, AAV8, AAV9, AAV10 or AAV11, or may be a non-natural variant. In one embodiment, the AAV is selected from: AAV1, AAV2 or AAV5.

According to a further aspect, there is provided a vector comprising the synthetic super-enhancer as described herein and a transgene (i.e. a payload, such as a therapeutic payload). As described herein, the synthetic super-enhancer may be operably linked to other expression elements, such as a promoter.

### Compositions and kits

The present disclosure also relates to compositions comprising the functional nucleic acid molecules or vectors described herein. The composition may comprise components which enable delivery of said functional nucleic acid molecules by viral vectors (AAV, lentivirus and the like) and non-viral vectors (nanoparticles, lipid particles and the like).

According to a further aspect, there is provided a composition comprising the synthetic super-enhancer, functional nucleic acid molecule or the vector as described herein, and an acceptable carrier. Suitable carriers are known in the art, In certain embodiments, the carrier is selected based upon its ability to facilitate the transfection of a target cell with one or more functional nucleic acid molecules.

According to a further aspect, there is provided a composition comprising the functional nucleic acid molecule or the vector as described herein, for use in therapy.

According to a further aspect, there is provided a composition comprising the functional nucleic acid molecule or the vector as described herein, for use in the treatment of cancer.

According to a further aspect, there is provided the use of the functional nucleic acid molecule (or vector or composition) as defined herein for the manufacture of a medicament, particularly for the treatment of cancer.

According to a further aspect, there is provided a kit comprising the functional nucleic acid molecule as described herein, wherein the adverse payload is a suicide gene which encodes a protein capable of converting an inactive prodrug into a cytotoxic drug. Therefore, in this aspect, the kit additionally comprises the inactive prodrug (i.e. for administration with the functional nucleic acid molecule).

### Treatment methods

According to a further aspect, there is provided a method for treating a disease comprising administering the functional nucleic acid molecule, the vector or the composition according as described herein, to a patient.

References to "subject", "patient" or "individual" refer to a subject, in particular a mammalian subject, to be treated. Mammalian subjects include humans, non-human primates, farm animals (such as cows), sports animals, or pet animals, such as dogs, cats, guinea pigs, rabbits, rats or mice. In some embodiment, the subject is a human. In alternative embodiments, the subject is a non-human mammal, such as a mouse.

As used herein, "treating" a disease or disorder means reducing the frequency and/or severity of at least one sign or symptom of the disease or disorder experienced by a subject.

In one embodiment, the disease is cancer. "Cancer," as used herein, refers to the abnormal growth or division of cells. Generally, the growth and/or life span of a cancer cell exceeds, and is not coordinated with, that of the normal cells and tissues around it. Cancers may be benign, pre-malignant or malignant. Cancers may also be primary cancers or secondary cancers. A primary cancer is the original organ or tissue where the cancer began, whereas a secondary cancer is the result of the primary cancer spreading (or metastasising) to another site in the body.

Cancer occurs in a variety of cells and tissues, including the oral cavity (e.g. mouth, tongue, pharynx), digestive system (e.g. oesophagus, stomach, small intestine, colon, rectum, liver, bile duct, gall bladder, pancreas), respiratory system (e.g. larynx, lung, bronchus), bones, joints, skin (e.g. basal cell, squamous cell, meningioma), breast, genital system (e.g. uterus, ovary, prostate, testis), urinary system (e.g. bladder, kidney, ureter), eye, nervous system (e.g. brain), endocrine system (e.g. thyroid), and hematopoietic system (e.g. lymphoma, myeloma, leukaemia, acute lymphocytic leukaemia, chronic lymphocytic leukaemia, acute myeloid leukaemia, chronic myeloid leukaemia).

In a further embodiment, the cancer is a cancer located in the brain or spinal cord (i.e. the central nervous system), such as a primary or secondary cancer located in the central nervous system. The disclosure finds particular use in the treatment of glioblastoma.

The functional nucleic acid molecule (or vector or composition) may be administered by any suitable mode of delivery for the intended treatment, such as intravenously, intraarterially, intracardially, intracutaneously, subcutaneously, interperitoneally, intramuscularly or orally. In one embodiment, the functional nucleic acid molecule, vector or composition is administered systemically. Administration may also be direct to the site of disease. Therefore, in one embodiment, the functional nucleic acid molecule, vector or composition is administered locally, e.g. directly into the organ or tissue, such as intratumorally.

According to a further aspect, there is provided a method for treating glioblastoma comprising administering a functional nucleic acid molecule and an inactive prodrug to a patient,
wherein the functional nucleic acid comprises a synthetic super-enhancer activated by a SOX transcription factor and a suicide gene which encodes a protein capable of converting the inactive prodrug into a cytotoxic drug, and
wherein the suicide gene is expressed upon activation of the synthetic super-enhancer by the SOX transcription factor in glioblastoma cells.

In one embodiment, the inactive prodrug is administered concomitantly or sequentially to the functional nucleic acid molecule.

It will be understood that methods of treatment will involve administration of a therapeutically effective amount. The term "therapeutically effective amount" is an amount that is effective to ameliorate or treat a symptom of a disease or disorder. A therapeutically effective amount can be a "prophylactically effective amount" as prophylaxis can be considered therapy.

It will be understood that the embodiments described herein may be applied to all aspects of the disclosure, *i.e.* the embodiment described for the uses may equally apply to the claimed methods and so forth.

### CLAUSES

A set of clauses defining the disclosure and its preferred aspects is as follows:
1. A synthetic super-enhancer activated by one or more transcription factors, wherein the synthetic super-enhancer comprises two or more enhancer sequences derived from different genomic loci, wherein each enhancer sequence comprises a binding site for the transcription factor and between 20 and 400 nucleotides upstream and/or downstream of the binding site for the transcription factor present in its genomic locus.
2. The synthetic super-enhancer according to clause 1, wherein each of the two or more enhancer sequences within the synthetic super-enhancer are less than 300 nucleotides long, in particular 160 nucleotides long.
3. The synthetic super-enhancer according to clause 1 or clause 2, wherein the synthetic super-enhancer is less than 1200 nucleotides long, in particular less than 700 nucleotides long.
4. The synthetic super-enhancer according to any one of clauses 1 to 3, wherein the synthetic super-enhancer comprises four enhancer sequences.
5. The synthetic super-enhancer according to any one of clauses 1 to 4, wherein the transcription factor is a transcription factor of the SOX family.
6. The synthetic super-enhancer according to any one of clauses 1 to 5, wherein the synthetic super-enhancer comprises at least one SOX dimer motif.
7. The synthetic super-enhancer according to clause 6, wherein the SOX dimer motif comprises SEQ ID NO: 1.
8. The synthetic super-enhancer according to any one of clauses 1 to 5, wherein the synthetic super-enhancer comprises at least one SOX motif.
9. The synthetic super-enhancer according to clause 8, wherein the SOX motif is a SOX2 motif.
10. The synthetic super-enhancer according to clause 8 or clause 9, wherein the SOX2 motif comprises SEQ ID NO: 3.
11. The synthetic super-enhancer according to any one of clauses 1 to 10, wherein the synthetic super-enhancer comprises one or more enhancer sequences presented in Table 1.
12. The synthetic super-enhancer according to any one of clauses 1 to 11, wherein the synthetic super-enhancer comprises a sequence with at least 80% sequence identity to a sequence selected from the group consisting of SEQ ID NO: 64-80.
13. The synthetic super-enhancer according to any one of clauses 1 to 12, wherein the synthetic super-enhancer additionally comprises a transcriptional regulator.
14. A functional nucleic acid molecule comprising the synthetic super-enhancer according to any one of clauses 1 to 13, operably linked to a transgene.
15. The functional nucleic acid molecule according to clause 14, wherein the transgene encodes a therapeutic payload.
16. The functional nucleic acid molecule according to clause 14, wherein the transgene encodes an adverse payload.
17. The functional nucleic acid molecule according to clause 16, wherein the adverse payload encodes a protein that stimulates an immune response that leads to the recruitment of cytotoxic immune cells.
18. The functional nucleic acid molecule according to clause 16, wherein the adverse payload encodes a cytotoxic substance.
19. The functional nucleic acid molecule according to any one of clauses 1 to 18, wherein the adverse payload is a suicide gene which encodes a protein capable of converting an inactive prodrug into a cytotoxic drug.
20. The functional nucleic acid molecule according to any one of clauses 14 to 19, wherein the synthetic super-enhancer is operably linked to a promoter.
21. A functional nucleic acid molecule comprising a synthetic super-enhancer activated by one or more transcription factors expressed in a target aberrant cell and an adverse payload, wherein said payload is expressed upon activation of the synthetic super-enhancer by the transcription factor(s).
22. The functional nucleic acid molecule according to clause 21, wherein the transcription factor is differentially expressed in the target aberrant cell.
23. The functional nucleic acid molecule according to clause 21 or clause 22, wherein the transcription factor is a developmental or stem-cell affiliated transcription factor.
24. The functional nucleic acid molecule according to any one of clauses 21 to 23, wherein the transcription factor is a transcription factor of the SOX family.
25. The functional nucleic acid molecule according to any one of clauses 21 to 24, which is activated by SOX2.
26. The functional nucleic acid molecule according to any one of clauses 21 to 25, wherein the synthetic super-enhancer comprises at least one SOX dimer motif.
27. The functional nucleic acid molecule according to clause 26, wherein the SOX dimer motif comprises SEQ ID NO: 1.
28. The functional nucleic acid molecule according to any one of clauses 21 to 25, wherein the synthetic super-enhancer comprises at least one SOX motif.
29. The functional nucleic acid molecule according to clause 28, wherein the SOX motif is a SOX2 motif.
30. The functional nucleic acid molecule according to clause 28 or clause 29, wherein the SOX2 motif comprises SEQ ID NO: 3.
31. The functional nucleic acid molecule according to any one of clauses 21 to 30, wherein the synthetic super-enhancer comprises two or more enhancer sequences.
32. The functional nucleic acid molecule according to any one of clauses 21 to 31, wherein the synthetic super-enhancer comprises four enhancer sequences.
33. The functional nucleic acid molecule according to clause 31 or clause 32, wherein each of the two or more enhancer sequences has a different sequence.
34. The functional nucleic acid molecule according to any one of clauses 31 to 33, wherein each of the two or more enhancer sequences is derived from different genomic loci.
35. The functional nucleic acid molecule according to any one of clauses 31 to 34, wherein each of the two or more enhancer sequences within the synthetic super-enhancer are less than 300 nucleotides long, in particular about 160 nucleotides long.
36. The functional nucleic acid molecule according to any one of clauses 31 to 35, wherein each of the two or more enhancer sequences within the synthetic super-enhancer comprise a sequence selected from: a SOX motif and/or SOX dimer motif.
37. The functional nucleic acid molecule according to any one of clauses 21 to 36, wherein the synthetic super-enhancer comprises one or more enhancer sequences presented in Table 1.
38. The functional nucleic acid molecule according to any one of clauses 21 to 37, wherein the synthetic super-enhancer comprises a sequence with at least 80% sequence identity to a sequence selected from the group consisting of SEQ ID NO: 64-80.
39. The functional nucleic acid according to any one of clauses 21 to 38, wherein the synthetic super-enhancer is operably linked to a promoter.
40. The functional nucleic acid molecule according to any one of clauses 21 to 39, wherein the adverse payload encodes a protein that stimulates an immune response that leads to the recruitment of cytotoxic immune cells.
41. The functional nucleic acid molecule according to any one of clauses 21 to 39, wherein the adverse payload encodes a cytotoxic substance.
42. The functional nucleic acid molecule according to any one of clauses 1 to 41, wherein the adverse payload is a suicide gene which encodes a protein capable of converting an inactive prodrug into a cytotoxic drug.
43. The functional nucleic acid molecule according to clause 42, wherein the suicide gene is a herpes simplex virus thymidine kinase (HSV-TK) gene and the prodrug is ganciclovir, acyclovir or valacyclovir.
44. The functional nucleic acid molecule according to any one of clauses 21 to 43, wherein the aberrant cell is a cancer cell or a neoplastic cell and the adverse payload is an anti-cancer payload.
45. The functional nucleic acid molecule according to any one of clauses 21 to 44, wherein the aberrant cell is a glioblastoma stem cell.
46. A synthetic super-enhancer activated by one or more transcription factors of the SOX family, wherein the synthetic super-enhancer comprises two or more enhancer sequences and at least one SOX motif and/or SOX dimer motif.
47. The synthetic super-enhancer according to clause 46, wherein each of the two or more enhancer sequences has a different sequence.
48. The synthetic super-enhancer according to clause 46 or clause 47, wherein each of the two or more enhancer sequences is derived from different genomic loci.
49. The synthetic super-enhancer according to any one of clauses 46 to 48, wherein the synthetic super-enhancer comprises four enhancer sequences.
50. The synthetic super-enhancer according to any one of clauses 46 to 49, wherein each of the two or more enhancer sequences within the synthetic super-enhancer are less than 300 nucleotides long, in particular 160 nucleotides long.
51. The synthetic super-enhancer according to any one of clauses 46 to 50, wherein each of the two or more enhancer sequences within the synthetic super-enhancer comprise a sequence selected from: a SOX motif and/or SOX dimer motif.
52. The synthetic super-enhancer according to any one of clauses 46 to 51, which is activated by SOX2.
53. The synthetic super-enhancer according to clause 52, wherein the SOX dimer motif comprises SEQ ID NO: 1.
54. The synthetic super-enhancer according to any one of clauses 46 to 51, wherein the SOX motif is a SOX2 motif.
55. The synthetic super-enhancer according to clause 54, wherein the SOX2 motif comprises SEQ ID NO: 3.
56. The synthetic super-enhancer according to any one of clauses 46 to 55, wherein the synthetic super-enhancer comprises one or more enhancer sequences presented in Table 1.
57. The synthetic super-enhancer according to any one of clauses 46 to 56, wherein the synthetic super-enhancer comprises a sequence with at least 80% sequence identity to a sequence selected from the group consisting of SEQ ID NO: 64-80.
58. A synthetic super-enhancer activated by SOX2, wherein the synthetic super-enhancer comprises four enhancer sequences derived from different genomic loci and wherein each of the enhancer sequences comprises at least one SOX dimer motif.
59. A vector comprising the synthetic super-enhancer according to any one of clauses 1 to 13 or 46-58, or the functional nucleic acid molecule according to any one of clauses 14 to 45.
60. The vector according to clause 59, wherein the vector is a viral vector.
61. The vector according to clause 59 or clause 60, wherein the vector is an adeno-associated virus (AAV).
62. A composition comprising the synthetic super-enhancer according to any one of clauses 1 to 13 or 46-58, the functional nucleic acid molecule according to any one of clauses 14 to 45 or the vector according to any one of clauses 59 to 61, and an acceptable carrier.
63. A composition comprising the functional nucleic acid molecule according to any one of clauses 14 to 45, for use in therapy.
64. A composition comprising the functional nucleic acid molecule according to any one of clauses 14 to 45, for use in the treatment of cancer.
65. A kit comprising the functional nucleic acid molecule according to any one of clauses 21 to 45, wherein the adverse payload is a suicide gene which encodes a protein capable of converting an inactive prodrug into a cytotoxic drug, and the kit additionally comprises said inactive prodrug for administration with the functional nucleic acid molecule.
66. A method for treating cancer comprising administering the functional nucleic acid molecule according to any one of clauses 14 to 45 or the composition according to clauses 63 or 64, to a patient.
67. The method according to clause 66, wherein the cancer is a brain cancer, such as glioblastoma.
68. The method according to clause 66 or clause 67, wherein the functional nucleic acid molecule or composition is administered systemically.
69. The method according to clause 66 or clause 67, wherein the functional nucleic acid molecule or composition is administered locally.
70. A method for treating glioblastoma comprising administering a functional nucleic acid molecule and an inactive prodrug to a patient,
   wherein the functional nucleic acid comprises a synthetic super-enhancer activated by a SOX transcription factor and a suicide gene which encodes a protein capable of converting the inactive prodrug into a cytotoxic drug, and
   wherein the suicide gene is expressed upon activation of the synthetic super-enhancer by the SOX transcription factor in glioblastoma cells.
71. The method according to clause 70, wherein the inactive prodrug is administered concomitantly or sequentially to the functional nucleic acid molecule.
72. A super-enhancer activated by one or more transcription factors, wherein the super-enhancer comprises two or more enhancer sequences derived from different genomic loci, wherein each enhancer sequence comprises a binding site for the transcription factor.
73. The super-enhancer according to clause 72, wherein each of the two or more enhancer sequences within the super-enhancer are less than 300 nucleotides long, in particular is about 160 nucleotides long and wherein the super-enhancer is less than 1200 nucleotides long, in particular less than 700 nucleotides long.
74. The super-enhancer according to clause 72 or clause 73, wherein the super-enhancer comprises four enhancer sequences.
75. The super-enhancer according to any one of clauses 72 to 74, wherein the super-enhancer comprises at least one SOX dimer motif and/or at least one SOX motif.
76. The super-enhancer according to clause 75, wherein the SOX dimer motif comprises SEQ ID NO: 1.
77. The super-enhancer according to clause 75, wherein the SOX motif comprises SEQ ID NO: 3.
78. The super-enhancer according to any one of clauses 72 to 77, wherein the super-enhancer comprises one or more enhancer sequences presented in Table 1.
79. The super-enhancer according to any one of clauses 72 to 78, wherein the super-enhancer comprises a sequence with at least 80% sequence identity to a sequence selected from the group consisting of SEQ ID NO: 64-80.
80. The super-enhancer according to any one of clauses 72 to 79, wherein each enhancer sequence further comprises between 20 and 400 nucleotides upstream and/or downstream of the binding site for the transcription factor present in its genomic locus.
81. A functional nucleic acid molecule comprising the super-enhancer according to any one of clauses 72 to 80, operably linked to a transgene.
82. The functional nucleic acid of clause 81, wherein the transgene encodes a gene for expression in a cell,a therapeutic payload or reprogramming factor.
83. A functional nucleic acid molecule comprising a super-enhancer activated by one or more transcription factors expressed in a target aberrant cell and an adverse payload, wherein said payload is expressed upon activation of the super-enhancer by the transcription factor(s).
84. The functional nucleic acid molecule according to clause 83, wherein the transcription factor is differentially expressed in the target aberrant cell, and optionally wherein the transcription factor is a developmental or stem-cell affiliated transcription factor.
85. The functional nucleic acid molecule according to clause 83 or clause 84, wherein the adverse payload encodes a protein that stimulates an immune response that leads to the recruitment of cytotoxic immune cells.
86. The functional nucleic acid molecule according to clause 83 or clause 84, wherein the adverse payload is a suicide gene which encodes a protein capable of converting an inactive prodrug into a cytotoxic drug.
87. A composition comprising the functional nucleic acid molecule according to any one of clauses 82 to 86, for use in the treatment of cancer, such as glioblastoma.
88. A super-enhancer activated by one or more transcription factors of the SOX family, wherein the super-enhancer comprises two or more enhancer sequences and at least one SOX motif and/or SOX dimer motif.
89. The super-enhancer according to clause 88, wherein each of the two or more enhancer sequences has a different sequence.
90. The super-enhancer according to clause 88 or clause 89, wherein each of the two or more enhancer sequences within the super-enhancer are less than 300 nucleotides long, in particular is about 160 nucleotides long.

The disclosure will now be illustrated with reference to the following non-limiting examples.

### EXAMPLES

### MATERIALS AND METHODS

### Destination vector cloning

In order to ensure high cloning efficiencies for both enhancer cloning and super-enhancer assembly, we built a new custom destination vector to enable efficient golden gate cloning. This also reduced reaction setup times/costs. This destination vector design encompassed: the reporter gene cassette Nanoluc-Ires-mNGreen-pA and a minimal CMV promoter (mCMV). The bacterial suicide ccdB cassette was used, for selection and efficient cloning. CDV2, contained a ccdB cassette spanning the positions enabled assembly of up to four enhancers and spacers. In contrast to CDV1, CDV2 also contains PiggyBac transposase recognition sites flanking the whole cassette. We used a Gibson assembly to generate a scar free custom destination vector (Gibson et al. 2009).

### Bioinformatics to design of a SOX2 enhancer oligonucleotide pool

Suvà and colleagues published technical replicates for one cell line for SOX2 binding in GSC (SOX2r1 and SOX2r2 in the MGG8 cell line, pro-neural subtype) and H3K27ac in three cell lines MGG4 (pro-neural subtype), MGG6 (classical subtype), and MGG8 (Suvà et al. 2014). They also include data for the differentiated MGG8 cell (referred to here as differentiated glioma cell (DGC)). For clarity, due to their tumorigenic properties, GSC lines are also referred to as tumour-propagating cells, in contrast to DGCs (which are derived here from GSCs via addition of serum which stimulates astrocytic differentiation).

To determine GSC-specific SOX2 peaks, GSC SOX2r1 and SOX2r2 were overlapped with DGC SOX2_H3K27ac (a H3K27ac Chip-Seq in an SOX2 overexpressing DGCs). Additionally, shared H3K27ac peaks were also identified between the GSC cell lines to define putative enhancer sequences. These shared peaks were individually overlapped with GSC specific SOX2r1 and GSC specific SOX2r2. This safeguarded against the loss of any potentially important regions. The resulting shared peaks were then combined into one file and peaks closer than 100 base pairs (bp) were merged which also removed duplicates. The length of the peaks ranged from 15 - 5300 bp with an average length of 411 bp. Next, the pool was manually curated to remove repetitive sequences and non-enhancer sequences, such as centromeres. This resulted in a curated preliminary pool comprised of 1721 peaks ranging from 15 - 3366 bp and an average length of 402 bp.

To avoid PCR biases towards small fragments we also discarded all regions smaller than 100 bp resulting in 1710 peaks ranging from 115 - 3366 bp and an average length of 404 bp. These peaks were then split into 160 bp fragments. Any fragments smaller than 100 bp were again removed to avoid PCR bias. 20 bp adapters were attached on each side.

### Cell lines

GSCs were derived using NSC culture conditions previously reported by Pollard et al. Briefly, cells were expanded in serum and feeder free conditions in a neural basal media with N2 and B27 supplements. Cells were grown adherently by supplementing the culture media with Laminin-1. GSC7 has been characterised by Stricker et al. HEK293 cells were obtained from the ATCC cell bank. All other GSC lines and huFb170 were derived in the Pollard lab (unpublished) and are being characterised as part of the Glioma Cellular Genetics Resource (www.gcgr.org.uk).

### Construction of an arrayed plasmid library of enhancers

We chose an arrayed library format, with 160 bp enhancer fragments individually cloned into separate plasmids and arrayed in 96 well plates. These 160 bp were synthesized as a single pool of oligos, then randomly cloned into the plasmid and isolated. 20 bp x 2 were needed for PCR amplification of the pool and so a library of 200 bp fragments was synthesized (Twist Biosciences). To meet our oligo synthesis strategy, ChlP-seq peaks expressed by GSCs were merged into a single oligo. This oligonucleotide pool was PCR-amplified (using limited cycles to reduce bias and 'jackpot' products meaning products which are easier to amplify will overtake the oligonucleotide pool) and cloned into the expression vector by an efficient Golden Gate reaction. 4579 individual plasmids were then randomly picked, isolated and plated as an arrayed plasmid DNA library on 96 well plates (x48). The bacterial colony picking and production of plasmid minipreps were supported by the Edinburgh Genome Foundry (S. Rosser). This library was then screened using the optimised Nanoglo DLR assay (Nano-Glo Dual-Luciferase Reporter Assay, Promega) in 384 well format. Each plate was transiently transfected together with PGK-FFLuc in a 1:10 ratio into GSC7 cells. 2 days later a Nanoglo DLR assay was performed and assayed on a plate reader.

### PCR amplification and clean-up for library production

We developed a very high efficiency of library amplification and plasmid cloning, to avoid inappropriate products, empty sequences and redundancy of the library by using a golden gate cloning and selection against the empty vector. PCR conditions were optimised to amplify the oligonucleotide pool with limited/no background and without amplification biases.

We used KAPA HiFi Hotstart Polymerase with GC buffer (Roche, KK2501) with 68°C annealing temperature and 5s extension time at 72°C. The oligonucleotide pool was first amplified for 10 cycles with 0.25 µl (2.5 ng input) and 0.5 µl of this reaction were used for the following 15 cycle amplification, in order to reduce PCR bias.

A magnetic bead-based Solid Phase Reversible Immobilization (SPRI) purification was used to minimise loss and clean up the DNA prior to golden gate cloning. Sanger sequencing of all inserts confirmed that they are diverse, and all sequences could be mapped back to a fragment of the pool and contained an adapter.

### Screening platform for 384 well plates

Cells were seeded in 384 well plates using a multidrop and transfected the following day using the CyBio Felix. Two days later the experiment was terminated with a Nanoglo DLR assay and all hits with a fold change to mCMV greater than 10 were Sanger sequenced and mapped back to the genome.

### Genomic features of validated enhancers

GREAT, an online tool, defines a basal regulatory domain for each gene as 1 kb downstream and 5 kb upstream of the transcription start site (TSS), meaning close genes are part of the same regulatory domain. To account for distal regulatory events, the basal gene regulatory domain is extended towards the nearest basal upstream and downstream regulatory domain within 1000 kb. GREAT then uses ontology of gene annotations and query sequences together with a binomial test to test for enrichment of gene ontology to predict the target gene (Mclean et al. 2010). We investigated the expression patterns in human adult tissues of the predicted target genes (https://gtexportal.org/home/).

### Oligonucleotide pull down and mass spectrometry

The oligo was PCR-amplified using biotinylated primers. The PCR product was then SPRI purified to remove biotinylated primer dimers. These dsDNA fragments were incubated with nuclear extract from GSC7 and GSC328. They were then bound to streptavidin magnetic beads, washed three times and the frozen beads were sent for mass spectrometry analysis.

### Cell culture procedures

Cell lines were cultured at 37°C in 5% CO₂. Cells were grown on uncoated cell culture plastic dishes. Glioblastoma stem cell lines were grown under serum free condition using previously reported conditions (Pollard et al., 2009). For passaging cell lines were rinsed with PBS and detached using Accutase for GSC or 0.5% Trypsin/EDTA for huFb170 and HEK293, respectively. Cells were taken up in wash media and spun down at 300 xg for 3 min. They were resuspended in their respective growth media and replated. For cryo-preservation cells were resuspended in their growth media supplemented with 10% DMSO and stored at -80°C for short-term storage. For long-term preservation vials were transferred to liquid nitrogen containers.

### Transfection of GSC, huFb170 and HEK293 cells

Cells were seeded at the required density. The following day the transfection was carried out. For this the Plus reagent and Lipofectamine LTX (Life Technologies, 15338030) were each diluted in half the volume of Opti-MEM I Reduced-Serum Medium (further referred to as Optimem) (Life Technologies, 31985062). Following this step, the Plus reagent/Optimem premix was added to all DNA samples followed by the Lipofectamine LTX/Optimem premix. The transfection mix was incubated for 5 min at room temperature and then carefully dropped on the cells. Generally, no media change was carried out. Cells were analysed two days following transfection.

HEK293 cells were seeded at the specified density in the respective plate format. The next day GMEM, DNA and PEI (homemade) are mixed and incubated for 15 min at room temperature to allow for complex formation. The transfection mix is subsequently dropped on the cells and analysis was carried out two days later.

### Nano-Glo Dual-Luciferase Reporter Assay System (Promega)

This assay consists of two steps. The transfection was carried out using a normalisation plasmid PGK-Firefly-Luciferase (Promega, E5011) that was transfected in a 1/10 ratio with the plasmid of interest. First, the Firefly Luciferase activity is measured which allows to normalise to transfection efficiency. In a second step, the activity of Nanoluc is determined which is the readout of the plasmid of interest. Cells were washed 3x with PBS and 20 µl was left in the 96 well plate after the final wash (25 µl in 384 well). Oneglo buffer was added and the plate was shaken for 5 min at 480 rpm to allow cell lysis. 20 µl cell lysate in 96 well format or 25 µl cell lysate in 384 well format was then transferred into the corresponding opaque white plate and luminescence was measured at for 0.1 s per well using the Ensight Multimode Plate Reader.

For the following Nanoluc reaction in a 96 well format 2 µl cell lysate was transferred into an opaque white plate containing 40 µl PBS, and 20 µl Stopglo buffer, supplemented with substrate, was added to each well. In a 384 well format 20 µl Stopglo buffer containing substrate were added on top of the undiluted cell lysate. The plate was shaken again for 5 min at 480 rpm to quench the firefly luciferase reaction and ensure good mixing. Nanoluc activity was measured for 0.1 s per well, again using Ensight Multimode Plate Reader.

Data obtained by the Nanoluc reaction was normalised to Firefly reaction to account for well-to-well variability of transfection efficiency. Normalised data were used to calculate the fold change to the empty vector control mCMV.

### Immunocytochemistry

Cells were washed twice with PBS and fixed in 4% PFA for 10 min. Cells were permeabilised with 0.1% Triton-100 in PBS (further referred to as PBST). They were blocked with blocking solution (1% BSA in PBST with 3% goat serum) and incubated with the primary antibody at 4°C overnight (Table 3). The next day cells were washed 3x in PBST and the respective secondary antibody was applied in blocking solution and incubated for 45-60 min at room temperature. Cells were washed with PBS and incubated for 5 min with a DAPI nuclear counterstain at 1 µg/ml final concentration. Images were acquired using the Nikon TiE Microscope and NIS elements software (Nikon).

**Table 3: List of antibodies used in immunohistochemistry experiments**

| **Antibody** | **Dilution** | **Supplier/Catalogue number** |
|---|---|---|
| *Primary antibodies* | | |
| Anti-SOX2 (rabbit) | 1:200 | Abcam (Ab92494) |
| Anti-SOX2 (goat) | 1:200 | R&D Systems (af2018) |
| Anti-Olig2 (rabbit) | 1:200 | Sigma (Ab9610) |
| Anti-Nestin (mouse) | 1:500 | R&D Systems (mab1259) |
| Anti-GFAP (mouse) | 1:500 | Millipore (ab5541) |
| Anti-SOX9 (rabbit) | 1:200 | Millipore (ab5535) |
| Anti-V5 (mouse IgG2b) | 1:1000 | eBioscience (14679682) |

| *Secondary antibodies* | | |
|---|---|---|
| Anti-rabbit (goat) - Alexa 488 fluorophore | 1:1000 | Invitrogen (A11008) |
| Anti-rabbit (goat) - Alexa 594 fluorophore | 1:1000 | Invitrogen (A11012) |
| Anti-goat (donkey) - Alexa 488 fluorophore | 1:1000 | Invitrogen (A11055) |
| Anti-mouse (goat) - Alexa 594 fluorophore | 1:1000 | Invitrogen (A21044) |

### Flow cytometry

For flow cytometry, cells were detached, pelleted and resuspended in an appropriate volume of flow cytometry buffer (1% BSA in PBS, v/v). Cells were stained with Draq7 (Abcam, ab109202, f.c. 0.1 µM) as a live/dead stain and analysed using the BD LSRFortessa cell analyser (4 lasers, BD Bioscience). Analysis of flow cytometry data was carried out in the FlowJo Analysis software (FlowJo, version 10.6.2).

### Quantitative Real-time polymerase chain reaction (qRT-PCR)

For qRT-PCR the TaqMan Universal PCR Master Mix (Applied Biosystems) and the TaqMan gene expression assays (Life Technologies) were used on a Quant Studio7 Flex Real-Time qPCR machine. RNA samples which did not undergo reverse transcription to assess DNA contamination and water controls were employed on every plate. Additionally, qRT-PCR was carried out in technical duplicates. Data analysis was performed using the ddCt method which assumes 100% PCR efficiency and is guaranteed with TaqMan assays. Briefly, the mean was calculated for technical replicates and normalised to the housekeeping gene GAPDH which yields the ddCt value. These values were further normalised to a calibrator sample (GSC7) to give the ddCt.

### Production of entry and final vector with the Extensible Modular Mammalian Assembly (EMMA) Toolkit

Vectors were designed and produced according to Martella et al. 2017. Briefly, for the domestication of a new part, Bsal and BsmBI sites were removed. The new part was PCR amplified using primers generating an overhang for the fusion sites and Bsal site. This ensured simple and efficient cloning into the part entry vector. All part entry vectors contain a red fluorescent protein (RFP) which is recombined with the to be cloned part, thus, increasing the cloning efficiency as only white colonies can contain the part. To assemble an expression vectors, all parts are mixed with the acceptor vector in an equimolar ratio. To increase cloning efficiency the acceptor vector contains the bacterial suicide cassette ccdB which is recombined with the part of the expression vector. Therefore, bacteria which take up the unmodified acceptor will not be able to make colonies.

### Western blotting

Cells were scraped in PBS and spun down at 0.3 g twice to remove all liquid. Cell pellets were resuspended in 70 µl of RIPA buffer (50 mM Tris-HCl pH 8.0, 150 mM NaCl, 1% NP-40, 0.5% deoxycholate, 0.1% SDS and protease inhibitor (Complete, Roche, 11697498001) and incubated for 5 min on ice. Lysates were centrifuged at 13000 rpm for 10 min at 4°C (centrifuge 5415D, Eppendorf). And the supernatant was collected in a new tube. Protein extracts were quantified using the Pierce BCA Protein Assay Kit (Thermo Scientific, Cat: 23225) according to the manufacturer's instructions.

5% of the total volume of 4x Lithium dodecyl sulfate (LDS) buffer containing 50 mM DTT was added to the cellular extracts and samples were denatured at 95°C for 10 min. Samples were loaded in 4-12% polyacrylamide gels prepared by Dr Carla Blin with a Spectra Multicolour Broad Range Protein Ladder (Thermo Scientific, Cat: 26634) or BioRad Precision Plus Protein Dual Colour standards (Cat: 1610377). Protein band transfer to a Immobilon PVDF membrane (Millipore, IPVH00010) was carried out by wet electroblotting or semi-dry blotting using the Biorad Trans-blot turbo system following manufacturer instructions. 5% milk in TBS-T (TBS + 0.1% Tween-20) was used for membrane blocking for 1 hour at room temperature, followed by primary antibody incubation in 5% milk in TBS-T while rocking overnight (Table 4). The next morning, the membrane was washed three times with 5 min washed at room temperature in TBS-T and incubated with secondary antibody coupled to horseradish peroxidase in 5% milk in TBS-T at room temperature for 1 hour (Table 4). Again, the membrane was washed three times for 5 min in TBS-T and developed using homemade enhanced chemiluminescence (ECL) solution or Clarity ECL Western Blotting substrate (Bio-Rad, Cat:170-5061) and imaged using X ray films or a Bio-Rad ChemiDoc^{™} Imager.

**Table 4: List of antibodies used in western blotting experiments**

| **Antibody** | **Dilution** | **Supplier/Catalogue number** |
|---|---|---|
| *Primary antibodies* | | |
| Anti-SOX2 (rabbit) | 1:1000 | Abcam (ab92494) |
| Anti-SOX9 (rabbit) | 1:500 | Millipore (ab5535) |
| Anti-GAPDH (mouse) | 1:10000 | Ambion (AM4300) |

| *Secondary antibodies* | | |
|---|---|---|
| Anti-rabbit | 1:5000 | Novex (A16110) |
| Anti- mouse | 1:5000 | Novex (A16027) |

### Nuclear extracts

All the buffers were prepared the day before, passed through a 22 µm filter (except the dialysis buffer) and left at 4°C overnight. DTT and protease inhibitors were added just before use. The cells were scraped on the media and collected on 50 ml falcons, which were spun at 1350 rcf 5 minutes at 4°C. The pellet was resuspended in 50 ml of ice cold PBS, an aliquot was taken for cell counting, and spun again spun at 1350 rcf 5 minutes at 4°C. The pellet was then resuspended in 5 ml per 40 million cells of ice cold buffer A (10 mM HEPES pH 7.9, 1.5 mM MgCl₂, 10 mM KCI, 0.5 mM DTT, protease inhibitors (Complete, Roche, 11697498001)) and incubated on ice for 10 min. The cell suspension was transferred to a glass Dounce homogenizer and dounced 40 times on ice. The cell suspension was transferred to a falcon and centrifuged at 1350 rcf for 10 min at 4°C. The supernatant was discarded (cytosolic extract) and the pellet resuspended in 100 µl per 10 million cells in ice cold buffer B (20 mM HEPES pH 7.9, 5% glycerol, 1M NaCl, 1.5 mM MgCl₂, 0.2 mM EDTA pH 8.0, 0.5 mM DTT, protease inhibitors (Complete, Roche, 11697498001)). The suspension was rotated for 30 minutes at 4°C and transferred into a dialysis membrane (SnakeSkin, Thermo Scientific, Cat 68100). Dialysis was carried out in 500 ml of dialysis buffer (20 mM HEPES pH 7.9, 5% glycerol, 100 mM KCI, 0.83 mM EDTA pH 8.0, 1.66 mM DTT, protease inhibitors (Complete, Roche, 11697498001)) for two hours at 4°C with rotation. Dialysis buffer was changed (500 ml) and the dialysis was continued overnight at 4°C. The extract was collected in an 1.5 ml tube and spun at maximum speed for 15 min at 4°C (centrifuge 5415D Eppendorf). The supernatant was collected into new 1.5 ml tubes and quantified (Pierce BCA Protein Assay Kit, Thermo Scientific, Cat 23225). 100 µg aliquots were flash frozen in liquid nitrogen and stored at -80°C.

### PCR amplification with biotinylated primers and purification

Enhancers were amplified with 5' biotinylated primers (Table 5) using PrimeStar Max (Takara) according to the manufacturer's instructions.

**Table 5: 5' Biotinylated primers for transcription factor pull down**

| **Primer number** | **Sequence** | **SEQ ID NO.** |
|---|---|---|
| Forward primer 595 | TGATCCGTCTCgCCCTactaggttactggtgcatgc | 81 |
| Reverse primer 596 | ACTAACGTCTCgGAGCacccaaactattggagcgag | 82 |

PCR purification using Agencourt AM Pure XP magnetic beads (Beckman Coulter) was carried out according to the manufacturer's instructions. PCR product quantification using Tapestation and reagents was carried out according to manufacturer's instructions.

### Precipitation of interacting proteins

Streptavidin Magnetic Bead (NEB, S1420S) were used according to the manufacturer's instructions. Briefly, 10 µl beads were aliquoted into LoBind tubes and washed three times with binding buffer (20 mM Tris-HCl pH 7.5, 0.5 M NaCl, 1 mM EDTA) on the magnetic stand. 20 µl biotinylated DNA (~ 20 pmol) were mixed with 200 µl binding buffer and was added to the beads. The suspension was rotated at room temperature for 2 hours. The beads were washed again three times with binding buffer and 50 µg nuclear extract was added (total volume 50 µl). Beads, DNA and nuclear extract, to which 150 µl dialysis buffer (20mM HEPES pH 7.9, 5% glycerol, 100 mM KCI, 0.83 mM EDTA pH 8.0, 1.66 mM DTT, protease inhibitors (Complete, Roche, 11697498001)) was added, were rotated overnight at 4°C. The following morning, beads were washed three times with wash buffer (20mM HEPES pH 7.9, 5% glycerol, 250 mM NaCl, 0.83 mM EDTA pH 8.0, 1.66 mM DTT). For western blotting, proteins were eluted in 20 µl loading buffer (lithium dodecyl sulfate (LDS) buffer containing 50 mM DTT) and boiled for 5 min to denature the proteins. For mass spectrometry beads were sent dry to the facility.

### Zebrafish experiments

All embryos are obtained by natural spawning and collected in conditioned aquarium water in 0.00001% methylene blue. Embryos were treated with 200 µM N-phenylthiourea (PTU) (Sigma) from 6-hours post fertilisation (hpf) for the duration of the experiment to inhibit pigmentation (Karlsson, Von Hofsten, and Olsson 2001).

Zygotes were injected at the one-cell stage of development. Eggs were kept in 1.5% Agarose (Sigma) moulds as described by Nusslein-Volhard and Dahm 2002 for microinjection. Microinjection needles were pulled from glass capillaries (Harvard Apparatus, USA) using the P-97 Flaming/Brown Micropipette Puller (Sutter Instruments, USA). Needle pull parameters are as follows: Heat: 550; Pull: 200; Velocity: 55; Time: 150. Microinjection was performed on the PV820 Pneumatic PicoPump (World Precision Instruments [WPI], USA) system. DNA constructs were created using the Tol2Kit system (Kawakami 2007, Kwan et al. 2007). Approximately 2 nL of plasmid DNA (30 ng/µL) containing Tol2 capped mRNA (20 ng/µL), supplemented with 0.2% w/v phenol red (Sigma) to facilitate visualization of injected volume, were injected. Embryos were imaged at 24-48 hpf on a wide-field fluorescent microscope live following anaesthesia with Tricaine.

### Adeno-associated virus (AAV) transduction assay

HEK293 cells are seeded the day before the transfection into a 6 well plates so that on the day of transfection confluence would be at 60-70%. Before HEK293 cells are transfected with PEI a media change is carried out as this media will be conditioned with virus over the for two days. The following days HEK293 or GSC7 cells are seeded at low density (10-20%) in the same or a smaller plate format (6 or 12 well plate). AAV-conditioned media is spun down at 1300 rpm for 4 min to remove contaminating cells and the supernatant is transferred to the HEK293 cells seeded at low density. Cells are analysed on the microscope or flow cytometry.

### Cell seeding and transduction for killing assays

On the day of cell seeding, cells were detached using above described method. Cells were counted using hemocytometer and seeded into either 96- (1,000 cells per well in 50µL), 24-(30,000 cells per well in 500µL) or 6-well (60,000 cells per well in 2mL) Corning plate and placed into incubator at 37°C/ 5% CO2. The next day, AAV virus stocks were thawed at room temperature and an appropriate amount of virus stock was added to required amount of culture media to achieve final multiplicity of infection (MOI) of 5 x 10⁵. Culture media containing viral particles was added to cells without replacement of existing media. Cells were returned to 37°C/5% CO₂.

### Drug treatment

Lyophilised GCV was diluted in DMSO to achieve 100mM stock concentration. GCV stocks were aliquoted and were stored at -20°C for no longer than 1 month. To make a working stock concentration, GCV was diluted 1:100 in appropriate culture media and then 20 µL of such working stock was added to wells already containing 80 µL of culture media (achieving another 1:5 dilution). Final concentration of GCV on cells was 200 µM.

As negative control, DMSO was diluted 1:100 in appropriate culture media to achieve working stock. 20 µL of working stock was added to wells already containing 80 µL culture media. As positive control, 20 µL of DMSO was added to wells with 80 µL of culture media, achieving final concentration of 20% DMSO.

### Incucyte Live-cell imaging

To track cell proliferation and morphological changes during treatments, cells were monitored using the Incucyte live-cell imaging system. Whole well imaging of Corning 96 well-plate every 4 hours. Basic confluence scoring analysis software (Incucyte) was used to estimate confluence. Images at specific time points were extracted to verify cell confluence and morphology.

### MTT assay

On the day of assay culture media was replaced with 0.3mg/mL MTT solution (diluted in cell line-appropriate culture media). Cells were placed in incubator at 37°C/5% CO₂ for 3 hours. After incubation, media was removed and 70 µL of DMSO is added to each well. Each plate was kept in the dark at 37°C for 20 minutes, shaking it occasionally. Before reading the plate, each well was visually inspected to make sure that all (formazan) crystals were dissolved. Plate was read with plate reader at 560nm absorbance.

### Data analysis

Most data analysis was carried out using Microsoft Excel Version 16.23 for Mac and GraphPad Prism 7. Error bars are shown as the standard deviation of the mean. Some data analysis was carried out using RStudio version 1.1.456 (RStudio Team 2015). For generating illustration biorender.com and Adobe Illustrator 22.0.1 were used. Furthermore, open-source programmes were used, such as bedtools (Quinlan and Hall 2010), GREAT (Mclean et al. 2010), fastasplitter (Stothard 2000) and MEME (Bailey et al. 2009) for various analyses.

### EXAMPLE 1 - Identification of functional SOX2 enhancers

To first establish proof of concept as to whether synergistic activity could be achieved when combining individually active enhancers into clustered multi-part arrays we first identified a small set of candidate enhancers. These were selected based on the proximal gene having a known role in neural stem cell (NSC) self-renewal (*POU3F2, POU3F3, CHD7, ASCL1, SOX6, ETV1*) as well as using available differential expression data for NSCs versus primary human fibroblasts. Five SOX2 candidate autoregulatory enhancers were also included. These enhancers (typically ~500-800bp) were cloned into plasmid expression vectors harbouring mNeonGreen and a Luciferase reporter cassette and tested for their individual enhancer activity using two independent patient-derived glioblastoma stem cell (GSC) cell lines (G7 and G328) (Figure 1). Six of these enhancers were functional with more than 10-fold increased expression of NanoLuc over mCMV (254, 270, 282, 292, 312 and 316; range from 10-300 fold). None of these were active in HEK293 cells (negative control).

**Table 6: Predicted target genes and distance from enhancer fragment to transcription start site (TSS)**

| **Enhancer** | **hg19** | **Gene 1** | **Distance to TSS1 for Gene 1** | **Gene 2** | **Distance to TSS2 for Gene 2** |
|---|---|---|---|---|---|
| 252 | chr8:61788019-61789039 | CLVS1 | -411985 | CHD7 | 197192 |
| 254 | chr7:14060491-14061192 | ETV1 | -31271 | DGKB | 882144 |
| 270 | chr9: 109363039-109363802 | ZNF462 | -261957 | TMEM38B | 906596 |
| 282 | chr3:181101249-181102012 | DNAJC19 | -394070 | SOX2 | -328091 |
| 284 | chr3:180970481-180971050 | SOX2 | -458956 | DNAJC19 | -263205 |
| 286 | chr3:180971430-180972034 | SOX2 | -457990 | DNAJC19 | -264171 |
| 292 | chr3:182010198-182010810 | ATP11B | -500784 | SOX2 | 580782 |
| 294 | chr3:182023544-182024242 | ATP11B | -487395 | SOX2 | 594171 |
| 310 | chr12:103359143-103359469 | STAB2 | -621745 | ASCL1 | 7842 |
| 312 | chr11:16169685-16170249 | SOX6 | 327968 | - | - |
| 316 | chr11:16213249-16214048 | SOX6 | 284286 | - | - |
| 324 | chr6:99195817-99196383 | POU3F2 | -86480 | - | - |
| 336 | chr2:105475793-105477403 | MRPS9 | -177843 | POU3F3 | 4629 |

### EXAMPLE 2 - Assembly of four different enhancers results in synergistic increases in transcriptional activity

In order to investigate whether combining multiple enhancers can increase activity we first built versions concatemers of either two enhancers (2x), four enhancers (4x) or eight enhancers (8x) (concatemers) (i.e. multiple copies of the same enhancer). We chose three different individual enhancers with variable strength: 270 (about 100-fold), 254 (about 20-fold), and 312 (about 10-fold). For 270, in both 2-part and 4-part enhancers, we noted only modest synergistic effects compared to the individual enhancer activity. The 4-part enhancer resulted in about 500-fold activity (Figure 1F), compared to about 100-fold for the individual enhancer. Surprisingly, we observed no additional gain by increasing the copies to an 8-part concatemer. Similar trends were observed for an independent patient line, GSC328. Only background levels of luciferase activation, comparable to the mCMV negative control, were seen in HEK293 cells for all constructs.

We next tested whether mixing together different enhancers from different genes would result in improved synergy (Figure 1K). We therefore combined the top 4 individual enhancers identified in G7 (270, 282, 292 and 316) into a new synthetic enhancer (termed T4). Indeed, we noted a remarkable increase with more than 5000-fold increased expression for T4 relative to mCMV (Figure 1L) using Nanoluc reporter assays (Nano-Glo Luciferase Assay and Nano-Glo Dual-Luciferase Reporter Assay, both Promega). This was further validated using flow cytometry to score mNGreen in G7 cells (Figure 1I) which indicated levels of T4 were approximately 50-60% of that observed for full length CMV in G7 cells. Combining enhancers from different genes therefore results in synergistic effects and major increases in enhancer activity in GSCs, but without increasing background expression in HEK293 cells. These cell type-specific synthetic clusters of enhancers are subsequently referred to as 'synthetic super-enhancers' (SSEs).

### EXAMPLE 3 - Activity of the T4 synthetic super-enhancer in GSCs is reduced following astrocyte differentiation

We next tested whether the SSE-T4 synthetic super-enhancer was specific to the GSC state and therefore lost upon their differentiation into astrocytes where SOX2 is downregulated. G7 cells can be efficiently differentiated into an astrocyte-like cells following exposure to foetal calf serum (FCS) over a 10-15 day period. GSCs (G7) were therefore stably transfected with the SSE-T4 expression cassette (using the PiggyBac transposase system) and mNGreen+ cells sorted using fluorescence activated cell sorting (FACs). These were plated into 5% FCS the following day and mNGreen expression was measured every 5 days using flow cytometry over a 15 day time-course (Figure 1H-J). Expression of mNGreen was significantly decreased by day 5 based on live cell fluorescence imaging. This was confirmed by flow cytometry, which showed approximately 50% of cells lost mNGreen by day 5, and approximately 80% by day 15. These data indicate the SSE-T4 has high activity in GSCs, but not in their differentiated astrocyte-like progeny and thus the SSE is cell-type specific.

### EXAMPLE 4 - Systematic screening of an arrayed plasmid library of SOX2 enhancers

The above data provided proof-of-principle that combining enhancers into four-part arrays can increase transcriptional activity, without compromising background expression in non-GSC cells. We reasoned that a functional screen of enhancer fragments (160bp), could be performed using the full set of candidate genome-wide SOX2 enhancers in high throughput 384-well plate based luciferase assays to systematically identify functional enhancers with optimal performance and smaller size when used in SSEs. By performing such screens in patient-derived GSCs, we can overcome the limitations of existing serum grown cancer cell lines, which do not have the appropriate identity and associated enhancers.

160 bp was chosen as this provides sufficient size for nucleosome binding and is likely to be an important attribute in the evolution of the grammar within functional enhancers (Soufi et al. 2015). Moreover, 160 bp is a convenient size for synthesis of a pooled oligonucleotide library that incorporates 20 bp ends for subsequent construction of an arrayed plasmid library. We favoured an arrayed plasmid library constructed from synthetic oligonucleotides, over other pooled library screening strategies, such as STARR-seq (Muerdter et al. 2018, Arnold et al. 2013), as this ensures improved signal to noise and straightforward validation of hits.

We therefore designed a set of oligonucleotides that could be synthesized as a pool. We re-analysed the datasets from Suvà et al. 2014, and defined a set of GSC-specific SOX2 binding peaks that overlapped with H3K27ac, which identified 1710 different enhancers. These enhancers were on average about 400 bp (range: 115-3366 bp). We split these sequences *in silico* into 160 bp elements that overlapped by 100 bp. 9523 different oligonucleotide sequences were then synthesized as a pool, PCR amplified, cloned into an arrayed plasmid library of 48 x 96 well plates (4579 individual plasmids in total). Sanger sequencing a subset of the resulting plasmids confirmed that the majority (~70%) of these plasmids contained an appropriate enhancer.

The SOX2 enhancer library was screened in 384-well format using optimised luciferase assays. 135 plasmids were identified as functional with more than 10-fold increase over mCMV. 52 of these 135 initial hits were then validated using independent Nanoglo DLR assays in triplicate (Figure 2). 16 of these 52 were found more than once. Hits were sanger-sequenced, confirmed to contain an enhancer and mapped back to the human genome with an expected fragment from the original designed set.

We next validated the top 17 fragments (more than 30-fold increase over mCMV) using an independent flow cytometry assay across multiple cell lines (Figure 2). These were active in an independent patient-derived GSC line (GSC328) but were inactive in non-neural cells tested (HEK293 cells and human primary fibroblasts, huFb170). We found that the majority of the top hits are evolutionarily conserved sequences across vertebrates. The top 5 enhancers were each more than 100-fold compared to mCMV (range: 100-260-fold) based on NanoLuc. These were validated by flow cytometry.

ID1101 is located in an intron of PRCP; ID2904 is located in the intronic region of the MYO18 gene and in proximity to SEZ6L; ID0109 is located in a gene-rich region between TPK1 and CNTNAP2; ID4328 is in the intronic region of the zinc finger transcription factor ZNF438. ID0876 is located in the intronic region of NWD2.

**Table 7: Predicted target genes and distance from enhancer fragment to transcription start site (TSS)**

| **Fragment** | **hg19** | **Gene 1** | **Distance to TSS1 for Gene 1** | **Gene 2** | **Distance to TSS2 for Gene 2** |
|---|---|---|---|---|---|
| **ID1101** | chr11:82553160-82553319 | PRCP | 58265 | | |
| **ID2904** | chr22:26319890-26320049 | SEZ6L | -245631 | MYO18B | 181790 |
| **ID0109** | chr7: 145217558-145217717 | TPK1 | -684464 | CNTNAP2 | -595843 |
| **ID4328** | chr10:31308050-31308209 | LYZL2 | -389438 | ZNF438 | 12737 |
| **ID0876** | chr4:37394656-37394815 | RELL1 | 293256 | | |
| **ID2339** | chr8:1845248-1845407 | MYOM2 | -147864 | ARHGEF10 | 73149 |
| **ID1737** | chr15:38880930-38881068 | THBS1 | -992325 | RASGRP1 | -23962 |
| **ID0663** | chr9:109338243-109338365 | ZNF462 | -287134 | TMEM38B | 881419 |
| **ID2706** | chr2:208637297-208637434 | FZD5 | -3078 | | |
| **ID3547** | chr16:27730146-27730305 | KIAA0556 | 168738 | GSG1L | 344619 |

Thus, our functional screening resulted in identification of many 160 bp enhancer fragments that have significantly improved activity compared to full-length enhancers (described above), despite being 4 to 5 times smaller in size.

### EXAMPLE 5 - Creation of strong and selective synthetic super enhancers using newly identified SOX2 enhancers

We constructed synthetic super-enhancers by clustering the individually most active fragments from the screen into 4-part arrays to identify whether their strength increases synergistically while retaining cell type selectivity.

In order to create the strongest possible synthetic super-enhancers we designed a set of four new 4-part arrays using sequences from the Top17 enhancers. These were assembled upstream of mCMV. Fragments were ranked according to their median fold change to mCMV. The 4 most active enhancers (ID1101, ID2904, ID0109, ID4328, denoted by '1') were clustered to a 4-part array into a synthetic super-enhancer. Enhancers comprising the second group (denoted by '2') were clustered into a synthetic super-enhancer and so forth. We hypothesized that the four strongest enhancers will exhibit the highest level of expression, the next group will be second highest etc. Furthermore, by testing the synthetic super-enhancers in different cell types (GSC7, GSC328, HEK293 and huFb170) we also investigated their cell type - selectivity.

All four constructs tested (C1, C3, C5 and C7) induced significant and selective expression in GSC7 and GSC328 and only background expression in HEK293. No expression was detected in huFb170. Each of these synthetic super-enhancers (C1, C3, C5 and C7) exhibited a higher fluorescence intensity and a higher percentage of mNGreen+ cells than SSE-T4 (1st generation SSE discussed above), the most active synthetic super-enhancer using candidate enhancers, in GSC7 and GSC328. Remarkably, C1 and C7 induced a higher expression level than the positive control CMV, which is itself one of the strongest viral promoters. We therefore, observed clear synergistic effects of building arrays of a 4-part enhancers, resulting in extremely strong and cell type-selective synthetic super enhancers.

We reduced the size of the synthetic super-enhancers further by removal of the adaptors and spacers (e.g. for C1, 974 bp to 640 bp). These synthetic super-enhancers were termed SSE 1-7, as they are 7 constructs and their design follows the same rationale as C1, C3, C5, C7. All constructs showed a similar level of activity. This was comparable in levels to the full CMV, and in some cases it was higher than CMV (SSE1, 2, 3, 5 by Nanoglo DLR assay and SSE-1, 2, 3, 5, 7 by flow cytometry in GSC7. SSE-1, 2, 5 and 7 also exhibited higher intensity than CMV in GSC7. Analysis of mNGreen expression by flow cytometry was also performed for GSC328, HEK293 and huFb170. The trend is similar in GSC328. The mean fluorescence intensity (MFI) was around CMV, with SSE-6 being clearly less intense than CMV. In HEK293 cells all synthetic super-enhancers induced transcription to a similar level like mCMV. mCMV exhibited 32.5% mNGreen+ cells in comparison to non-transfected cells. SSE demonstrated small increase in expression in HEK293 compared to mCMV. The activity of SSE1-7 in huFb170 was undetectable (more than 1%). A tendency towards higher activity with smaller synthetic super-enhancers in both reporter gene assays (Nanoglo DLR assay and flow cytometry) was observed for each construct C1 vs SSE-1; C3 vs SSE-3 etc (Figure 3B, C, D). This was confirmed by live cell images (Figure 3E). Each of synthetic super-enhancers SSE-1, 3, 5 and 7 tested were similar in activity to CMV.

### EXAMPLE 6 - Synthetic super-enhancers are less active in differentiated GSCs

In order to investigate the activity of SSE-2-SSE-7 under differentiating conditions, GSC7 cells were stably transfected using the Piggybac transposase system and sorted for mNGreen+ cells. A similar number of live cells was sorted for the mCMV and untransfected controls. Cells were then placed into media containing no growth factors but 5% FCS to induce astrocytic differentiation.

mNGreen expression was measured at 0, 1, 5, 10 and 15 days during differentiation (Figure 4E and G). We observed a drop of signal over the time course, suggesting that the synthetic super-enhancers are most highly expressed in the GSCs, and activity is decreased upon differentiation.

### EXAMPLE 7 - Zebrafish embryos reveal highly selective, region-specific expression in forebrain

Zebrafish are well-suited as an experimental model to explore the tissue specificity of enhancers. We identified tissues which are shared between all constructs like ventral spinal cord expression, otic placode as well as midbrain/hindbrain and anterior forebrain. Some fragment had additional activity, such as the anterior/posterior intestine. We generally observed low expression at 24 hpf which kept increasing up to the latest measured timepoint at 48 hpf. Regarding the controls, we observed broad, unspecific expression of EGFP in the CMV control and no expression in the mCMV using the same assay (Figure 4). The mCMV control exhibited green fluorescent eyes which is probably due to spurious transcription from the Crystal eye promoter (Figure 4K).

### EXAMPLE 8 - SSEs can be used in adeno-associated viruses to induce selective killing of GSCs

To test if SSEs are suitable to drive a cytotoxic payload and selectively kill GSCs, we transduced GSC7 and huFB170 cell lines with the adeno-associated virus 2 (AAV2) containing the suicide gene thymidine kinase (TK) derived from the Herpes simplex virus (HSV) in a polycistronic mRNA with the reporter gene mCherry joined by p2A. The cassettes were either driven by a constitutively active CMV promoter as a positive control (CMV_HSV-TK-v5_P2A_mCherry) or the promoter of interest, SSE-7 (SSE-7-HSV-TK-v5-P2A-mCherry). HSV-TK is a well-established prodrug which metabolises the non-cytotoxic prodrug ganciclovir (GCV) into a cytotoxic product by phosphorylation. Phosphorylated GCV competes with dGTP as a substrate for polymerase thereby leading to interference of DNA synthesis causing premature termination and cytotoxicity.

Flow cytometry and western blotting confirmed SSE-7 is driving both mCherry and HSV-TKv5 expression in GSC7 but not in fibroblasts (huFb170) (Figure 5). Cell confluence was recorded throughout the cytotoxicity experiment. A decrease in cell proliferation and confluence was specifically observed in cells which were transduced with CMV or SSE-7-driven TKv5-P2A-mCherry and received 200 µM GCV (Figure 6A). No significant negative effect on cell confluence was observed in cells which received virus or ganciclovir alone. MTT assay data demonstrates loss (or lack) of metabolic activity, which serves as an approximate readout for cell viability in GSC7 cells treated with CMV or SSE-7-driven TKv5-P2A-mCherry and GCV (Figure 6B). There was no statistical difference between CMV and SSE-7 (13% vs 12% viability) (p-value = 0.7792). There was a small effect of ganciclovir alone on GSC7 cell viability (89% viability). Live cell imaging confirmed these findings showing lower cell density and morphological changes linked to cellular stress, damage or death (rounded cells) after treatment (Figure 6C). In summary, both CMV and SSE-7 can drive expression of HSV-TK-v5-P2A-mCherry construct to a sufficient level in GSC7 cells to induce cell death in the presence of ganciclovir.

Based on live cell imaging data at the end of experiment (day 17), the majority of the huFB170 cells which were transduced with CMV-driven, but not SSE-7-driven, virus were killed (Figure 6D). This was confirmed with MTT data, demonstrating 6.3% viability in cells transduced with CMV-driven virus versus full viability in cells transduced with SSE-7-driven TKv5-P2A-mCherry (Figure 6E, n=1).

### EXAMPLE 9 - SSEs show activity in a range of GSCs

It is desirable for an SSE in a proposed gene therapy to be expressed highly across diverse patient-derived glioblastoma cell lines, but inactive in other cell types. The experiment described in Example 8 was repeated in five patient-derived glioblastoma cell lines (E17, E21, E28, E31, E34). These lines were selected for this experiment as they cover a spectrum of genetic and transcription GBM subtypes and have been shown to be tumour inducing in a mouse brain. Altogether, SSE7 is active in all cell lines, but to different degrees: it is highly expressed and comparable to full-length CMV in the positive control cell line GSC7 (Figure 7C);and E17 and E28 (Figure 7C), which both belong to the classical subtype, but has a lower frequency of expression in E21, E31 and E34 (Figure 7C).

### EXAMPLE 10 - Analysis of de novo motifs identifies a SOX dimer motif enriched in the Top 32 hits

We used the MEME tool, which can identify *de novo* motifs (Bailey et al. 2009). We used the top 32 hits as input. Unexpectedly, the significant motif that emerged was a partial palindromic SOX motif (Figure 8A). SOX2 does not form homo or heterodimers with other SOX factors; however, members of the SOXE group are well established to dimerise (SOX8, SOX9, SOX10). Interestingly, SOX9 is a well-known regulator of NSCs and GSCs, but has not been as deeply studied as SOX2 (Bulstrode et al. 2017, Huang et al. 2015, Mateo et al. 2015, and D. K. Singh et al. 2017). This motif suggested immediately that SOX2 and SOX9 might be operating at shared enhancers (Figure 8C).

We then investigated whether this dimer motif is a general feature of the oligonucleotide pool of sequences and mapped it to the Top 52 hits (all sequences with a 10x fold change over mCMV) compared to unconfirmed hits of the validation and in 10 random sequences of the initial oligonucleotide pool. The motif is present at high frequency in the Top 32 hits and with lower frequency in the remaining Top 33-Top 52.

### EXAMPLE 11 - A SOX dimeric motif is essential for the activity of ID2904 in GSCs and suggests cooperation of SOX2 and SOX9 at key GSC functional enhancers

We next explored whether the SOX dimer was essential for enhancer function. We generated a series of variants of the motif for ID2904. The variants synthesized had: 1) decreased spacing between the half sites (-2n); 2) increased spacing between the motif half sites; 3) and 4) deletion of one half site; 5) replacement of the flanking sequences by random sequences; 6) inversion of the motif; 7) mutation of the core nucleotides; 8) replacement by the high affinity motif as reported by Jolma et al. 2013; 9) a random sequence as negative control; and 10) concatemerisation of the motif four times. These different variants enabled us to functionally dissect the key sequences within the dimer motif (Figure 8).

We observed an almost complete loss of activity with almost all variations of the SOX dimer motif in ID2904, confirming the importance of this dimer motif for the function of the enhancer. An exception was the altered distance between the two half-sites, which led to an increased activity. This is intriguing, as it is consistent with the notion that the spacing could be 'suboptimised' and the 2bp reduction might drive stronger binding of the SOX9 dimer (Farley et al. 2015). We conclude that the SOX dimer motif is essential for the enhancer activity of ID2904. The reduction of the spacing is also in accordance with Huang et al. 2015, who defined the optimal spacing for SOXE factor motifs.

Next, we aimed to confirm that indeed SOX2 and SOX9 are binding to the enhancer and the SOX dimer motif. To this end we carried out pull down assays in which the ID1101 (top hit of the screen) and ID2901 were biotinylated and incubated with nuclear extracts. Western blotting and mass spectrometry were performed to identify the key transcription factors interacting with the fragments (Figure 8). For data analysis, ID1101 was normalised to the average value of the random sequence 599 (in each cell line). The cut-off was set at two fold enrichment over 599 in both cell lines. To filter for transcription factors a list of all transcription factors was obtained from UniProt and overlapped with the detected proteins in both cell lines.

In coimmunoprecipitation experiments, SOX2 was pulled down specifically with ID1101 in GSC7 and GSC328, demonstrating SOX2 binding to the enhancer (Figure 8). This was confirmed in mass spectrometry as SOX2 was 23-fold enriched in GSC328 and a 4.4 fold enriched in G7, confirming the binding of this transcription factor.

Additionally, data described above and shown in Figure 8J demonstrates that the sequence context (grammar) surrounding the motif is functionally important as replacement of the wild-type (WT) sequence 20bp up and downstream resulted in complete loss of activity.

### EXAMPLE 12 - A SOX dimer motif helps to filter for active enhancers in silico

We postulated that the presence of the motif might have the ability to increase our prediction power about which enhancer is active. To test this hypothesis, we mapped the motif to all full-length enhancers of the original SOX2 ChIP-seq datasets. 70 regions were selected for an initial screen based on their predicted target gene with a known function in GBM or neural stem cells and on clustering within the genome, meaning the regulation of the same predicted target gene. They were tested in the Nanoglo DLR assay. These were termed 'Clustered SOX dimer motif enhancers' (CSEs). Using the SOX dimer motif frequency and rational design we therefore increased the success of identifying positive enhancers around 11-fold; from ~2% in the original screen (52/2610) to ~22.8%, in our rational selection (16/70). Positive hits were further validated by flow cytometry in GSC7, GSC328, HEK293 and huFb170 (Figure 9).

An additional screen with 209 enhancers was carried out to identify further active enhancers. The enhancers were reduced to 160 bp based on their predicted motifs (termed minCSEs - see Table 1). 160 bp enhancer fragments were mixed from different target genes to cover pathways like proliferation, stemness or brain-enriched/brain-specific or glioma-enriched/glioma-specific predicted target genes based on data from the human protein atlas (https://www.proteinatlas.org/), to generate further SSEs for screening.

**Table 8: Verified CSE hits with genomic coordinates, predicted target gene and their distance from enhancer fragment to TSS**

| **Fragment** | **hg19** | **Gene 1** | **Distance to TSS1 for Gene 1** | **Gene 2** | **Distance to TSS2 for Gene 2** |
|---|---|---|---|---|---|
| CSE012 | chr11:13123591-13123982 | ARNTL | -175558 | TEAD1 | 357199 |
| CSE005 | chr1:239997099-239997375 | FMN2 | -257943 | CHRM3 | 204864 |
| CSE034 | chr17:70684065-70684473 | SLC39A11 | 404558 | SOX9 | 567108 |
| CSE078 | chr7:47867049-47867394 | C7orf69 | 32333 | PKD1L1 | 120866 |
| CSE088 | chr8:130645429-130646106 | GSDMC | 153366 | - | - |
| CSE025 | chr14:70453941-70454604 | SMOC1 | 108140 | SLC8A3 | 201514 |
| CSE033 | chr17:70627702-70628067 | SLC39A11 | 460942 | SOX9 | 510724 |
| CSE014 | chr11:16169648-16170321 | SOX6 | 327950 | | |
| CSE061 | chr6:118334186-118334492 | PLN | -535122 | SLC35F1 | 105650 |
| CSE075 | chr7:14543795-14544174 | ETV1 | -514414 | DGKB | 399001 |
| CSE054 | chr5:124993695-124994093 | ZNF608 | -912776 | GRAMD3 | -701930 |
| CSE074 | chr7:14311089-14311579 | ETV1 | -281763 | DGKB | 631652 |
| CSE031 | chr17:70572258-70572879 | SOX9 | 455408 | SLC39A11 | 516258 |
| CSE006 | chr1:61298892-61299426 | C1orf87 | -759717 | NFIA | -248375 |
| CSE194 | chr6:99235165-99236055 | POU3F2 | -46970 | - | - |
| CSE208 | chr7:154534737-154535331 | PAXIP1 | 259760 | DPP6 | 785269 |
| CSE222 | chr7:8421667-8422191 | ICA1 | -120018 | NXPH1 | -51656 |
| CSE229 | chr8:82359507-82360175 | PMP2 | -83 | - | - |

### CONCLUSION

Mapping of transcription factor binding sites, chromatin accessibility or enhancer-associated histone marks are widely used to search for critical transcription factors (TFs) and associated enhancers. However, a major limitation is that TFs bind rather promiscuously, and the functional enhancers represent only a minor subset. We demonstrate herein that combining an array of functional enhancer sequences can lead to the formation of synthetic super-enhancers. By harnessing the natural 'grammar' of endogenous functional enhancer sequences but combining these in optimal sizes and clusters, we were able to obtain SSEs with synergistic increases of transcriptional activation without sacrificing cell type selectivity. Our data therefore support that individual enhancers can be combined to create synthetic super-enhancers that retain selectivity, yet are extremely strong at driving transcription.

### REFERENCES

Arnold et al. 2013 "Genome-wide quantitative enhancer activity maps identified by STARR-seq." Science 339(6123): 1074-1077
Bailey et al. 2009. "MEME Suite: Tools for Motif Discovery and Searching." Nucleic Acids Research 37(Suppl. 2): 202-8.
Boumahdi et al. 2014. "SOX2 controls tumour initiation and cancer stem-cell functions in squamous-cell carcinoma." Nature 511(7508): 246-50.
Bulstrode et al. 2017. "Elevated FOXG1 and SOX2 in Glioblastoma Enforces Neural Stem Cell Identity through Transcriptional Control of Cell Cycle and Epigenetic Regulators." Genes & Development 31(8): 757-73.
Corish and Tyler-Smith, 1999. "Attenuation of Green Fluorescent Protein Half-Life in Mammalian Cells." Protein Engineering 12(12): 1035-40.
Farley et al. 2015 "Suboptimization of Developmental Enhancers." Science (New York, N.Y.) 350(6258): 325-28.
Gangemi et al. 2009 "SOX2 Silencing in Glioblastoma Tumor-Initiating Cells Causes Stop of Proliferation and Loss of Tumorigenicity." Stem Cells 27(1): 40-48.
Gibson et al. 2009 "Enzymatic Assembly of DNA Molecules up to Several Hundred Kilobases." Nature Methods 6(5): 343-45.
Guerra-Rebollo et al. 2019 "Targeting of Replicating CD133 and OCT4/SOX2 Expressing Glioma Stem Cells Selects a Cell Population That Reinitiates Tumors upon Release of Therapeutic Pressure." Scientific Reports 9(1): 9549.
Huang et al. 2015 "SOXE Transcription Factors Form Selective Dimers on Non-Compact DNA Motifs through Multifaceted Interactions between Dimerization and High-Mobility Group Domains." Scientific Reports 5(January): 1-12.
Jolma et al. 2013 "Multiplexed Massively Parallel SELEX for Characterization of Human Transcription Factor Binding Specificities." Genome Research 20(6): 861-73.
Karlsson, Von Hofsten, and Olsson, 2001 "Generating Transparent Zebrafish: A Refined Method to Improve Detection of Gene Expression during Embryonic Development." Marine Biotechnology 3(6): 522-27.
Kawakami, 2007 "Tol2: A Versatile Gene Transfer Vector in Vertebrates." Genome Biology 8(Suppl. 1): 1-10.
Kwan et al. 2007 "The Tol2kit: A Multisite Gateway-Based Construction Kit for Tol2 Transposon Transgenesis Constructs." Developmental Dynamics 236(11): 3088-99. Lopez-Bertoni et al. 2015 "DNMT-Dependent Suppression of MicroRNA Regulates the Induction of GBM Tumor-Propagating Phenotype by Oct4 and Sox2." Oncogene 34(30): 3994-4004.
Lujan et al. 2012 "Direct Conversion of Mouse Fibroblasts to Self-Renewing, Tripotent Neural Precursor Cells." PNAS 109(7): 2527-32.
MacLeod et al. 2019 "Genome-Wide CRISPR-Cas9 Screens Expose Genetic Vulnerabilities and Mechanisms of Temozolomide Sensitivity in Glioblastoma Stem Cells." Cell Reports 27(3): 971-986.e9.
Martella et al. 2017 "EMMA: An Extensible Mammalian Modular Assembly Toolkit for the Rapid Design and Production of Diverse Expression Vectors." ACS Synthetic Biology 6(7): 1380-92.
Mclean et al. 2010 "GREAT Improves Functional Interpretation of Cis-Regulatory Regions." Nature Biotechnology 28(5): 495-501.
Muerdter et al. 2018 "Resolving Systematic Errors in Widely Used Enhancer Activity Assays in Human Cells." Nature Methods 15(2): 141-49.
Nusslein-Volhard and Dahm, 2002 "Zebrafish: a practical approach" New York: Oxford University Press: 303 pages.
Pollard et al. 2009 "Glioma stem cell lines expanded in adherent culture have tumor-specific phenotypes and are suitable for chemical and genetic screens" Cell Stem Cell 4(6): 568-80.
Quinlan and Hall, 2010 "BEDTools: A Flexible Suite of Utilities for Comparing Genomic Features." Bioinformatics 26(6): 841-42.
D. K. Singh et al. 2017 "Oncogenes Activate an Autonomous Transcriptional Regulatory Circuit That Drives Glioblastoma." Cell Reports 18(4): 961-76.
S. K. Singh et al. 2004 "Identification of Human Brain Tumour Initiating Cells." Nature 432(7015): 396-401.
Soufi et al. 2015 "Pioneer Transcription Factors Target Partial DNA Motifs on Nucleosomes to Initiate Reprogramming." Cell 161(3): 555-68.
Stothard, 2000 "Internet On-Ramp." BioTechniques 28(6): 1102-4.
Stricker et al. 2013 "Widespread resetting of DNA methylation in glioblastoma-initiating cells suppresses malignant cellular behavior in a lineage-dependent manner" Genes Dev. 27(6): 654-69.
Suvà et al. 2014 "Reconstructing and Reprogramming the Tumor-Propagating Potential of Glioblastoma Stem-like Cells." Cell 157(3): 580-94.

**TABLE 1 - Sequences (without adapters) of the enhancers**

| **Fragment** | **Fold change (Median)** | **Sequence** | **SEQ ID NO.** |
|---|---|---|---|
| ID1101 | 258.8 | GTGAGTGTGACTCACATGCTGGCATCAGAGCCCCTCTTAGAGCTTAGAACACTGGGCCTC TGTCAGGCAAAGAAAAGGAGTCCCTATGAATAGCCATCTCCACAAATAGCAGTTACTGTCA CTTCTTAAAAGACACAAGCATGGCTGGAGACCGGCACTG | 4 |
| ID2904 | 251.6 | TGCATGTTCTTGGGAAAAAGTGGTATTTAATGAAGGCGCTTTGAGCTGAATCAAAGAGCCT CATTCTTCTGAAAGTCCTTCTTCCTAGTCTCTGCATCCGGCTCCTATCAAAGGAAATCCAC AGCTTTGGCTTTTCGTTGCTGTTGTCATGGAAATCGTT | 5 |
| ID0109 | 116.4 | GAAATTGGATCCTGAGGAGAATGTCACCACCTCTTTGACAATAACTAGCTTTCAGAGCTGA ACACAGTGAATCTGGATACTGTGTATATGTTTAAATGAGTTAGGAAATGACATCACATTATT TATTTCTTCTTTTGTGACTTAGTAAATTGACTTGGCC | 6 |
| ID4328 | 100.7 | TACAATCTATTCTCCATAGTCATCAGAGTTTCTTTAAAGGCACGGTTCAGACAATGGCACC CTCTGTTCCAAACCCTCCAGGGCTTCCTATTTCACTTAGCATAAAAACCAAAGTCCTCACA ATGACCGACAAGTCTTCACACAATGTGCCCCTTCATTG | 7 |
| ID0876 | 91.1 | GAGCAGGCAGTCATTTGGTTCCCTTCTCTGAATGCATTGGCTCTGATCCACGGCGCCTCA GGAAGTGAAAGGGAGCAGGCAGTGAGGCCAAGCCATGAATGTTGGGCCCTTTTCTTAGA AAACAAAGGGTTCACTTTAGGCTAGGAGGAAACATCCGGGG | 8 |
| ID2339 | 79.6 | GTCGGCGGAACTGAGTCTCCTTTGTGGAGCCCCTCATTTGCATGATAAACCCCGGGGACA AAGTGTGTTCTTGTTACTTGGACTGTTTCTTGGAAACTGCTGCTACCTGGAGCGAGTGAAC ACAGGGCAAGTATTCTCGCCCACAGCAACGTGTCATTGA | 9 |
| ID1737 | 77.1 | CAACGTTTTTGGCAGATGCAGCTCTTTCTGAACAGTAAGTCATTGTTGAGTGACATGAATA GGACGAGAGCTACAGAAAATGAAGGTTTCAAAAGATTGTCTAAAACAAAAGTCCTTTCAGT CCCGTATTCAGAGTT | 10 |
| ID0663 | 59.1 | ATAAATTCCGATTAGCCAAGGAAGCTCTGAGTCTAAATACCAGCAAAGCTCAGCCCCTGC AAGTGGGGACGAATTATTTGAAAGGTCATCCTAGAGACACTGAACAAAGGGCCTCTAGCA GCT | 11 |
| ID2706 | 57.2 | CCAATGCCAAAGCAGTTTACATTTTACAGATGCCATGGCAACCGTCAGGAAGTTACCCTGT ATGGTCTAAGAGGGGGAGGAACCCTCAGTTCCGGGAATTGCCCGCCCCTTTCCCGGAAA CTCATGAATAATCCACCC | 12 |
| ID3547 | 53.2 | CGTTCCCCCTTAACAAAAGCCACCTCCTCCCTCCTGAAAGGCACCATTGGTCACCAAAAC AAGTCCCCTTTGTCCTCCACCGCAGCTGGAGCCTGGGCCTTCCATCTCCTGGGGGCCAA TAAAACCCCAGATCAGCCAATCCTCCCTCCCTGGCTCTGTCA | 13 |
| ID0885 | 42.6 | CAGCAAAGGCTGCACGCAATGGGGGAGGGGGAAGGAGGGAAGGATATTTCCTCCTTTTT AGATCAGTTTCTTTTCTGAGCACAAGTGCTTTCTGTGTCATCCTAACAATGTAAGAGCCCA CTAAGTTTAGGACGCATAATACCGGCCTTTGTTCTACTCC | 14 |
| ID3548 | 40.0 | TCCAAACACTCCCCTGCAACAGATGTACACACAGAGCCCATTGTTTTCTCTAGAAATGTAA ACTGCACGTCACAGTTACTCCATACACACCACAAACAGACTCCCTTTGTTCTCAGGATATG TGTTGACACAAGAATATGTCATCCTCTCAGCTCCAGAC | 15 |
| ID3146 | 37.2 | TGAAAGAGATGGGTGTGTGGGAGTGGGGGAAGCTGAGCTCCAGGCAGACAGCACAGAG CCGCCTTGTCCAACCCAGCCAGGTGCCATGGCAACCAACAAAGGCCCCATTGTCCCGTG TCTGCTGGGCCTTCCTGAGGCTCTGGGGTCAGAGTCCCGGTT | 16 |
| ID4310 | 36.3 | TTGTCCTGCCTTTGAGAACACTGGAAGGGCACTGAAGGCTGACCAGCATGCACCTCACCC CACCTCCTCTCTGTCTCATCCTCAGCCATCTAAGCGGGAGACTCATCAGGCTTAAATTCTC ATTCTTATCCAAGATTCTTCTGCTTCAAATGAG | 17 |
| ID4428 | 36.2 | TCTAAATGGAGATCCTCCCATAAAGGGAGTTCTGTTTGGGCTTCCCTTGGAAGTCCATTGA AAGCGTTTCACATTTCAATGCTTCTTTACATTCTCCTCTAGAATGAATCTGGAAGGGCCATT TCATTCCAGCCCTATCAGATTTACTACTTCCTCCTGT | 18 |
| ID0785 | 34.3 | AAGAATGAACTGGGCCCAGCTGGGCATTTGCATCAAAGTGCCTCACCAGAGTTCCCAGCT GCTCTGGCAGAGTGCCCAGGCTTGCCCCAGAGCCCAAACAGAATGCTCTTTGAGATGCC TCTGGTTTTCA | 19 |
| ID3836 | 33.4 | TACCCTTCCAGGGGAGCAGTTTCCTGGCTGTGTCGTCAGATACTGTGGTTTACGCTGTTC CGGAGTCTGGAGGTCTGTGTGCCTTTGATCTCTTTCCCAACTAATTTGCACAGCTGCTGTG GAGGCCCTCATGAATGACCTCTTTCTTTCCTTTCT | 20 |
| ID3765 | 31.7 | TTGTCCTGCCTTTGAGAACACTGGAAGGGCACTGAAGGCTGACCAGCATGCACCTCACCC CACCTCCTCTCTGTCTCATCCTCAGCCATCTAAGCGGGAGACTCATCAGGCTTAAATTCTC ATTCTTATCCAAGATTCTTCTGCTTCAAATGAG | 21 |
| ID1176 | 31.3 | ACAAACTAAACACAGACATTTTCTCAATTAGTCATTTTCAGGTTTATTGCCTAAGATGCTAA TTGAAATTCAGGGCAGGCAAAAATACATCATTGGCCTTTGTATATCTTTGAAAGGCCTTCT GATGGGACCTTGCTCCGTATAAAGGCTTCTCTTCTCT | 22 |
| ID1888 | 31.1 | GACCTTGCCTCTTTCTGGCCTGTGGCCTTTCTCCTCCCCCAGCTGCTGTCTGGCCGCCCC ACCCCCAAGTCTCTGCCACAACCAAAGCTCCTTTGTTGCCTCCCCCTGCCCCGGGCCTCT CCACCTGCTGCTAGCTCAATCCCCTCTTTGTGTGGTTGGT | 23 |
| ID0291 | 29.0 | CATTCTTTGCAGATAGGAAACAGTTTGCTATCTGAGAGACTGGCCAAAAACCTTACTTTCA AGTAAAATAGGGCTCTATTCATAAGCTCCTACATGTACACAAGTTACTCTTTATACTCCTTT CAGACAATGATAAGAATAGAAACTATTTAATTCAGGA | 24 |
| ID1244 | 27.8 | ACAGCAGCCAGAGGAAGTGGCTGTGTACAGAGAGCTTTTGTGAGAGAAGAGGGGGAGGG GGAAAGAGCAAAGAAAAAGAAGGAAAGAAGAGAGGGAAGAGGCCAGAGACAGAGAAGG CCAGAGCA | 25 |
| ID0456 | 27.2 | ATCACAAAGTGGTTAGCATGCAGGGGAGTGCGCCGGTCCCCCGCCTGTATTTGCATGAC AAAAGCGCCTGCCCAACGGCTCTCGGTGGCTTTGTTGAGAGCTGCAGCCTCGCTCAGGC TGAGCCTTGGGAATTGTTCCCCGACATTGGTTGTTCCTGGGC | 26 |
| ID1322 | 26.7 | GGGCAACAAAGGATGCCATGCAAAGCAGCTCAGAACTTGAGTTGTTTCAACAGATGCTGT TTTATGTTTTGCCAAAGAATGGCCCCTTTTATTTCTGTCATGAATGCAGTCTTTCTTTCAGT TTCTAAATGTTTGCCAGTATTAACCCTAATCCTTATCT | 27 |
| ID2082 | 26.4 | ACAGAAGCCGTTGCTGCAGTAACCACTGCCGCAAGAGCAACTTTGGCAGCAGGCAGGTG CCAGGCAGCAAGCTGAGAGAACACTGACAGATTCAAAGTGTCTTCAGATACAAGGCTTTG TTTGTGCACAGTGAACAAAGGGCATATTGGGAAGGCCTCC | 28 |
| ID4035 | 26.2 | CCCAGACTGCCATCTCCACGGCCCCCACACCAGCACACAGATGCCTCATTCACGTGGTG CTCCCAGGGCCTGGCACCAGTTCTGAGGCTTAAAATAGGCACACAACGGGCCTTTGTCG GATAGAGGAAGGAAGGAAGGAGCGTTTCAGACAAAGGAGACA | 29 |
| ID1667 | 25.2 | ACCCTTTGCAGAGAGGAAAGAGAAAGGAGGTCTCCTGTTGGGGAGATCTGCTTTAGAGGT TAGGAAGAATGGTGGAGTTGTAAGACATTGGAATCACTGTCTAGGAAGGACAGTGGCCTT TTATCCAGGGCTATTTAAAAACAGACAGAGCTTCATCTAT | 30 |
| ID2816 | 25.1 | TTCCTGCATCTGGCCCTCTTTGAGAGCCCCTTGTGTGCCAATGACAAACCCTTCTTTATTT AAGCTCCTCTGAAGTGCCTTCCTGGTCTTGGCAGCTGGACAGGGCCCTGAATAAGACAGC GAGCCTCAATGGCAGGCTACCCCAGGGCTGGGTGACGTA | 31 |
| ID0403-1 | 24.9 | TGCCTCTCTTCCATTGGCATTGGAGTGGCTATAGGCCTTTGGAGGACCTGGCAAAGGGGA AGTTGATTTAGGGGTTCATTGAATTGAGGGGAAGTATGTAAGTGGTTTGCAGTCACTTCCT TGTGTGGTACTCTTGTATAGTAATGATTTCCTGGAATA | 32 |
| ID0403-2 | 24.9 | ATGTTAAACAGTGAAATCATCAGCAAAAAGCACAAAACTGCAGAAAACATGGCACTAAATA GAACACAAAAAGGACACTTGTTTACAGTATGAGAGCAGAAACAAGGAAACAGTGTTTCCTT GTTCAGCCACAGCTGGGACCTTGTGTGT | 33 |
| ID4421 | 21.7 | GCATTCATTTTACAGCTGGGGTAAGCTTTCTCTGAAATGCAGCTGGAATAGGCATTTCAAA GGCCCACAGTGTTATGCAAATGCTTCTATTCTCCATGTGGGCGG | 34 |
| ID4431 | 21.2 | AAATGGTGATTATCATCATTTGTTCAGGGACATTAGCATGAAGGCCGAATGTTCTGGCACA AAGACACGTCTGTGGGACAGGCAC | 35 |
| ID3499 | 20.7 | AAGAATGAACTGGGCCCAGCTGGGCATTTGCATCAAAGTGCCTCACCAGAGTTCCCAGCT GCTCTGGCAGAGTGCCCAGGCTTGCCCCAGAGCCCAAACAGAATGCTCTTTGAGATGCC TCTGGTTTTCA | 36 |
| CSE194 | 14.54 | ATCTCATATATGTGTATATGTATTTATGATTTACATATATACAGACGTATCACACACTCCGAA TAGAATACAGATTTCCTTCAATGACAGCAACAACTCAATGACCCGCCCACTAGCATGGCTA AAATACTAAACCACTAGATAGCTTTATGTGAACTTGAGCTTGATTGTTTCTTAGCTCTGCTT CAGAACCTCATGATCATGTGCATGCAATTTCTACAGAAGCGGAAACGTAGATGCTGTGGG TCAGTGAGCATTCCTTTCCAGGGTGCTTCTGAGATGAAGCCCCACGATTAACACAGGGCC CTTAATGATGGGCGGGGGACTAAGCATTCCAAGATTGGCCTGGATTCCTGCCAGAAGGG AGAGAAAGGGGTCTTTGTATGTGGACATCTGAAGGGCGGGCATAAACATGTTAGTTGATA CTTAACACCCAAAACCAGTGCCAAAACCGTTAGCTCCCCTCAATAGCCTACTCTGGTTTCA ACTGTCCGCGACAATGGAGCAGCCACCTCAATATACAAAACTTGCTCTTTAAACATCCTCA GAGGAATTCCCAAAATAGCAGCTGTTAGAAGCAAACAGACTCTAATTCATTGACATATGCA GACCTTTTAATGTTTTTCTGGTGACGTTTACATTTTTTTTTGTACCTACGCCATCTATGCATT TTTAATAATTACCTTGTAGATTACGTATGCAATCCCTCCAGGCACTTGAAGAAGGAGCCCC GGGATTCATCATCAGATGAAATTGTTAGTTTTTACGGCAAGAGGCCCAGCTAATTTATGCG GGGAATTTTTCAGACGTTTTTCCTCTGGGCTCCCTTCCCTGCTGTCCTCGCGCCTTTCTTC CTCAGTACATTAAGAGATTCTGATGTGCCTGCAGCA | 37 |
| CSE208 | 45.5 | AGATAAAGCCAGAAATAAAAGGGAAAGAAACAGTTATATGATTTTGGTGGGAATTTGCAGA AATACAGGCTGAAGCTGAAGTCTTCCATCAGGTGAGGTTTTTTCCATTGATCTCATCTCTC GGGCTCAGCTCTTTTATCTCCAGCGCATTTTGGATGTGGCTTCTCTTTCCACCTGCTGGGC CTTTAGATAGAGTACTTCCTTTTGTTTCAAGGTAGCATTTAACTGAGTCTGGAAAAACCATC ATTAAGGGAAAATAAAGCTGAAAGATGAATTGAAAACTATGTAAAAGGGCCCTTTCAGGCT GCCGGAGAGATTGCCTTTCCAGCCTAATGAGCCTGTGCTGCCTCTGATTCAGATGGTGCA AGGACAGGAGAAAAGAATGGAGTTTTGTGCACTGCGATTTTCCAAAGCACATAATGCTTAG TCATTTGGGGTGTTAGGAAAGAGAGAAAGTCGGAGCGGAGTGTGTGGTGAAGTGAGAGA | 38 |
| | | AAGAAAGAAGGCTGAATTTTCGTTAAGCTGCTCAAATGCCTGCTTTAACAAAAGTCTGCCA GGGCTAGAGGTGATTAGCCATCAGTGCTGCTGCTGTTTTTGCTGTCG | |
| CSE222 | 145.9 | CTCTTTGGGTCTAGGTATCATTTTGGGATCACATCATGATAACTCTATGTGTGTGTGCTGG GAGGGCAAAGTTGCAAAGCAATTACTCAGCAATGCTGAAGAAAGAAGCAATTACTGGAAC ACATTTTAGCCCCGCTTGTCAGAGTGACTTTCACTGGGGCACAAGGGAAGCATTCACTTA GGCAGTATCCCTTTCAGAGGCTTAATTTAGTTTTTTCTTCCCTTTAATTGTGTGATTCCAGT ATTTTAGCTACAATCCCTAGGCTGTAGGAACCTTTAAACACTTCTTTTTATAAAAGAAATGC ATGGGCTTTTGAAGAATAAACAGGGGGCTGCATAATTTATAGGAAAATGGCACCATGGAC TTTTCCTTCACAAAGAACATTGGGGTTACATTGGAACAAGCCAAATATGTTTTTAATGAATA GTCAGTAATCAGCAGCAGCTACAGTAAGTGCTTGAAATTTTTCTTTTATGTGCTATGAGTC CACAAACTAAGCATCTGATTCTGATTGCAGCCATGA | 39 |
| CSE012 | 10.3 | GTAGAGAATAGATTCTGTAGGGCTTTTAACCTCCACTGTTATTCCAAGGATGGGGCTCAGC TTCCTTCTTTTCCTTTCTGACTCTGTCTATATCAAGGGTCCCGTGGGTTGTTCCTTTCCATT GTGGTCTCACTGCCCAGCTTTAAGGCACCATCCTGGCACAGTGATAGGTTTACCTTTGTCT AAAAGTGTCCATTTGCATCTCTGTGGCCTTCTTGCCTGGGTGACACCCACTTGATTAGGCA TTTGCCCAGTGTGAGATCAGAGTCCCTCATTTACATTTCCCCACAATAGTGAATGGCTTAC AGGCTGTTCAAATACAGTTTCTAAACAGTGAATAATTCAGCCAGAAAGCCAGCCTTTTAAG GTTTGGATTTGGGGCATTTCTGAG | 40 |
| CSE005 | 11.5 | TCCCTTGGAAAGTGTTCAAATCAAAGCTACCTTTATACCACACTCCTTTGTGTCCTGATGTC CTGAATAGTGAGATTATCGGGTACACCCACCTGTTCCTGCACTTCCTTCCTGCTCAATTTA AATAAACAATGAGCTGTTTTTCTTCTTGACAAAGAGCCAGTCTATACAACAGCAGCAGGAA GATTACTTTCTATCTGTATGCAAACAGATACATAGCTGAGTTCTCTCCAATCCCCGGGAAA AGACCCTTTTAACTAAATGACTTATGCTTCT | 41 |
| CSE034 | 14.3 | GCCATAATTTGAACCTCGGAGTGCCCCACTGACAGCCAACGACACCAGAGGGTCCCTTTG TATGTAAGGATTGCATGACTCCAGTTGAATGACCCAAGCCGGGGACAAACAGGAAAGAAC | 42 |
| | | CCAGCAGCTCTGGAGGCCACTTGAGCTGACTGTCAAATGCTCTGGGCTGATTAACGACTA CGAACTTCAGGGAAGACAAGTGTGCTTTGATGCCACAGAGCTATCCTGGGCCTGTACAGG CCCGTTTCTGCTCTGAATGAGGAAGCTGTACTTTGCAGGCACCCGGCAGAACAAGAATAG GGCCTAAGATACCTGAATGAAGGGCACAGAGGAAGTACAAAATACAGTCATTGTTGGAGC TAGAAGCTGGTGCCAGGCTACGAGGGGGCCCAGTGGCCAAGCATTATG | |
| CSE078 | 10 | TGTCGGGCAGCCAAGACCTGTCAGGGACAAGAGTGCTGTGAGCTGCGGGCTGGTGGGT GGAAGGAGAGAGGCCCCTGGTGCTCCTGCCAAGCCAAGGAATGTGTCATTGCTGTGCTG TGCCTTCAGCCTGGCATTTGGGAACAAAGAGGTACTTTGTATGGATAACATAGACCAGGTT GTTGGGGAAGGGATCAGAGATGGGGGTAGACCCATTTTTAAAAATCTGCTCATGTCCCAG GCAAGTTTTTTTGGCAGGGATTGCAGTTGGGCTATTACAAAGAGCTGCTGTACACGAGGG GTATGTCTACTACTGTCAAATTACAGCAGCAATTATCTTGGGCACTG | 43 |
| CSE088 | 16.5 | CTCGATACCCACATTCCTAACCTTTTAATGCCAAGCAGCCTATAAGCATGTCAAATTTTACT CATGTTCCTGCTCTTTCCACTCAAGTTGGCTTTGTGGGTTGCTGGTTTTATCAAAATTGAGT GAGAGTGTGTTCAATACAATGTTGGATTCATTAGGGAGAAAAAAGCCAAATAAAAACCTTG CTTCTTCAATGCCAGGAGCTTACAAAGACATATAAACCATGAGGAATTCCAAGGACTTGCT CAGAAGCCGTTCTGCAAATGCATTTCTTGAACAAAGGGCCAATGTTCCTTTCAGGGAAGA GCGCGGGACTGGTTTCCGGCATTTTTCTTTAAATCATTAGGACCATGCCAGCAAACAAACA TATACAGCCCCTGGGAGAAAGAATCTCATTCAACACAGCTTACAAGAGTTTGATTATTAGG GAGGGGTGCTTCATGGCACGTGCCTCACCAGCCGGCCCCCAAATGTACAGATCTGTTTAA AGGGCTTTATTGATGGTGGCCGCAGACAGATAGAGCTTGTGAGGAGAACTGACATCCCAT GGATACTAACATATCAGCATCATCCTGACCAGCACACAATCCAGTCAGAGAGAGCTGAAA AGTCTGCCTCATTCCCTTGAAAATGTGCTTCTTAAGACAAATAAAATATAGAAATAAGAAAT TCACCTC | 44 |
| CSE025 | 21.3 | ATGGACAATGGCAGCCTGAAAGAATATGAGGGAGGGTCACCAGTGATGGACATGGTTTG GGGAGAGCCCAAGAATTGAAGGGGACTTGGGATTGTTGACAGACCTTCCACATCTCAGAA TGGGGGAAGTGGTTTCTTTTTTCCTCATTCTCCAAGGCTTCTCAGTCTCAGAGACCCCAAG | 45 |
| | | AGATGCCATTAATACAGATTCCTCCTCTGGCTCCTTTGAGACATTTGCTTTTGAAGGACCT GGAAGAGAAAGCTCTTTGTATGTGGCCAGAATAAACTCATTGTACACCCAAACAATTAACT CCTCAATGGACTTGCCAGCCCCTTGAAAGGCCTGGCCCTGCGTCCCTGGACCTCTTGGG CTCTATTCAGTCCCTTAAGCACACCAATGTTTTTGGGTTTTTGTTTTGTTTTGTTTGTTATTC TGAAAAGTCTTTCCCTGAGAATAGTCTGAGCGGATCAACATTGTCCTCAGCCTGGCATTTA TCTCTCCTCGCCAGCGAATGTCAATCAGGAATCAAAAAGCCAAACAGAGGAGGCTTTGAA CGGTGTCATCGGGGGTCGAAGTGACTTCCAGCTGTCTGTGCGTGGGCCAGGCCTTCTCG CAGGGCAAGGAATTGTGCTGTCACACAAACCCACTGTCTGTATAGGCTCACAGAGCCCA | |
| CSE033 | 20.9 | CTGGGGAACAAAACTTAGATCTTGATCATCCAGCATAGCTCTGGCATGCTGAGCGTGTGA TACACCTGTCTCTACCGTCAGGCAAACGAGTGGGTTCACAGGCAGAGGCCTGCCCTGCC AAACAATGCTCCCCTTTGTCCCCGCCCAGGGCTCAGGAGCCGCAAGGAAGCCAAATCCA TCAGCACTTGTCAGGGCCCTTGATGCACACAATGCAAGTTATGATCCACAGTCCCCGGCA AAGCCCCAGGCTTCCAGCAGGCAGCATTAACTGACACGCAATGTGTCAGTCAGCCTTCTA AAAGCCAGGGGTGGGGATCCGTGCCAGGGGGCCACTGAGCTCTCATTCCCAGAAGCCAA CCAGAGCC | 46 |
| CSE014 | 27.7 | GCCTTTTAGCTGATTAACAAATACAGTGAATTTAACTTTGTAGATTCTTAACATTTAAAGACA ACAGTCTAAGGCAATTTAAAGTATATTCTGCCAATCAGTTGTTGAGTTAGACCACCCATAG CCTTTTTTTATTCTTCATTGTTTGAATAATATGGGAGGAAATTTAGTGTATACTCTCTTCTTC CTGTATTTTCCAGTTAGATTAAGCTGTTGTCTTCACAATTGCCTCCCAGCTGCTGTGACTT GTGAAAACAACAGACCCTGCTCTGTTGTCTGGAGAAAGCCGGGATTGTTATGAGTAACTTT ATTGCAGGCACATGAGTGTGTTAAGACTTTAAAACACGCTCAAATGTCATGTGCACACCCA ATGACGGGCAAGAATAATTATGAAAAAACAAGAAATCTATGTCTATGAAACCGTGTTGGAC AAAATGGGCCATTCATTGTGTTCCCCTGGGAATGCCTGGACTCTGCAGTCAGAAAACAAA AAAGGCACTTTTTTCCTGCCGTAGGCTGAATGCTTAGCTTTGCCATCTGAGCCCTAAGAAA ACATGGGCCCCTCTACTGCTGGCGCACCCACGCACACTAAAAAATATTAATGCCTCTCTCT CCTTACTTTTTTCCAGGCATTCCCTCGTGTAGGCTGTAGCCAATATAATGTCAACACAAA | 47 |
| CSE061 | 58.3 | GAACAGCACTTAAATATTAATAGCATCACTGGGAAAACACGACAGCTATTTCCCTGTTTTCT GCAGCTCAATGAAGAGTTGTTCAGTGCAGACCCCAGTGCTCTTGCCTCATGAATGGACGT GATTGTGGGGACATCATCTCCTACTAAGGCAAAACGTGACACTGAGAACAAAGGCTGGTT TTTAGCCTGATGTGATGAGCTCACCCCAGTGCCCATGTGGGGACTTTGAAGCTCGTCCAG GTGCCAGTTTTCTGTGTATATGACATTCTTGGTTGGGGAGGGTGTGGACTGGGCAGCAGG CTTT | 48 |
| CSE075 | 58.1 | ATAAGCCCTTTAAATTAAATCTTTATGCAGATTCTATTTTATACTCCAAACTTTTCATGGAGC CTTGTGTAATTCTGATGTGTAAACCAACAAACAGAGAGGCTCTGTGTATGGAGTAGCTGGT TTGGATACCAGCTGGCTTGGCTGTGCGCCTTTCTAATGGTAATGAAGTCACACAGCTGCT AGGACAATCAGCTGGAGTTCAGATTGTCACTAGCTAAATGGATATCCATTGACTAGCTTCT TATCCGGACTCATTAAAGTTTAGTATTGACAAATAGTGGCTGGAGGTTTACAACTAAGAGG CATGTAGAAATGAACTTGTTGACTATTTTTTCCCTATAGCTTTTGCTTTGTGATTCACAGAA TGTCATTCATC | 49 |
| CSE054 | 65.7 | GCCAACAACTCATATAGTGGAGGCTTGAGCACACCATGCTTATTCCCAGAGTTCCAATGCT CTGCTTTTGAACCATTACCATGCCTTTCAGCAGCACTGTCAACAGAGCTCAGAGAAGGTCT TGAACTCCAGAGAATGAATGAGATATGCCAAATGAAGCATAATTACTTTATTGACGTCATTC AGCTTTCAACTCACATTTAATGAGAAAGCTTCTACTGCTTGAAACAGTGGGCCATTTGTAG CACATTATGCAAATGATAAGACTAATCTTTTGCATTATACATAAGAGCTGCTTATTGTGCAT TAACTAATTTATCTGACTAGGAAGGGGCTATGAATGCTGATTTTTATTAAGCTCTTCATTAA GTGAAAATGTAGGCATGGCAAGTGCATAT | 50 |
| CSE074 | 250.6 | ATACATTTTAGCACCAAACACAATTTAATAACAGGGCACAGAGGCCGCAAAGCCTCCTGAT TCTGTTCCATTACTAATTTATCAGGGGCTGAAAGCAACCAAACTTAGGATCCCAGGCAGAC AATGGAGCCTTTGAGGGGCAAGGGAAGCATCATTTAAAAGGAAAGCATTACCATTCTAAAA TAATGGCAAAGCACATGAACTGAGTGCTCTGAAAGAGAGCACTACAAAAAAGGTATTCTCA GCTTAAATGCCCCAAACACAAAAGCTTTTAGTTGGGACTTAGACGGGAAAAAGAAAATAGC TCCCAATAAATTGGTTTTACCAGTATCCTTTTGTGTGTGCTGGTGCCAAGGGGGCGAGGG | 51 |
| | | TACAGGCAAAGCGTGATTTTCCAAAACCTAGCTGAATTATGTTTCTTCTTTAGAACAAAACC CAACATTTTGCTAATGAGGATAAAGACAGTAAAACTGATATTCCTGTGCTATTACTAACTTG G | |
| CSE031 | 249.3 | CAGAGGAGAGAGGAGGTCATTTGGGACCATTTACTCATGCGAGAGTCACTGCCCCATGCT AAGATTTCCCCTAAAAATAAAATGATAAGATAATAACTCATAATGCTCTCCCCAAATCAGTA CCACACAGACCCCCCTCTTCTGTTTGCTCAGACCCCCGCTCTCCAGCACAAAGTCTTTATT GGTCTCCCTGCAGGGGTGAAAATAAAGGGGGTTTTGTTCTTCTCCTCCCCCTCCCCCCTC GAAGGGCTGAGTGAGTTTATCTGACACTGCAGCAGTCTTTGTAAACTGAGTGTCCCAGGA TAAACGCTCCCTTAAGAGTCGCTTGGCCTGGGACAGAGGGACCCTCTCCAGAGGAGAAT GGGGAGCTTGTTCCAATGGTCTTTTAATATGGGGGGCGGGGTGTCACCAAGGACAGGCC TCCAGCACACTCTCACAGAAGGGTCTCTGTCCTGAGGCTACAGTGGAGGGAATTCTTAAA GACACAGATGCTGAAAAGGGGGAAGGAATTTTTTTTTCCCCTAAGGGGAGGGGAGGGAG GTTAGGGCGGGAAGGAGAAGGGGGAGTGGCGAGGACTCCTCATTCCCCAGAACAATTCC AGTTACAACAAGGCGTCTCTAT | 52 |
| CSE006 | 524.3 | AGGAAGAAGCATGCTGGGTCATTTTTGCCTTGTTTCTAGCTGCATGAATTGAAAAGCCAGG CCCAGGTTCAATGCATCATTCTTGAAGCAGTGAACAAAATCCCTCTTTGTTCCCCAGTGAA ACTTATCTCTGAAGAGCTGCACTACTTCCTGTGAGATATAGCCCTGCAGATGTGTCGTGG GAGACCCGCCCGGCTGCACTGCCTTCCATAAAAGTGCAAGAATGGGCACTTTGTACTGCT GGACTGAAAATGACACTCACTCTTCTGATCCCCACTGGGGTTACCAAGTGGGCAAAACTT CCTGCTTAGAGGAGGGGTCATAGACAGCAGGGGGAGTGTGTGATTTTGTTTTGTTCCTTT GTGAATTTGTTTAGCTATTTATAGAGCTAAAGGAGAAAGCAAACGGGCACAATGTAGCACA GTGTTTTTCAAAGTGTATTCCATGGAACCCTTTCGTGGGTTGCAGACCCCAGGGGTGGAG GCAGAGGATGGGGTGGTGACAGCAGCAGGTGCTGTCCAGGGCCTTCTGTCT | 53 |
| minCSE031 | | GGACAGGCCTCCAGCACACTCTCACAGAAGGGTCTCTGTCCTGAGGCTACAGTGGAGGG AATTCTTAAAGACACAGATGCTGAAAAGGGGGAAGGAATTTTTTTTTCCCCTAAGGGGAGG GGAGGGAGGTTAGGGCGGGAAGGAGAAGGGGGAGTGGCGA | 54 |
| minCSE033 | | GGCAAACGAGTGGGTTCACAGGCAGAGGCCTGCCCTGCCAAACAATGCTCCCCTTTGTC CCCGCCCAGGGCTCAGGAGCCGCAAGGAAGCCAAATCCATCAGCACTTGTCAGGGCCCT TGATGCACACAATGCAAGTTATGATCCACAGTCCCCGGCAAA | 55 |
| minCSE034 | | GGGAAGACAAGTGTGCTTTGATGCCACAGAGCTATCCTGGGCCTGTACAGGCCCGTTTCT GCTCTGAATGAGGAAGCTGTACTTTGCAGGCACCCGGCAGAACAAGAATAGGGCCTAAG ATACCTGAATGAAGGGCACAGAGGAAGTACAAAATACAGTC | 56 |
| minCSE061 | | AATGGACGTGATTGTGGGGACATCATCTCCTACTAAGGCAAAACGTGACACTGAGAACAA AGGCTGGTTTTTAGCCTGATGTGATGAGCTCACCCCAGTGCCCATGTGGGGACTTTGAAG CTCGTCCAGGTGCCAGTTTTCTGTGTATATGACATTCTTG | 57 |
| minCSE074 | | CAAACACAATTTAATAACAGGGCACAGAGGCCGCAAAGCCTCCTGATTCTGTTCCATTACT AATTTATCAGGGGCTGAAAGCAACCAAACTTAGGATCCCAGGCAGACAATGGAGCCTTTG AGGGGCAAGGGAAGCATCATTTAAAAGGAAAGCATTACC | 58 |
| minCSE075 | | CTCCAAACTTTTCATGGAGCCTTGTGTAATTCTGATGTGTAAACCAACAAACAGAGAGGCT CTGTGTATGGAGTAGCTGGTTTGGATACCAGCTGGCTTGGCTGTGCGCCTTTCTAATGGT AATGAAGTCACACAGCTGCTAGGACAATCAGCTGGAGTT | 59 |
| minCSE194 | | GGTCAGTGAGCATTCCTTTCCAGGGTGCTTCTGAGATGAAGCCCCACGATTAACACAGGG CCCTTAATGATGGGCGGGGGACTAAGCATTCCAAGATTGGCCTGGATTCCTGCCAGAAG GGAGAGAAAGGGGTCTTTGTATGTGGACATCTGAAGGGCGG | 60 |
| minCSE208 | | GATGTGGCTTCTCTTTCCACCTGCTGGGCCTTTAGATAGAGTACTTCCTTTTGTTTCAAGG TAGCATTTAACTGAGTCTGGAAAAACCATCATTAAGGGAAAATAAAGCTGAAAGATGAATT GAAAACTATGTAAAAGGGCCCTTTCAGGCTGCCGGAGA | 61 |
| minCSE222 | | CAATTACTCAGCAATGCTGAAGAAAGAAGCAATTACTGGAACACATTTTAGCCCCGCTTGT CAGAGTGACTTTCACTGGGGCACAAGGGAAGCATTCACTTAGGCAGTATCCCTTTCAGAG GCTTAATTTAGTTTTTTCTTCCCTTTAATTGTGTGATTC | 62 |
| minCSE229 | | TTCTTTTCAAGGATGCCCAAAGCGTGTGACTCTTACAGAGAGCCAATGGGGAAAGGCAGT GTCGGGACTAAGCAATGAATGGCTCTTCAATGGCCAGCTGCCCGCCCAATAGGATAAAAG AAAACCCCACATAATACTTCCCTTTGTCTCCAAAAAAATT | 63 |

| | | | |
|---|---|---|---|
| Note: Fold Change refers to the expression fold change of the individual enhancer compared to mCMV. | | | |

**TABLE 2 - Sequences of full SSEs**

| **SSE** | **Sequence** | **SEQ ID NO.** |
|---|---|---|
| SSE-1 (ID1101_ID2904_ ID0109_ID4328) | GTGAGTGTGACTCACATGCTGGCATCAGAGCCCCTCTTAGAGCTTAGAACACTGGGCCTCTGTCAGGCAAAG AAAAGGAGTCCCTATGAATAGCCATCTCCACAAATAGCAGTTACTGTCACTTCTTAAAAGACACAAGCATGGC TGGAGACCGGCACTGTGCATGTTCTTGGGAAAAAGTGGTATTTAATGAAGGCGCTTTGAGCTGAATCAAAGA GCCTCATTCTTCTGAAAGTCCTTCTTCCTAGTCTCTGCATCCGGCTCCTATCAAAGGAAATCCACAGCTTTGG CTTTTCGTTGCTGTTGTCATGGAAATCGTTGAAATTGGATCCTGAGGAGAATGTCACCACCTCTTTGACAATAA CTAGCTTTCAGAGCTGAACACAGTGAATCTGGATACTGTGTATATGTTTAAATGAGTTAGGAAATGACATCACA TTATTTATTTCTTCTTTTGTGACTTAGTAAATTGACTTGGCCTACAATCTATTCTCCATAGTCATCAGAGTTTCTT TAAAGGCACGGTTCAGACAATGGCACCCTCTGTTCCAAACCCTCCAGGGCTTCCTATTTCACTTAGCATAAAA ACCAAAGTCCTCACAATGACCGACAAGTCTTCACACAATGTGCCCCTTCATTG | 64 |
| SSE-2 (ID0109_ID4328_ ID0876_ID2339) | GAAATTGGATCCTGAGGAGAATGTCACCACCTCTTTGACAATAACTAGCTTTCAGAGCTGAACACAGTGAATC TGGATACTGTGTATATGTTTAAATGAGTTAGGAAATGACATCACATTATTTATTTCTTCTTTTGTGACTTAGTAA ATTGACTTGGCCTACAATCTATTCTCCATAGTCATCAGAGTTTCTTTAAAGGCACGGTTCAGACAATGGCACCC TCTGTTCCAAACCCTCCAGGGCTTCCTATTTCACTTAGCATAAAAACCAAAGTCCTCACAATGACCGACAAGT CTTCACACAATGTGCCCCTTCATTGGAGCAGGCAGTCATTTGGTTCCCTTCTCTGAATGCATTGGCTCTGATC CACGGCGCCTCAGGAAGTGAAAGGGAGCAGGCAGTGAGGCCAAGCCATGAATGTTGGGCCCTTTTCTTAGA AAACAAAGGGTTCACTTTAGGCTAGGAGGAAACATCCGGGGGTCGGCGGAACTGAGTCTCCTTTGTGGAGC CCCTCATTTGCATGATAAACCCCGGGGACAAAGTGTGTTCTTGTTACTTGGACTGTTTCTTGGAAACTGCTGC TACCTGGAGCGAGTGAACACAGGGCAAGTATTCTCGCCCACAGCAACGTGTCATTGA | 65 |
| SSE-3 (ID0876_ID2339_ ID1737_ID0663) | GAGCAGGCAGTCATTTGGTTCCCTTCTCTGAATGCATTGGCTCTGATCCACGGCGCCTCAGGAAGTGAAAGG GAGCAGGCAGTGAGGCCAAGCCATGAATGTTGGGCCCTTTTCTTAGAAAACAAAGGGTTCACTTTAGGCTAG GAGGAAACATCCGGGGGTCGGCGGAACTGAGTCTCCTTTGTGGAGCCCCTCATTTGCATGATAAACCCCGG GGACAAAGTGTGTTCTTGTTACTTGGACTGTTTCTTGGAAACTGCTGCTACCTGGAGCGAGTGAACACAGGG CAAGTATTCTCGCCCACAGCAACGTGTCATTGACAACGTTTTTGGCAGATGCAGCTCTTTCTGAACAGTAAGT CATTGTTGAGTGACATGAATAGGACGAGAGCTACAGAAAATGAAGGTTTCAAAAGATTGTCTAAAACAAAAGT CCTTTCAGTCCCGTATTCAGAGTTATAAATTCCGATTAGCCAAGGAAGCTCTGAGTCTAAATACCAGCAAAGC TCAGCCCCTGCAAGTGGGGACGAATTATTTGAAAGGTCATCCTAGAGACACTGAACAAAGGGCCTCTAGCAG CT | 66 |
| SSE-4 (ID1737_ID0663_ ID2706_ID3547) | CAACGTTTTTGGCAGATGCAGCTCTTTCTGAACAGTAAGTCATTGTTGAGTGACATGAATAGGACGAGAGCTA CAGAAAATGAAGGTTTCAAAAGATTGTCTAAAACAAAAGTCCTTTCAGTCCCGTATTCAGAGTTATAAATTCCG ATTAGCCAAGGAAGCTCTGAGTCTAAATACCAGCAAAGCTCAGCCCCTGCAAGTGGGGACGAATTATTTGAAA GGTCATCCTAGAGACACTGAACAAAGGGCCTCTAGCAGCTCCAATGCCAAAGCAGTTTACATTTTACAGATGC CATGGCAACCGTCAGGAAGTTACCCTGTATGGTCTAAGAGGGGGAGGAACCCTCAGTTCCGGGAATTGCCC GCCCCTTTCCCGGAAACTCATGAATAATCCACCCCGTTCCCCCTTAACAAAAGCCACCTCCTCCCTCCTGAAA GGCACCATTGGTCACCAAAACAAGTCCCCTTTGTCCTCCACCGCAGCTGGAGCCTGGGCCTTCCATCTCCTG GGGGCCAATAAAACCCCAGATCAGCCAATCCTCCCTCCCTGGCTCTGTCA | 67 |
| SSE-5 (ID2706_ID3547_ ID3548_ID3146) | CCAATGCCAAAGCAGTTTACATTTTACAGATGCCATGGCAACCGTCAGGAAGTTACCCTGTATGGTCTAAGAG GGGGAGGAACCCTCAGTTCCGGGAATTGCCCGCCCCTTTCCCGGAAACTCATGAATAATCCACCCCGTTCCC CCTTAACAAAAGCCACCTCCTCCCTCCTGAAAGGCACCATTGGTCACCAAAACAAGTCCCCTTTGTCCTCCAC CGCAGCTGGAGCCTGGGCCTTCCATCTCCTGGGGGCCAATAAAACCCCAGATCAGCCAATCCTCCCTCCCT GGCTCTGTCATCCAAACACTCCCCTGCAACAGATGTACACACAGAGCCCATTGTTTTCTCTAGAAATGTAAAC TGCACGTCACAGTTACTCCATACACACCACAAACAGACTCCCTTTGTTCTCAGGATATGTGTTGACACAAGAA TATGTCATCCTCTCAGCTCCAGACTGAAAGAGATGGGTGTGTGGGAGTGGGGGAAGCTGAGCTCCAGGCAG ACAGCACAGAGCCGCCTTGTCCAACCCAGCCAGGTGCCATGGCAACCAACAAAGGCCCCATTGTCCCGTGT CTGCTGGGCCTTCCTGAGGCTCTGGGGTCAGAGTCCCGGTT | 68 |
| SSE-6 (ID3548_ID3146_ ID4310_ID4428) | TCCAAACACTCCCCTGCAACAGATGTACACACAGAGCCCATTGTTTTCTCTAGAAATGTAAACTGCACGTCAC AGTTACTCCATACACACCACAAACAGACTCCCTTTGTTCTCAGGATATGTGTTGACACAAGAATATGTCATCCT CTCAGCTCCAGACTGAAAGAGATGGGTGTGTGGGAGTGGGGGAAGCTGAGCTCCAGGCAGACAGCACAGA GCCGCCTTGTCCAACCCAGCCAGGTGCCATGGCAACCAACAAAGGCCCCATTGTCCCGTGTCTGCTGGGCC TTCCTGAGGCTCTGGGGTCAGAGTCCCGGTTTTGTCCTGCCTTTGAGAACACTGGAAGGGCACTGAAGGCTG ACCAGCATGCACCTCACCCCACCTCCTCTCTGTCTCATCCTCAGCCATCTAAGCGGGAGACTCATCAGGCTT AAATTCTCATTCTTATCCAAGATTCTTCTGCTTCAAATGAGTCTAAATGGAGATCCTCCCATAAAGGGAGTTCT GTTTGGGCTTCCCTTGGAAGTCCATTGAAAGCGTTTCACATTTCAATGCTTCTTTACATTCTCCTCTAGAATGA ATCTGGAAGGGCCATTTCATTCCAGCCCTATCAGATTTACTACTTCCTCCTGT | 69 |
| SSE-7 (ID4310_ID4428_ ID0785_ID3836) | TTGTCCTGCCTTTGAGAACACTGGAAGGGCACTGAAGGCTGACCAGCATGCACCTCACCCCACCTCCTCTCT GTCTCATCCTCAGCCATCTAAGCGGGAGACTCATCAGGCTTAAATTCTCATTCTTATCCAAGATTCTTCTGCTT CAAATGAGTCTAAATGGAGATCCTCCCATAAAGGGAGTTCTGTTTGGGCTTCCCTTGGAAGTCCATTGAAAGC GTTTCACATTTCAATGCTTCTTTACATTCTCCTCTAGAATGAATCTGGAAGGGCCATTTCATTCCAGCCCTATC AGATTTACTACTTCCTCCTGTAAGAATGAACTGGGCCCAGCTGGGCATTTGCATCAAAGTGCCTCACCAGAGT TCCCAGCTGCTCTGGCAGAGTGCCCAGGCTTGCCCCAGAGCCCAAACAGAATGCTCTTTGAGATGCCTCTGG TTTTCATACCCTTCCAGGGGAGCAGTTTCCTGGCTGTGTCGTCAGATACTGTGGTTTACGCTGTTCCGGAGTC TGGAGGTCTGTGTGCCTTTGATCTCTTTCCCAACTAATTTGCACAGCTGCTGTGGAGGCCCTCATGAATGACC TCTTTCTTTCCTTTCT | 70 |
| SSE-8 (minCSE034_minC SE061_minCSE07 5_minCSE222) | GGGAAGACAAGTGTGCTTTGATGCCACAGAGCTATCCTGGGCCTGTACAGGCCCGTTTCTGCTCTGAATGAG GAAGCTGTACTTTGCAGGCACCCGGCAGAACAAGAATAGGGCCTAAGATACCTGAATGAAGGGCACAGAGG AAGTACAAAATACAGTCAATGGACGTGATTGTGGGGACATCATCTCCTACTAAGGCAAAACGTGACACTGAGA ACAAAGGCTGGTTTTTAGCCTGATGTGATGAGCTCACCCCAGTGCCCATGTGGGGACTTTGAAGCTCGTCCA GGTGCCAGTTTTCTGTGTATATGACATTCTTGCTCCAAACTTTTCATGGAGCCTTGTGTAATTCTGATGTGTAA ACCAACAAACAGAGAGGCTCTGTGTATGGAGTAGCTGGTTTGGATACCAGCTGGCTTGGCTGTGCGCCTTTC TAATGGTAATGAAGTCACACAGCTGCTAGGACAATCAGCTGGAGTTCAATTACTCAGCAATGCTGAAGAAAGA | 71 |
| | AGCAATTACTGGAACACATTTTAGCCCCGCTTGTCAGAGTGACTTTCACTGGGGCACAAGGGAAGCATTCACT TAGGCAGTATCCCTTTCAGAGGCTTAATTTAGTTTTTTCTTCCCTTTAATTGTGTGATTC | |
| SSE-9 (minCSE034_minC SE074_minCSE07 5_minCSE222) | GGGAAGACAAGTGTGCTTTGATGCCACAGAGCTATCCTGGGCCTGTACAGGCCCGTTTCTGCTCTGAATGAG GAAGCTGTACTTTGCAGGCACCCGGCAGAACAAGAATAGGGCCTAAGATACCTGAATGAAGGGCACAGAGG AAGTACAAAATACAGTCCAAACACAATTTAATAACAGGGCACAGAGGCCGCAAAGCCTCCTGATTCTGTTCCA TTACTAATTTATCAGGGGCTGAAAGCAACCAAACTTAGGATCCCAGGCAGACAATGGAGCCTTTGAGGGGCA AGGGAAGCATCATTTAAAAGGAAAGCATTACCCTCCAAACTTTTCATGGAGCCTTGTGTAATTCTGATGTGTAA ACCAACAAACAGAGAGGCTCTGTGTATGGAGTAGCTGGTTTGGATACCAGCTGGCTTGGCTGTGCGCCTTTC TAATGGTAATGAAGTCACACAGCTGCTAGGACAATCAGCTGGAGTTCAATTACTCAGCAATGCTGAAGAAAGA AGCAATTACTGGAACACATTTTAGCCCCGCTTGTCAGAGTGACTTTCACTGGGGCACAAGGGAAGCATTCACT TAGGCAGTATCCCTTTCAGAGGCTTAATTTAGTTTTTTCTTCCCTTTAATTGTGTGATTC | 72 |
| SSE-10 (minCSE061_minC SE074_minCSE19 4_minCSE222) | AATGGACGTGATTGTGGGGACATCATCTCCTACTAAGGCAAAACGTGACACTGAGAACAAAGGCTGGTTTTTA GCCTGATGTGATGAGCTCACCCCAGTGCCCATGTGGGGACTTTGAAGCTCGTCCAGGTGCCAGTTTTCTGTG TATATGACATTCTTGCAAACACAATTTAATAACAGGGCACAGAGGCCGCAAAGCCTCCTGATTCTGTTCCATTA CTAATTTATCAGGGGCTGAAAGCAACCAAACTTAGGATCCCAGGCAGACAATGGAGCCTTTGAGGGGCAAGG GAAGCATCATTTAAAAGGAAAGCATTACCGGTCAGTGAGCATTCCTTTCCAGGGTGCTTCTGAGATGAAGCCC CACGATTAACACAGGGCCCTTAATGATGGGCGGGGGACTAAGCATTCCAAGATTGGCCTGGATTCCTGCCAG AAGGGAGAGAAAGGGGTCTTTGTATGTGGACATCTGAAGGGCGGCAATTACTCAGCAATGCTGAAGAAAGAA GCAATTACTGGAACACATTTTAGCCCCGCTTGTCAGAGTGACTTTCACTGGGGCACAAGGGAAGCATTCACTT AGGCAGTATCCCTTTCAGAGGCTTAATTTAGTTTTTTCTTCCCTTTAATTGTGTGATTC | 73 |
| SSE-11 (minCSE075_minC SE194_minCSE22 2_minCSE229) | CTCCAAACTTTTCATGGAGCCTTGTGTAATTCTGATGTGTAAACCAACAAACAGAGAGGCTCTGTGTATGGAG TAGCTGGTTTGGATACCAGCTGGCTTGGCTGTGCGCCTTTCTAATGGTAATGAAGTCACACAGCTGCTAGGA CAATCAGCTGGAGTTGGTCAGTGAGCATTCCTTTCCAGGGTGCTTCTGAGATGAAGCCCCACGATTAACACA GGGCCCTTAATGATGGGCGGGGGACTAAGCATTCCAAGATTGGCCTGGATTCCTGCCAGAAGGGAGAGAAA GGGGTCTTTGTATGTGGACATCTGAAGGGCGGCAATTACTCAGCAATGCTGAAGAAAGAAGCAATTACTGGA ACACATTTTAGCCCCGCTTGTCAGAGTGACTTTCACTGGGGCACAAGGGAAGCATTCACTTAGGCAGTATCC | 74 |
| | CTTTCAGAGGCTTAATTTAGTTTTTTCTTCCCTTTAATTGTGTGATTCTTCTTTTCAAGGATGCCCAAAGCGTGT GACTCTTACAGAGAGCCAATGGGGAAAGGCAGTGTCGGGACTAAGCAATGAATGGCTCTTCAATGGCCAGCT GCCCGCCCAATAGGATAAAAGAAAACCCCACATAATACTTCCCTTTGTCTCCAAAAAAATT | |
| SSE-12 (minCSE034_minC SE075_minCSE19 4_minCSE222) | GGGAAGACAAGTGTGCTTTGATGCCACAGAGCTATCCTGGGCCTGTACAGGCCCGTTTCTGCTCTGAATGAG GAAGCTGTACTTTGCAGGCACCCGGCAGAACAAGAATAGGGCCTAAGATACCTGAATGAAGGGCACAGAGG AAGTACAAAATACAGTCCTCCAAACTTTTCATGGAGCCTTGTGTAATTCTGATGTGTAAACCAACAAACAGAGA GGCTCTGTGTATGGAGTAGCTGGTTTGGATACCAGCTGGCTTGGCTGTGCGCCTTTCTAATGGTAATGAAGT CACACAGCTGCTAGGACAATCAGCTGGAGTTGGTCAGTGAGCATTCCTTTCCAGGGTGCTTCTGAGATGAAG CCCCACGATTAACACAGGGCCCTTAATGATGGGCGGGGGACTAAGCATTCCAAGATTGGCCTGGATTCCTGC CAGAAGGGAGAGAAAGGGGTCTTTGTATGTGGACATCTGAAGGGCGGCAATTACTCAGCAATGCTGAAGAAA GAAGCAATTACTGGAACACATTTTAGCCCCGCTTGTCAGAGTGACTTTCACTGGGGCACAAGGGAAGCATTC ACTTAGGCAGTATCCCTTTCAGAGGCTTAATTTAGTTTTTTCTTCCCTTTAATTGTGTGATTC | 75 |
| SSE-13 (minCSE074_minC SE075_minCSE19 4_minCSE229) | CAAACACAATTTAATAACAGGGCACAGAGGCCGCAAAGCCTCCTGATTCTGTTCCATTACTAATTTATCAGGG GCTGAAAGCAACCAAACTTAGGATCCCAGGCAGACAATGGAGCCTTTGAGGGGCAAGGGAAGCATCATTTAA AAGGAAAGCATTACCCTCCAAACTTTTCATGGAGCCTTGTGTAATTCTGATGTGTAAACCAACAAACAGAGAG GCTCTGTGTATGGAGTAGCTGGTTTGGATACCAGCTGGCTTGGCTGTGCGCCTTTCTAATGGTAATGAAGTCA CACAGCTGCTAGGACAATCAGCTGGAGTTGGTCAGTGAGCATTCCTTTCCAGGGTGCTTCTGAGATGAAGCC CCACGATTAACACAGGGCCCTTAATGATGGGCGGGGGACTAAGCATTCCAAGATTGGCCTGGATTCCTGCCA GAAGGGAGAGAAAGGGGTCTTTGTATGTGGACATCTGAAGGGCGGTTCTTTTCAAGGATGCCCAAAGCGTGT GACTCTTACAGAGAGCCAATGGGGAAAGGCAGTGTCGGGACTAAGCAATGAATGGCTCTTCAATGGCCAGCT GCCCGCCCAATAGGATAAAAGAAAACCCCACATAATACTTCCCTTTGTCTCCAAAAAAATT | 76 |
| SSE-14 (minCSE034_minC SE061_minCSE22 2_minCSE229) | GGGAAGACAAGTGTGCTTTGATGCCACAGAGCTATCCTGGGCCTGTACAGGCCCGTTTCTGCTCTGAATGAG GAAGCTGTACTTTGCAGGCACCCGGCAGAACAAGAATAGGGCCTAAGATACCTGAATGAAGGGCACAGAGG AAGTACAAAATACAGTCAATGGACGTGATTGTGGGGACATCATCTCCTACTAAGGCAAAACGTGACACTGAGA ACAAAGGCTGGTTTTTAGCCTGATGTGATGAGCTCACCCCAGTGCCCATGTGGGGACTTTGAAGCTCGTCCA GGTGCCAGTTTTCTGTGTATATGACATTCTTGCAATTACTCAGCAATGCTGAAGAAAGAAGCAATTACTGGAAC | 77 |
| | ACATTTTAGCCCCGCTTGTCAGAGTGACTTTCACTGGGGCACAAGGGAAGCATTCACTTAGGCAGTATCCCTT TCAGAGGCTTAATTTAGTTTTTTCTTCCCTTTAATTGTGTGATTCTTCTTTTCAAGGATGCCCAAAGCGTGTGAC TCTTACAGAGAGCCAATGGGGAAAGGCAGTGTCGGGACTAAGCAATGAATGGCTCTTCAATGGCCAGCTGCC CGCCCAATAGGATAAAAGAAAACCCCACATAATACTTCCCTTTGTCTCCAAAAAAATT | |
| SSE-15 (minCSE034_minC SE061_minCSE07 5_minCSE229) | GGGAAGACAAGTGTGCTTTGATGCCACAGAGCTATCCTGGGCCTGTACAGGCCCGTTTCTGCTCTGAATGAG GAAGCTGTACTTTGCAGGCACCCGGCAGAACAAGAATAGGGCCTAAGATACCTGAATGAAGGGCACAGAGG AAGTACAAAATACAGTCAATGGACGTGATTGTGGGGACATCATCTCCTACTAAGGCAAAACGTGACACTGAGA ACAAAGGCTGGTTTTTAGCCTGATGTGATGAGCTCACCCCAGTGCCCATGTGGGGACTTTGAAGCTCGTCCA GGTGCCAGTTTTCTGTGTATATGACATTCTTGCTCCAAACTTTTCATGGAGCCTTGTGTAATTCTGATGTGTAA ACCAACAAACAGAGAGGCTCTGTGTATGGAGTAGCTGGTTTGGATACCAGCTGGCTTGGCTGTGCGCCTTTC TAATGGTAATGAAGTCACACAGCTGCTAGGACAATCAGCTGGAGTTTTCTTTTCAAGGATGCCCAAAGCGTGT GACTCTTACAGAGAGCCAATGGGGAAAGGCAGTGTCGGGACTAAGCAATGAATGGCTCTTCAATGGCCAGCT GCCCGCCCAATAGGATAAAAGAAAACCCCACATAATACTTCCCTTTGTCTCCAAAAAAATT | 78 |
| SSE-16 (minCSE075_minC SE194_minCSE20 8_minCSE222) | CTCCAAACTTTTCATGGAGCCTTGTGTAATTCTGATGTGTAAACCAACAAACAGAGAGGCTCTGTGTATGGAG TAGCTGGTTTGGATACCAGCTGGCTTGGCTGTGCGCCTTTCTAATGGTAATGAAGTCACACAGCTGCTAGGA CAATCAGCTGGAGTTGGTCAGTGAGCATTCCTTTCCAGGGTGCTTCTGAGATGAAGCCCCACGATTAACACA GGGCCCTTAATGATGGGCGGGGGACTAAGCATTCCAAGATTGGCCTGGATTCCTGCCAGAAGGGAGAGAAA GGGGTCTTTGTATGTGGACATCTGAAGGGCGGGATGTGGCTTCTCTTTCCACCTGCTGGGCCTTTAGATAGA GTACTTCCTTTTGTTTCAAGGTAGCATTTAACTGAGTCTGGAAAAACCATCATTAAGGGAAAATAAAGCTGAAA GATGAATTGAAAACTATGTAAAAGGGCCCTTTCAGGCTGCCGGAGACAATTACTCAGCAATGCTGAAGAAAGA AGCAATTACTGGAACACATTTTAGCCCCGCTTGTCAGAGTGACTTTCACTGGGGCACAAGGGAAGCATTCACT TAGGCAGTATCCCTTTCAGAGGCTTAATTTAGTTTTTTCTTCCCTTTAATTGTGTGATTC | 79 |
| SSE-17 (minCSE074_minC | CAAACACAATTTAATAACAGGGCACAGAGGCCGCAAAGCCTCCTGATTCTGTTCCATTACTAATTTATCAGGG GCTGAAAGCAACCAAACTTAGGGCAGACAATGGAGCCTTTGAGGGGCAAGGGAAGCATCATTTAAAAGGAAA GCATTACCGGTCAGTGAGCATTCCTTTCCAGGGTGCTTCTGAGATGAAGCCCCACGATTAACACAGGGCCCT TAATGATGGGCGGGGGACTAAGCATTCCAAGATTGGCCTGGATTCCTGCCAGAAGGGAGAGAAAGGGGTCT | 80 |
| SE194_minCSE22 2_minCSE229) | TTGTATGTGGACATCTGAAGGGCGGCAATTACTCAGCAATGCTGAAGAAAGAAGCAATTACTGGAACACATTT TAGCCCCGCTTGTCAGAGTGACTTTCACTGGGGCACAAGGGAAGCATTCACTTAGGCAGTATCCCTTTCAGA GGCTTAATTTAGTTTTTTCTTCCCTTTAATTGTGTGATTCTTCTTTTCAAGGATGCCCAAAGCGTGTGACTCTTA CAGAGAGCCAATGGGGAAAGGCAGTGTCGGGACTAAGCAATGAATGGCTCTTCAATGGCCAGCTGCCCGCC CAATAGGATAAAAGAAAACCCCACATAATACTTCCCTTTGTCTCCAAAAAAATTATCCCAG | |

## Claims

1. A super-enhancer activated by one or more transcription factors, wherein the super-enhancer comprises two or more enhancer sequences derived from different genomic loci, wherein each enhancer sequence comprises a binding site for the transcription factor, wherein the super-enhancer comprises four enhancer sequences and wherein the super-enhancer comprises at least one SOX dimer motif and/or at least one SOX motif, wherein the SOX dimer motif comprises SEQ ID NO: 1 or SEQ ID NO: 2 and wherein the SOX motif comprises SEQ ID NO: 3.

2. The super-enhancer according to claim 1, wherein each of the two or more enhancer sequences within the super-enhancer are less than 300 nucleotides long, in particular is about 160 nucleotides long and wherein the super-enhancer is less than 1200 nucleotides long, in particular less than 700 nucleotides long.

3. The super-enhancer according to any one of claims 1 to 2, wherein the super-enhancer comprises a sequence with at least 80% sequence identity to a sequence selected from the group consisting of SEQ ID NO: 4-63, optionally wherein the super-enhancer comprises four enhancer sequences selected from the group consisting of SEQ ID NO: 4-63.

4. The super-enhancer according to any one of claims 1 to 3, wherein the super-enhancer comprises a sequence with at least 80% sequence identity to a sequence selected from the group consisting of SEQ ID NO: 64-80, and/or wherein each enhancer sequence further comprises between 20 and 400 nucleotides upstream and/or downstream of the binding site for the transcription factor present in its genomic locus.

5. A functional nucleic acid molecule comprising the super-enhancer according to any one of claims 1 to 4, operably linked to a transgene.

6. The functional nucleic acid of claim 5, wherein the transgene encodes a gene for expression in a cell, a therapeutic payload or reprogramming factor.

7. A functional nucleic acid molecule comprising a super-enhancer activated by one or more transcription factors expressed in a target aberrant cell and an adverse payload, wherein said payload is expressed upon activation of the super-enhancer by the transcription factor(s), wherein the synthetic super-enhancer comprises at least one SOX dimer motif or at least one SOX motif, wherein the SOX dimer motif comprises SEQ ID NO: 1 or SEQ ID NO: 2 and the SOX motif comprises SEQ ID NO: 3, and wherein the synthetic super-enhancer comprises four enhancer sequences.

8. The functional nucleic acid molecule according to claim 7, wherein the transcription factor is differentially expressed in the target aberrant cell, and optionally wherein the transcription factor is a developmental or stem-cell affiliated transcription factor.

9. The functional nucleic acid molecule according to claim 7 or claim 8, wherein the adverse payload encodes a protein that stimulates an immune response that leads to the recruitment of cytotoxic immune cells, optionally wherein the adverse payload is a cytokine such as IL-12, and/or wherein the adverse payload is a suicide gene which encodes a protein capable of converting an inactive prodrug into a cytotoxic drug, optionally wherein the suicide gene is a herpes simplex virus thymidine kinase (HSV-TK) gene and the prodrug is ganciclovir, acyclovir or valacyclovir.

10. A composition comprising the functional nucleic acid molecule according to any one of claims 6 to 9, for use in the treatment of cancer, such as glioblastoma.

11. A super-enhancer activated by one or more transcription factors of the SOX family, wherein the super-enhancer comprises two or more enhancer sequences and at least one SOX motif and/or SOX dimer motif, wherein the synthetic super-enhancer comprises four enhancer sequences, wherein the SOX dimer motif comprises SEQ ID NO: 1 or SEQ ID NO: 2 and wherein the SOX motif comprises SEQ ID NO: 3.

12. The super-enhancer according to claim 11, wherein each of the two or more enhancer sequences has a different sequence, and/or wherein each of the two or more enhancer sequences within the super-enhancer are less than 300 nucleotides long, in particular is about 160 nucleotides long.

13. A vector comprising the synthetic super-enhancer according to any one of claims 1-4, 11-12 or the functional nucleic acid molecule according to any one of claims 5-9, optionally a viral vector such as AAV.

14. A composition comprising the synthetic super-enhancer 1-4, 11-12, the functional nucleic acid molecule according to any one of claims 5-9 or the vector according to claim 13 and an acceptable carrier.

15. A kit comprising the functional nucleic acid molecule according to any one of claims 5-9 comprising an adverse payload, wherein the adverse payload is a suicide gene which encodes a protein capable of converting an inactive prodrug into a cytotoxic drug, and wherein the kit additionally comprises said inactive prodrug for administration with the functional nucleic acid molecule.
